# EUROPEAN PATENT SPECIFICATION

(11) **EP 3 119 884 B1**
(45) Date of publication and mention of the grant of the patent: **10.07.2019**
(21) Application number: 15716230.6
(22) Date of filing: 20.03.2015
(51) Int. Cl.: C12N 9/54

(54) **SERINE PROTEASES OF BACILLUS SPECIES**
SERINPROTEASEN AUS EINER BACILLUS-SPEZIES
SÉRINE-PROTÉASES DE L'ESPÈCE BACILLUS

(30) Priority: 21.03.2014 US 201461968853 P
(43) Date of publication of application: 25.01.2017
(60) Divisional application: 19177372.0
(73) Proprietor: Danisco US Inc., Palo Alto, California 94304 (US)
(72) Inventor: KOLKMAN, Marc, Palo Alto, California 94304 (US); HEMMINGSEN, Anja, Palo Alto, California 94304 (US); MEJLDAL, Rie, Palo Alto, California 94304 (US); BOTT, Richard, Palo Alto, California 94304 (US); BABE, Lilia, Palo Alto, California 94304 (US)
(74) Representative: Mewburn Ellis LLP
(86) International application number: PCT/US2015/021813
(87) International publication number: WO 2015/143360

(56) References cited:
- WO-A2-2012/175708
- JOSHI SWATI ET AL: "Characteristics and applications of a recombinant alkaline serine protease from a novel bacteriumBacillus lehensis", BIORESOURCE TECHNOLOGY, ELSEVIER BV, GB, vol. 131, 25 December 2012 (2012-12-25), pages 76-85, XP028989213, ISSN: 0960-8524, DOI: 10.1016/J.BIORTECH.2012.12.124
- SAEKI ET AL: "Detergent alkaline proteases: enzymatic properties, genes, and crystal structures", JOURNAL OF BIOSCIENCE AND BIOENGINEERING, ELSEVIER, AMSTERDAM, NL, vol. 103, no. 6, 1 June 2007 (2007-06-01), pages 501-508, XP022153072, ISSN: 1389-1723, DOI: 10.1263/JBB.103.501

## Description

### BACKGROUND

The present disclosure relates to serine proteases cloned from *Bacillus spp.,* and variants thereof Compositions containing the serine proteases are suitable for use in cleaning fabrics and hard surfaces, as well as in a variety of industrial applications.

Serine proteases are enzymes (EC No. 3.4.21) possessing an active site serine that initiates hydrolysis of peptide bonds of proteins. There are two broad categories of serine proteases, based on their structure: chymotrypsin-like (trypsin-like) and subtilisin-like. The prototypical subtilisin (EC No. 3.4.21.62) was initially obtained from *B. subtilis.* Subtilisins and their homologues are members of the S8 peptidase family of the MEROPS classification scheme. Members of family S8 have a catalytic triad in the order Asp, His and Ser in their amino acid sequence. WO2012/175708 describes polypeptides having protease activity and polynucleotides encoding the polypeptides. In particular, amino acid sequences of a *Bacillus sp.* NN01832 S8A protease, a *Bacillus bogoriensis* protease and a *Paenibacillus dendritiformis* S8A protease are disclosed.

Although serine proteases have long been known in the art of industrial enzymes, there remains a need for engineered proteases that are suitable for particular conditions and uses.

### SUMMARY

The present compositions and methods relate to recombinant serine proteases cloned from *Bacillus spp.,* and variants thereof, as defined in the claims. Compositions containing the serine proteases are suitable for use in cleaning fabrics and hard surfaces, as well as in a variety of industrial applications.

In a first aspect the present invention provides a recombinant polypeptide, or an active fragment thereof, comprising an amino acid sequence, wherein the amino acid sequence of the recombinant polypeptide or of the active fragment has at least 90% identity to the amino acid sequence of SEQ ID NO:3 or SEQ ID NO:4, and wherein the recombinant polypeptide or the active fragment has proteolytic activity, and wherein the recombinant polypeptide or the active fragment retains at least 80% activity after 20 minutes at 50°C under stressed conditions comprising a Tris/EDTA assay.

In some embodiments the polypeptide or the active fragment comprises a DTGIDXXHXXLXNLVXTSLGXSXVGGXXXDVXGH motif, wherein the initial D is the active site Aspartic acid, the terminal H is the active site Histidine, and X is any amino acid.

In some embodiments the polypeptide or the active fragment comprises a DTGIDXXHXXLXₐNLVXTSLGXSXVGGX_{b}XX_{c}DVXGH motif, wherein the initial D is the active site Aspartic acid, the terminal H is the active site Histidine, and X, Xa, Xb, and Xc are any amino acid, provided that when Xa is arginine, Xb and Xc are not glycine.

In some embodiments the polypeptide or the active fragment comprises a VSG sequence at residue positions 80-82, wherein the amino acid positions of the polypeptide or the active fragment are numbered by correspondence with the amino acid sequence set forth in SEQ ID NO:7.

In some embodiments the polypeptide or the active fragment comprises an insertion of at least one amino acid residue compared to SEQ ID NO:18, wherein the insertion is between residue positions 39-47, wherein the residue positions are numbered by correspondence with the amino acid sequence set forth in SEQ ID NO:18, optionally wherein the residue positions 39-47 are replaced with HQSLANLVNTSLG.

In some embodiments the polypeptide or the active fragment comprises a deletion of at least one amino acid residue compared to SEQ ID NO:18, wherein the deletion is between residue positions 51-64, wherein the residue positions are numbered by correspondence with the amino acid sequence set forth in SEQ ID NO:18, optionally wherein the residue positions 51-64 are replaced with VGGSTMDVQGH, VGGSA/PEDVQGH, VGGNPEDRQGH, or VGGTPADVHGH.

In some embodiments the polypeptide or the active fragment comprises a deletion of at least one amino acid residue compared to SEQ ID NO:18, wherein the deletion is between residue positions 68-95, wherein the residue positions are numbered by correspondence with the amino acid sequence set forth in SEQ ID NO:18, optionally wherein the residue positions 68-95 are replaced with VAGTIASYGSVSGVMHNATLVPVKV.

In some embodiments the polypeptide retains at least 50% of its maximal protease activity at a pH range of 5 to 12.

In some embodiments the polypeptide retains at least 50% of its maximal protease activity at a temperature range of 55°C to 80°C.

In some embodiments the recombinant polypeptide or the active fragment has at least 95% identity to the amino acid sequence of SEQ ID NO:3 or SEQ ID NO:4.

In some embodiments the stressed conditions are the Tris/EDTA assay conditions of Example 11.

In some embodiments the recombinant polypeptide or the active fragment comprises at least one substitution selected from X003N, X006R, X010E, X020I, X026N, X028R, X029I, X038A, X041P, X042N, X044R, X048D, X053R X059G, X061G, X085Q, X088R, X090I, X096G, X098N, X103M, X104Y, X107Q, X113A, X115S, X117N, X131D, X132S, X133D, X136N, X137N, X138I, X139N, X143S, X144S, X146T, X147L, X157R, X168N, X169A, X178N, X179R, X180T, X204Y, X207G, X208Q, X209F, X210R, X212L, X219T, X222V, X229I, X230K, X231S, X231A, X239T, X240Q, X241V, X243N, X245L, X246R, X247D, X255L, X256N, X257Q, X264N, X266Y, X271A, and X273G.

In a second aspect the present invention provides a composition comprising a surfactant and the recombinant polypeptide of the first aspect of the invention. In some embodiments the composition is a detergent composition selected from a laundry detergent, a fabric softening detergent, an automatic dishwashing detergent, a hand dish detergent, and a hard-surface cleaning detergent.

In some embodiments the composition further comprises: at least one calcium ion and/or zinc ion, at least one stabilizer, at least one bleaching agent, at least one adjunct ingredient, and/or one or more additional enzymes or enzyme derivatives selected from acyl transferases, alpha-amylases, beta-amylases, alpha-galactosidases, arabinosidases, aryl esterases, beta-galactosidases, carrageenases, catalases, cellobiohydrolases, cellulases, chondroitinases, cutinases, endo-beta-1,4-glucanases, endo-beta-mannanases, esterases, exo-mannanases, galactanases, glucoamylases, hemicellulases, hyaluronidases, keratinases, laccases, lactases, ligninases, lipases, lipoxygenases, mannanases, oxidases, pectate lyases, pectin acetyl esterases, pectinases, pentosanases, peroxidases, phenoloxidases, phosphatases, phospholipases, phytases, polygalacturonases, proteases, pullulanases, reductases, rhamnogalacturonases, beta-glucanases, tannases, transglutaminases, xylan acetyl-esterases, xylanases, xyloglucanases, xylosidases, metalloproteases, additional serine proteases, and combinations thereof.

In some embodiments the composition comprises from about 0.001% to about 1.0 weight % of a recombinant polypeptide of the first aspect of the invention.

In some embodiments the composition is phosphate-free.

In some embodiments the composition contains phosphate.

In some embodiments the composition is boron-free.

In some embodiments the composition contains boron.

In some embodiments the composition is formulated at a pH of from about 7 to about 12.

In a third aspect the present invention provides a method of cleaning, comprising contacting a surface or an item with a composition comprising (i) a buffer and the recombinant polypeptide of the first aspect of the invention, or (ii) the composition of the third aspect of the invention.

In a fourth aspect the present invention provides a method for producing a recombinant polypeptide comprising:
(a) stably transforming a host cell with an expression vector comprising a polynucleotide encoding the polypeptide of the first aspect of the invention;
(b) cultivating said transformed host cell under conditions suitable for said host cell to produce said polypeptide; and
(c) recovering said polypeptide.

In a fifth aspect the present invention provides a polynucleotide comprising a nucleic acid sequence encoding an amino acid sequence selected from SEQ ID NO:3, and SEQ ID NO:4.

In a sixth aspect the present invention provides an expression vector comprising the polynucleotide of the fifth aspect of the invention.

In a seventh aspect the present invention provides a host cell transformed with the vector of the sixth aspect of the invention.

In an eighth aspect the present invention provides a textile or leather processing composition or a feather processing, animal feed, contact lens cleaning, wound cleaning composition comprising the polypeptide of the first aspect of the invention.

### BRIEF DESCRIPTION OF THE DRAWINGS

Figure 1 provides a plasmid map of pHYT- BspAG00296 for expression of the BspAG00296 serine protease.
Figure 2 provides a plasmid map of pBN- BspM04033 for expression of the BspM04033 serine protease.
Figure 3 provides a plot of the protease activity of BspAG00296 on a DMC substrate.
Figure 4 provides a plot of the protease activity of BspM04033 on a DMC substrate.
Figure 5 provides cleaning efficiency curves of BspAG00296 in heavy duty liquid (HDL) laundry detergents.
Figure 6 provides cleaning efficiency curves of BspAG00296 in heavy duty dry (HDD) laundry detergents.
Figure 7 provides cleaning efficiency curves of BspAG00296 in automatic dish washing (ADW) detergents.
Figure 8 provides cleaning efficiency curves of BspM04033 in heavy duty liquid (HDL) laundry detergents.
Figure 9 provides cleaning efficiency curves of BspM04033 in heavy duty dry (HDD) laundry detergents.
Figure 10 provides cleaning efficiency curves of BspM04033 in automatic dish washing (ADW) detergents.
Figure 11 provides a phylogenetic tree of the WHY-clade, SWT77-clade, BspAG00296-clade, WP026675114-clade, and SWT22-clade subtilisins , and various other bacterial serine proteases.
Figure 12A-1 - 12E provides a CLUSTAL W alignment of the amino acid sequences of subtilisins BspAG00296, BspM04033, BspWO65, BspAA02831, SWT4, SWT22, SWT32, SWT40, SWT41, SWT77, SWT123, with the sequences of several other bacterial serine proteases. The numbering of residues in the 1JEA and 1CSE structures is with respect to subtilisin BPN'; while the numbering of residues for BspM04033 and all other proteases shown is the consecutive linear sequence.
Figure 13A - 13B provides a structure-based alignment of the region of the WHY-clade amino acid sequences comprising the motif that is bracketed by the catalytic residues D33 and H66 (residue numbering according to BspM04033 linear sequence).
Figure 14 provides a structural image of sequence motif changes found in WHY-clade subtilisins.
Figure 15A provides a schematic showing superimposition of a monomer from the crystallographic structures of BspAG00296 and SWT77-tr.
Figure 15B provides a structural image of sequence motif changes found when the structure of SWT77-tr was compared with *B. lentus* subtilisin.

### DETAILED DESCRIPTION

Described are compositions and methods relating to recombinant serine proteases from several *Bacillus* species. The compositions and methods are based, in part, on the observation that recombinant BspAG00296 and BspM04033, among others, have protease activity in the presence of a surfactant, in basic reaction conditions, and at elevated temperatures. These features of BspAG00296, BspM04033, which are predicted to be shared by SWT77, BspWO765, BspAA02831, SWT4, SWT22, SWT32, SWT40, SWT41, and SWT123 make these proteases well suited for use in cleansing fabrics and hard surfaces, as well as in textile, leather and feather processing. The new proteases are also well suited to inclusion in compositions for protein degradation, including but not limited to laundry and dish washing detergents. The present invention is as defined in the claims.

### I. Definitions

Prior to describing the present compositions and methods in detail, the following terms are defined for clarity. Terms and abbreviations not defined should be accorded their ordinary meaning as used in the art. Unless defined otherwise herein, all technical and scientific terms used herein have the same meaning as commonly understood by one of ordinary skill in the art. Unless otherwise indicated, the practice of the present disclosure involves conventional techniques commonly used in molecular biology, protein engineering, microbiology, and recombinant DNA, which are within the skill of the art. Such techniques are known to those of skill in the art and are described in numerous texts and reference works well known to those of skill in the art. Although any methods and materials similar or equivalent to those described herein find use in the practice of the present disclosure, some suitable methods and materials are described herein. The terms defined immediately below are more fully described by reference to the Specification as a whole.

As used herein, the singular "a," "an" and "the" includes the plural unless the context clearly indicates otherwise. Unless otherwise indicated, nucleic acid sequences are written left to right in 5' to 3' orientation; and amino acid sequences are written left to right in amino to carboxy orientation. It is to be understood that this disclosure is not limited to the particular methodology, protocols, and reagents described herein, absent an indication to the contrary.

It is intended that every maximum numerical limitation given throughout this Specification includes every lower numerical limitation, as if such lower numerical limitations were expressly written herein. Every minimum numerical limitation given throughout this Specification will include every higher numerical limitation, as if such higher numerical limitations were expressly written herein. Every numerical range given throughout this Specification will include every narrower numerical range that falls within such broader numerical range, as if such narrower numerical ranges were all expressly written herein.

As used herein in connection with a numerical value, the term "about" refers to a range of +/- 0.5 of the numerical value, unless the term is otherwise specifically defined in context. For instance, the phrase a "pH value of about 6" refers to pH values of from 5.5 to 6.5, unless the pH value is specifically defined otherwise.

As used herein, the terms "protease" and "proteinase" refer to an enzyme that has the ability to break down proteins and peptides. A protease has the ability to conduct "proteolysis," by hydrolysis of peptide bonds that link amino acids together in a peptide or polypeptide chain forming the protein. This activity of a protease as a protein-digesting enzyme is referred to as "proteolytic activity." Many well-known procedures exist for measuring proteolytic activity. For example, proteolytic activity may be ascertained by comparative assays that analyze the respective protease's ability to hydrolyze a suitable substrate. Exemplary substrates useful in the analysis of protease or proteolytic activity, include, but are not limited to, di-methyl casein (Sigma C-9801), bovine collagen (Sigma C-9879), bovine elastin (Sigma E-1625), and bovine keratin (ICN Biomedical 902111). Colorimetric assays utilizing these substrates are well known in the art (See e.g., WO 99/34011 and U.S. Pat. No. 6,376,450). The pNA peptidyl assay (See e.g., Del Mar et al., Anal Biochem, 99:316-320, 1979) also finds use in determining the active enzyme concentration. This assay measures the rate at which p-nitroaniline is released as the enzyme hydrolyzes a soluble synthetic substrate, such as succinyl-alanine-alanine-proline-phenylalanine-p-nitroanilide (suc-AAPF-pNA). The rate of production of yellow color from the hydrolysis reaction is measured at 410 nm on a spectrophotometer and is proportional to the active enzyme concentration. In addition, absorbance measurements at 280 nanometers (nm) can be used to determine the total protein concentration in a sample of purified protein. The activity on substrate/protein concentration gives the enzyme specific activity.

As used herein in connection to a polypeptide such as a protease, the term "variant" refers to a polypeptide comprising an amino acid sequence that differs in at least one amino acid residue from the amino acid sequence of a parent or reference polypeptide (including but not limited to wild-type polypeptides). The difference can be a modification which is either an insertion, deletion, or substitution. In some embodiments, the polypeptide variant that differs from the amino acid sequence of a parent or reference polypeptide contains one or more naturally-occurring or man-made substitutions, insertions, or deletions of an amino acid. In other embodiments, the polypeptide variant that differs from the amino acid sequence of a parent or reference polypeptide contains one or more naturally-occurring substitutions, insertions, or deletions of an amino acid. In further embodiments the polypeptide variant that differs from the amino acid sequence of a parent or reference polypeptide contains one or more man-made substitutions, insertions, or deletions of an amino acid.

As used herein, "the genus *Bacillus"* includes all species within the genus *"Bacillus,"* as known to those of skill in the art, including but not limited to *B. subtilis, B. licheniformis, B. lentus, B. brevis, B. stearothermophilus, B. alkalophilus, B. amyloliquefaciens, B. clausii, B. sonorensis, B. halodurans, B. pumilus, B. lautus, B. pabuli, B. cereus, B. agaradhaerens, B akibai, B. clarkii, B. pseudofirmus, B. lehensis, B. megaterium, B. coagulans, B. circulans, B. gibsonii,* and *B. thuringiensis.* It is recognized that the genus Bacillus continues to undergo taxonomical reorganization. Thus, it is intended that the genus include species that have been reclassified, including but not limited to such organisms as *B. stearothermophilus,* which is now named *"Geobacillus stearothermophilus",* or *B. polymyxa,* which is now *"Paenibacillus polymyxa".* The production of resistant endospores under stressful environmental conditions is considered the defining feature of the genus *Bacillus,* although this characteristic also applies to the recently named *Alicyclobacillus, Amphibacillus, Aneurinibacillus, Anoxybacillus, Brevibacillus, Filobacillus, Gracilibacillus, Halobacillus, Paenibacillus, Salibacillus, Thermobacillus, Ureibacillus,* and *Virgibacillus.*

The terms "polynucleotide" and "nucleic acid," which are used interchangeably herein, refer to a polymer of any length of nucleotide monomers covalently bonded in a chain. DNA (deoxyribonucleic acid), a polynucleotide comprising deoxyribonucleotides, and RNA (ribonucleic acid), a polymer of ribonucleotides, are examples of polynucleotides or nucleic acids having distinct biological functions. Polynucleotides or nucleic acids include, but are not limited to, a single-, double- or triple-stranded DNA, genomic DNA, cDNA, RNA, DNA-RNA hybrid, or a polymer comprising purine and pyrimidine bases, or other natural, chemically, biochemically modified, non-natural or derivatized nucleotide bases. The following are non-limiting examples of polynucleotides: genes, gene fragments, chromosomal fragments, expressed sequence tag(s) (EST(s)), exons, introns, messenger RNA (mRNA), transfer RNA (tRNA), ribosomal RNA (rRNA), ribozymes, complementary DNA (cDNA), recombinant polynucleotides, branched polynucleotides, plasmids, vectors, isolated DNA of any sequence, isolated RNA of any sequence, nucleic acid probes, and primers.

As used herein, the term "mutation" refers to changes made to a reference amino acid or nucleic acid sequence. It is intended that the term encompass substitutions, insertions and deletions.

As used herein, the term "vector" refers to a nucleic acid construct used to introduce or transfer nucleic acid(s) into a target cell or tissue. A vector is typically used to introduce foreign DNA into a cell or tissue. Vectors include plasmids, cloning vectors, bacteriophages, viruses (e.g., viral vector), cosmids, expression vectors, shuttle vectors, and the like. A vector typically includes an origin of replication, a multicloning site, and a selectable marker. The process of inserting a vector into a target cell is typically referred to as transformation. The present invention includes, in some embodiments, a vector that comprises a DNA sequence encoding a serine protease polypeptide (e.g., precursor or mature serine protease polypeptide) that is operably linked to a suitable prosequence (e.g., secretory, signal peptide sequence, etc.) capable of effecting the expression of the DNA sequence in a suitable host, and the folding and translocation of the recombinant polypeptide chain.

As used herein, the term "expression cassette," "expression plasmid" or "expression vector" refers to a nucleic acid construct or vector generated recombinantly or synthetically for the expression of a nucleic acid of interest in a target cell. An expression vector or expression cassette typically comprises a promoter nucleotide sequence that drives expression of the foreign nucleic acid. The expression vector or cassette also typically includes any other specified nucleic acid elements that permit transcription of a particular nucleic acid in a target cell. A recombinant expression cassette can be incorporated into a plasmid, chromosome, mitochondrial DNA, plastid DNA, virus, or nucleic acid fragment. Many prokaryotic and eukaryotic expression vectors are commercially available.

As used herein, a "plasmid" refers to an extrachromosomal DNA molecule which is capable of replicating independently from the chromosomal DNA. A plasmid is double stranded (ds) and may be circular and is typically used as a cloning vector.

As used herein in the context of introducing a nucleic acid sequence into a cell, the term "introduced" refers to any method suitable for transferring the nucleic acid sequence into the cell. Such methods for introduction include but are not limited to protoplast fusion, transfection, transformation, electroporation, conjugation, and transduction. Transformation refers to the genetic alteration of a cell which results from the uptake, optional genomic incorporation, and expression of genetic material (e.g., DNA).

As used herein, a nucleic acid is "operably linked" with another nucleic acid sequence when it is placed into a functional relationship with another nucleic acid sequence. For example, a promoter or enhancer is operably linked to a nucleotide coding sequence if the promoter affects the transcription of the coding sequence. A ribosome binding site may be operably linked to a coding sequence if it is positioned so as to facilitate translation of the coding sequence. Typically, "operably linked" DNA sequences are contiguous. However, enhancers do not have to be contiguous. Linking is accomplished by ligation at convenient restriction sites. If such sites do not exist, synthetic oligonucleotide adaptors or linkers may be used in accordance with conventional practice.

As used herein the term "gene" refers to a polynucleotide (e.g., a DNA segment), that encodes a polypeptide and includes regions preceding and following the coding regions. In some instances a gene includes intervening sequences (introns) between individual coding segments (exons).

As used herein, "recombinant" when used with reference to a cell typically indicates that the cell has been modified by the introduction of a foreign nucleic acid sequence or that the cell is derived from a cell so modified. For example, a recombinant cell may comprise a gene not found in identical form within the native (non-recombinant) form of the cell, or a recombinant cell may comprise a native gene (found in the native form of the cell) that has been modified and re-introduced into the cell. A recombinant cell may comprise a nucleic acid endogenous to the cell that has been modified without removing the nucleic acid from the cell; such modifications include those obtained by gene replacement, site-specific mutation, and related techniques known to those of ordinary skill in the art. Recombinant DNA technology includes techniques for the production of recombinant DNA in vitro and transfer of the recombinant DNA into cells where it may be expressed or propagated, thereby producing a recombinant polypeptide. "Recombination" and "recombining" of polynucleotides or nucleic acids refer generally to the assembly or combining of two or more nucleic acid or polynucleotide strands or fragments to generate a new polynucleotide or nucleic acid.

A nucleic acid or polynucleotide is said to "encode" a polypeptide if, in its native state or when manipulated by methods known to those of skill in the art, it can be transcribed and/or translated to produce the polypeptide or a fragment thereof. The anti-sense strand of such a nucleic acid is also said to encode the sequence.

The terms "host strain" and "host cell" refer to a suitable host for an expression vector comprising a DNA sequence of interest.

A "protein" or "polypeptide" comprises a polymeric sequence of amino acid residues. The terms "protein" and "polypeptide" are used interchangeably herein. The single and 3-letter code for amino acids as defined in conformity with the IUPAC-IUB Joint Commission on Biochemical Nomenclature (JCBN) is used throughout this disclosure. The single letter X refers to any of the twenty amino acids. It is also understood that a polypeptide may be coded for by more than one nucleotide sequence due to the degeneracy of the genetic code. Mutations can be named by the one letter code for the parent amino acid, followed by a position number and then the one letter code for the variant amino acid. For example, mutating glycine (G) at position 87 to serine (S) is represented as "G087S" or "G87S". Mutations can also be named by using the three letter code for an amino acid followed by its position in the polypeptide chain as counted from the N-terminus; for example, Ala10 for alanine at position 10. Multiple mutations are indicated by inserting a "-""+," "/," or ";" between the mutations. Mutations at positions 87 and 90 are represented as either "G087S-A090Y" or "G87S-A90Y" or "G87S + A90Y" or "G087S + A090Y". For deletions, the one letter code "Z" is used. For an insertion relative to the parent sequence, the one letter code "Z" is on the left side of the position number. For a deletion, the one letter code "Z" is on the right side of the position number. For insertions, the position number is the position number before the inserted amino acid(s), plus 0.01 for each amino acid. For example, an insertion of three amino acids alanine (A), serine (S) and tyrosine (Y) between position 87 and 88 is shown as "Z087.01A-Z087.02S-Z087.03Y." Thus, combining all the mutations above plus a deletion at position 100 is: "G087S- Z087.01A-Z087.02S-Z087.03Y-A090Y-A100Z." When describing modifications, a position followed by amino acids listed in parentheses indicates a list of substitutions at that position by any of the listed amino acids. For example, 6(L,I) means position 6 can be substituted with a leucine or isoleucine.

A "prosequence" or "propeptide sequence" refers to an amino acid sequence between the signal peptide sequence and mature protease sequence that is necessary for the proper folding and secretion of the protease; they are sometimes referred to as intramolecular chaperones. Cleavage of the prosequence or propeptide sequence results in a mature active protease. Bacterial serine proteases are often expressed as pro-enzymes.

The terms "signal sequence" and "signal peptide" refer to a sequence of amino acid residues that may participate in the secretion or direct transport of the mature or precursor form of a protein. The signal sequence is typically located N-terminal to the precursor or mature protein sequence. The signal sequence may be endogenous or exogenous. A signal sequence is normally absent from the mature protein. A signal sequence is typically cleaved from the protein by a signal peptidase after the protein is transported.

The term "mature" form of a protein, polypeptide, or peptide refers to the functional form of the protein, polypeptide, or peptide without the signal peptide sequence and propeptide sequence.

The term "precursor" form of a protein or peptide refers to a mature form of the protein having a prosequence operably linked to the amino or carbonyl terminus of the protein. The precursor may also have a "signal" sequence operably linked to the amino terminus of the prosequence. The precursor may also have additional polypeptides that are involved in post-translational activity (e.g., polypeptides cleaved therefrom to leave the mature form of a protein or peptide).

The term "wild-type" in reference to an amino acid sequence or nucleic acid sequence indicates that the amino acid sequence or nucleic acid sequence is a native or naturally-occurring sequence. As used herein, the term "naturally-occurring" refers to anything (e.g., proteins, amino acids, or nucleic acid sequences) that is found in nature. Conversely, the term "non-naturally occurring" refers to anything that is not found in nature (e.g., recombinant nucleic acids and protein sequences produced in the laboratory or modification of the wild-type sequence).

As used herein with regard to amino acid residue positions, "corresponding to" or "corresponds to" or "corresponds" refers to an amino acid residue at the enumerated position in a protein or peptide, or an amino acid residue that is analogous, homologous, or equivalent to an enumerated residue in a protein or peptide. As used herein, "corresponding region" generally refers to an analogous position in a related proteins or a reference protein.

The terms "derived from" and "obtained from" refer to not only a protein produced or producible by a strain of the organism in question, but also a protein encoded by a DNA sequence isolated from such strain and produced in a host organism containing such DNA sequence. Additionally, the term refers to a protein which is encoded by a DNA sequence of synthetic and/or cDNA origin and which has the identifying characteristics of the protein in question. To exemplify, "proteases derived from Bacillus" refers to those enzymes having proteolytic activity that are naturally produced by Bacillus, as well as to serine proteases like those produced by Bacillus sources but which through the use of genetic engineering techniques are produced by other host cells transformed with a nucleic acid encoding the serine proteases.

The term "identical" in the context of two polynucleotide or polypeptide sequences refers to the nucleic acids or amino acids in the two sequences that are the same when aligned for maximum correspondence, as measured using sequence comparison or analysis algorithms described below and known in the art.

As used herein, "homologous genes" or "homologous proteins" refers to a pair of genes or proteins which are identical or very similar to each other and are believed to derive from a common ancestor. The term encompasses genes or proteins that are separated by speciation (i.e., the development of new species) (e.g., orthologous genes or orthologous proteins), as well as genes or proteins that have been separated by genetic duplication (e.g., paralogous genes or paralogous proteins).

As used herein, "% identity" or percent identity" or "PID" refers to protein sequence identity. Percent identity may be determined using standard techniques known in the art. Useful algorithms include the BLAST algorithms (See, Altschul et al., J Mol Biol, 215:403-410, 1990; and Karlin and Altschul, Proc Natl Acad Sci USA, 90:5873-5787, 1993). The BLAST program uses several search parameters, most of which are set to the default values. The NCBI BLAST algorithm finds the most relevant sequences in terms of biological similarity but is not recommended for query sequences of less than 20 residues (Altschul et al., Nucleic Acids Res, 25:3389-3402, 1997; and Schaffer et al., Nucleic Acids Res, 29:2994-3005, 2001). Exemplary default BLAST parameters for a nucleic acid sequence searches include: Neighboring words threshold = 11; E-value cutoff = 10; Scoring Matrix = NUC.3.1 (match = 1, mismatch = -3);Gap Opening = 5; and Gap Extension = 2. Exemplary default BLAST parameters for amino acid sequence searches include: Word size = 3; E-value cutoff = 10; Scoring Matrix = BLOSUM62; Gap Opening = 11; and Gap extension = 1. A percent (%) amino acid sequence identity value is determined by the number of matching identical residues divided by the total number of residues of the "reference" sequence including any gaps created by the program for optimal/maximum alignment. BLAST algorithms refer to the "reference" sequence as the "query" sequence.

As used herein, "homologous proteins" or "homologous proteases" refers to proteins that have distinct similarity in primary, secondary, and/or tertiary structure. Protein homology can refer to the similarity in linear amino acid sequence when proteins are aligned. Homologous search of protein sequences can be done using BLASTP and PSI-BLAST from NCBI BLAST with threshold (E-value cut-off) at 0.001. (Altschul SF, Madde TL, Shaffer AA, Zhang J, Zhang Z, Miller W, Lipman DJ. Gapped BLAST and PSI BLAST a new generation of protein database search programs. Nucleic Acids Res 1997 Set 1;25(17):3389-402). Using this information, proteins sequences can be grouped. A phylogenetic tree can be built using the amino acid sequences. Amino acid sequences can be entered in a program such as the Vector NTI Advance suite and a Guide Tree can be created using the Neighbor Joining (NJ) method (Saitou and Nei, Mol Biol Evol, 4:406-425, 1987). The tree construction can be calculated using Kimura's correction for sequence distance and ignoring positions with gaps. A program such as AlignX can display the calculated distance values in parenthesis following the molecule name displayed on the phylogenetic tree.

Understanding the homology between molecules can reveal the evolutionary history of the molecules as well as information about their function; if a newly sequenced protein is homologous to an already characterized protein, there is a strong indication of the new protein's biochemical function. The most fundamental relationship between two entities is homology; two molecules are said to be homologous if they have been derived from a common ancestor. Homologous molecules, or homologs, can be divided into two classes, paralogs and orthologs. Paralogs are homologs that are present within one species. Paralogs often differ in their detailed biochemical functions. Orthologs are homologs that are present within different species and have very similar or identical functions. A protein superfamily is the largest grouping (clade) of proteins for which common ancestry can be inferred. Usually this common ancestry is based on sequence alignment and mechanistic similarity. Superfamilies typically contain several protein families which show sequence similarity within the family. The term "protein clan" is commonly used for protease superfamilies based on the MEROPS protease classification system.

The CLUSTAL W algorithm is another example of a sequence alignment algorithm (See, Thompson et al., Nucleic Acids Res, 22:4673-4680, 1994). Default parameters for the CLUSTAL W algorithm include: Gap opening penalty = 10.0; Gap extension penalty = 0.05; Protein weight matrix = BLOSUM series; DNA weight matrix = IUB; Delay divergent sequences % = 40; Gap separation distance = 8; DNA transitions weight = 0.50; List hydrophilic residues = GPSNDQEKR; Use negative matrix = OFF; Toggle Residue specific penalties = ON; Toggle hydrophilic penalties = ON; and Toggle end gap separation penalty = OFF. In CLUSTAL algorithms, deletions occurring at either terminus are included. For example, a variant with a five amino acid deletion at either terminus (or within the polypeptide) of a polypeptide of 500 amino acids would have a percent sequence identity of 99% (495/500 identical residues × 100) relative to the "reference" polypeptide. Such a variant would be encompassed by a variant having "at least 99% sequence identity" to the polypeptide.

A nucleic acid or polynucleotide is "isolated" when it is at least partially or completely separated from other components, including but not limited to for example, other proteins, nucleic acids, cells, etc. Similarly, a polypeptide, protein or peptide is "isolated" when it is at least partially or completely separated from other components, including but not limited to for example, other proteins, nucleic acids, cells, etc. On a molar basis, an isolated species is more abundant than are other species in a composition. For example, an isolated species may comprise at least about 60%, about 65%, about 70%, about 75%, about 80%, about 85%, about 90%, about 91%, about 92%, about 93%, about 94%, about 95%, about 96%, about 97%, about 98%, about 99%, or about 100% (on a molar basis) of all macromolecular species present. Preferably, the species of interest is purified to essential homogeneity (i.e., contaminant species cannot be detected in the composition by conventional detection methods). Purity and homogeneity can be determined using a number of techniques well known in the art, such as agarose or polyacrylamide gel electrophoresis of a nucleic acid or a protein sample, respectively, followed by visualization upon staining. If desired, a high-resolution technique, such as high performance liquid chromatography (HPLC) or a similar means can be utilized for purification of the material.

The term "purified" as applied to nucleic acids or polypeptides generally denotes a nucleic acid or polypeptide that is essentially free from other components as determined by analytical techniques well known in the art (e.g., a purified polypeptide or polynucleotide forms a discrete band in an electrophoretic gel, chromatographic eluate, and/or a media subjected to density gradient centrifugation). For example, a nucleic acid or polypeptide that gives rise to essentially one band in an electrophoretic gel is "purified." A purified nucleic acid or polypeptide is at least about 50% pure, usually at least about 60%, about 65%, about 70%, about 75%, about 80%, about 85%, about 90%, about 91%, about 92%, about 93%, about 94%, about 95%, about 96%, about 97%, about 98%, about 99%, about 99.5%, about 99.6%, about 99.7%, about 99.8% or more pure (e.g., percent by weight on a molar basis). In a related sense, a composition is enriched for a molecule when there is a substantial increase in the concentration of the molecule after application of a purification or enrichment technique. The term "enriched" refers to a compound, polypeptide, cell, nucleic acid, amino acid, or other specified material or component that is present in a composition at a relative or absolute concentration that is higher than a starting composition.

As used herein, the term "functional assay" refers to an assay that provides an indication of a protein's activity. In some embodiments, the term refers to assay systems in which a protein is analyzed for its ability to function in its usual capacity. For example, in the case of a protease, a functional assay involves determining the effectiveness of the protease to hydrolyze a proteinaceous substrate.

The term "cleaning activity" refers to a cleaning performance achieved by a serine protease polypeptide or reference protease under conditions prevailing during the proteolytic, hydrolyzing, cleaning, or other process of the disclosure. In some embodiments, cleaning performance of a serine protease polypeptide or reference protease may be determined by using various assays for cleaning one or more various enzyme sensitive stains on an item or surface (e.g., a stain resulting from food, grass, blood, ink, milk, oil, and/or egg protein). Cleaning performance of a variant or reference protease can be determined by subjecting the stain on the item or surface to standard wash condition(s) and assessing the degree to which the stain is removed by using various chromatographic, spectrophotometric, or other quantitative methodologies. Exemplary cleaning assays and methods are known in the art and include, but are not limited to those described in WO99/34011 and U.S. Pat. 6,605,458 , as well as those cleaning assays and methods included in the Examples provided below.

The term "cleaning effective amount" of a serine protease polypeptide or reference protease refers to the amount of protease that achieves a desired level of enzymatic activity in a specific cleaning composition. Such effective amounts are readily ascertained by one of ordinary skill in the art and are based on many factors, such as the particular protease used, the cleaning application, the specific composition of the cleaning composition, and whether a liquid or dry (e.g., granular, tablet, bar) composition is required, etc.

The term "cleaning adjunct material" refers to any liquid, solid, or gaseous material included in cleaning composition other than a serine protease polypeptide of the disclosure. In some embodiments, the cleaning compositions of the present disclosure include one or more cleaning adjunct materials. Each cleaning adjunct material is typically selected depending on the particular type and form of cleaning composition (e.g., liquid, granule, powder, bar, paste, spray, tablet, gel, foam, or other composition). Preferably, each cleaning adjunct material is compatible with the protease enzyme used in the composition.

Cleaning compositions and cleaning formulations include any composition that is suited for cleaning, bleaching, disinfecting, and/or sterilizing any object, item, and/or surface. Such compositions and formulations include, but are not limited to for example, liquid and/or solid compositions, including cleaning or detergent compositions (e.g., liquid, tablet, gel, bar, granule, and/or solid laundry cleaning or detergent compositions and fine fabric detergent compositions; hard surface cleaning compositions and formulations, such as for glass, wood, ceramic and metal counter tops and windows; carpet cleaners; oven cleaners; fabric fresheners; fabric softeners; and textile, laundry booster cleaning or detergent compositions, laundry additive cleaning compositions, and laundry pre-spotter cleaning compositions; dishwashing compositions, including hand or manual dishwashing compositions (e.g., "hand" or "manual" dishwashing detergents) and automatic dishwashing compositions (e.g., "automatic dishwashing detergents"). Single dosage unit forms also find use with the present invention, including but not limited to pills, tablets, gelcaps, or other single dosage units such as pre-measured powders, suspensions, or liquids.

Cleaning composition or cleaning formulations, as used herein, include, unless otherwise indicated, granular or powder-form all-purpose or heavy-duty washing agents, especially cleaning detergents; liquid, granular, gel, solid, tablet, paste, or unit dosage form all-purpose washing agents, especially the so-called heavy-duty liquid (HDL) or heavy-duty dry (HDD) detergent types; liquid fine-fabric detergents; hand or manual dishwashing agents, including those of the high-foaming type; hand or manual dishwashing, automatic dishwashing, or dishware or tableware washing agents, including the various tablet, powder, solid, granular, liquid, gel, and rinse-aid types for household and institutional use; liquid cleaning and disinfecting agents, including antibacterial hand-wash types, cleaning bars, mouthwashes, denture cleaners, car shampoos, carpet shampoos, bathroom cleaners; hair shampoos and/or hair-rinses for humans and other animals; shower gels and foam baths and metal cleaners; as well as cleaning auxiliaries, such as bleach additives and "stain-stick" or pre-treat types. In some embodiments, granular compositions are in "compact" form; in some embodiments, liquid compositions are in a "concentrated" form.

As used herein, "fabric cleaning compositions" include hand and machine laundry detergent compositions including laundry additive compositions and compositions suitable for use in the soaking and/or pretreatment of stained fabrics (e.g., clothes, linens, and other textile materials).

As used herein, "non-fabric cleaning compositions" include non-textile (i.e., non-fabric) surface cleaning compositions, including, but not limited to for example, hand or manual or automatic dishwashing detergent compositions, oral cleaning compositions, denture cleaning compositions, contact lens cleaning compositions, wound debridement compositions, and personal cleansing compositions.

As used herein, the term "detergent composition" or "detergent formulation" is used in reference to a composition intended for use in a wash medium for the cleaning of soiled or dirty objects, including particular fabric and/or non-fabric objects or items. Such compositions of the present disclosure are not limited to any particular detergent composition or formulation. Indeed, in some embodiments, the detergents of the disclosure comprise at least one serine protease polypeptide of the disclosure and, in addition, one or more surfactants, transferase(s), hydrolytic enzymes, oxido reductases, builders (e.g., a builder salt), bleaching agents, bleach activators, bluing agents, fluorescent dyes, caking inhibitors, masking agents, enzyme activators, antioxidants, and/or solubilizers. In some instances, a builder salt is a mixture of a silicate salt and a phosphate salt, preferably with more silicate (e.g., sodium metasilicate) than phosphate (e.g., sodium tripolyphosphate). Some compositions of the disclosure, such as, but not limited to, cleaning compositions or detergent compositions, do not contain any phosphate (e.g., phosphate salt or phosphate builder).

As used herein, the term "bleaching" refers to the treatment of a material (e.g., fabric, laundry, pulp, etc.) or surface for a sufficient length of time and/or under appropriate pH and/or temperature conditions to effect a brightening (i.e., whitening) and/or cleaning of the material. Examples of chemicals suitable for bleaching include, but are not limited to, for example, ClO₂, H₂O₂, peracids, NO₂, etc.

As used herein, "wash performance" of a protease (e.g., a serine protease polypeptide of the disclosure) refers to the contribution of a serine protease polypeptide to washing that provides additional cleaning performance to the detergent as compared to the detergent without the addition of the serine protease polypeptide to the composition. Wash performance is compared under relevant washing conditions. In some test systems, other relevant factors, such as detergent composition, SUD concentration, water hardness, washing mechanics, time, pH, and/or temperature, can be controlled in such a way that condition(s) typical for household application in a certain market segment (e.g., hand or manual dishwashing, automatic dishwashing, dishware cleaning, tableware cleaning, fabric cleaning, etc.) are imitated.

The term "relevant washing conditions" is used herein to indicate the conditions, particularly washing temperature, time, washing mechanics, SUD concentration, type of detergent and water hardness, actually used in households in a hand dishwashing, automatic dishwashing, or laundry detergent market segment.

The term "improved wash performance" is used to indicate that a better end result is obtained in stain removal under relevant washing conditions, or that less serine protease polypeptide of the disclosure, on weight basis, is needed to obtain the same end result relative to the corresponding wild-type or starting parent protease.

As used herein, the term "disinfecting" refers to the removal of contaminants from the surfaces, as well as the inhibition or killing of microbes on the surfaces of items. It is not intended that the present disclosure be limited to any particular surface, item, or contaminant(s) or microbes to be removed.

The "compact" form of the cleaning compositions herein is best reflected by density and, in terms of composition, by the amount of inorganic filler salt. Inorganic filler salts are conventional ingredients of detergent compositions in powder form. In conventional detergent compositions, the filler salts are present in substantial amounts, typically about 17 to about 35% by weight of the total composition. In contrast, in compact compositions, the filler salt is present in amounts not exceeding about 15% of the total composition. In some embodiments, the filler salt is present in amounts that do not exceed about 10%, or more preferably, about 5%, by weight of the composition. In some embodiments, the inorganic filler salts are selected from the alkali and alkaline-earth-metal salts of sulfates and chlorides. In some embodiments, the filler salt is sodium sulfate.

### II. Serine Protease Polypeptides

The present disclosure provides novel serine protease enzymes, as defined in the claims. The serine protease polypeptides of the present disclosure include isolated, recombinant, substantially pure, or non-naturally occurring polypeptides. In some embodiments, the polypeptides are useful in cleaning applications and can be incorporated into cleaning compositions that are useful in methods of cleaning an item or a surface in need thereof.

In some embodiments, the polypeptide of the present invention, or the active fragment, can be a WHY-clade polypeptide, provided that the polypeptide is as defined in the claims. The WHY-clade derives from the complete conserved residues WHY near the N-terminus (W residue position 7 in BspAG00296, BspM04033 and other members of this clade).

In some embodiments, the polypeptide of the present invention, or an active fragment thereof, can be a novel polypeptide or any of the above described polypeptides of the present invention, wherein the recombinant polypeptide or an active fragment thereof comprises a DTGIDXXHXXLXNLVXTSLGXSXVGGXXXDVXGH motif, wherein the initial D is the active site Aspartic acid, the terminal H is the active site Histidine, and X is any amino acid, and wherein the polypeptide is as defined in the claims.

In some embodiments the recombinant polypeptide or the active fragment is as defined in claim 6. In some embodiments, the substitution is selected from the group consisting of P003N, Q006R, N010E, T020I, S026N, I028R, Q029I, H038A, Q041P, S042N, A044R, N048D, Q053R, S059G, M061G, H085Q, T088R, V090I, N096G, S098N, L103M, F104Y, T107Q, S113A, D115S, G117N, N131D, Q132S, S133D, A136N, A137N, A138I, Q139N, N143S, A144S, S146T, I147L, A157R, S168N, V169A, T178N, G179R, A180T, V204Y, N207G, G208Q, Y209F, A210R, F212L, S219T, A222V, N229I, R230K, A231S, V231A, S239T, N240Q, A241V, S243N, M245L, Q246R, N247D, P255L, T256N, F257Q, D264N, N266Y, Q271A, and S273G.

In some embodiments, the invention is a recombinant polypeptide comprising an amino acid sequence of SEQ ID NO:3 or SEQ ID NO:4.

As noted above, the variant enzyme polypeptides of the invention have enzymatic activities (protease activities) and thus are useful in cleaning applications, including but not limited to, methods for cleaning dishware items, tableware items, fabrics, and items having hard surfaces (e.g., the hard surface of a table, table top, wall, furniture item, floor, ceiling, etc.). Exemplary cleaning compositions comprising one or more variant serine protease enzyme polypeptides of the invention are described infra. The enzymatic activity (protease enzyme activity) of an enzyme polypeptide of the invention can be determined readily using procedures well known to those of ordinary skill in the art. The Examples presented infra describe methods for evaluating the enzymatic activity and cleaning performance. The performance of polypeptide enzymes of the invention in removing stains (e.g., a protein stain such as blood/milk/ink or egg yolk), cleaning hard surfaces, or cleaning laundry, dishware or tableware item(s) can be readily determined using procedures well known in the art and/or by using procedures set forth in the Examples.

The serine protease polypeptides of the present invention can have protease activity over a broad range of pH conditions. In some embodiments, the serine protease polypeptides have protease activity on dimethylcasein as a substrate, as demonstrated in Example 7. In some embodiments, the serine protease polypeptides have protease activity at a pH of from about 4.0 to about 12.0. In some embodiments, the serine protease polypeptides have protease activity at a pH of from about 6.0 to about 12.0. In some embodiments, the serine protease polypeptides have at least 50%, 60%, 70%, 80% or 90% of maximal protease activity at a pH of from about 6.0 to about 12.0, or from about 7.0 to about 12.0. In some embodiments, the serine protease polypeptides have protease activity at a pH above 6.0, 6.5, 7.0, 7.5, 8.0, 8.5, 9.0, 9.5, 10.0, 10.5, 11.0 or 11.5. In some embodiments, the serine protease polypeptides have protease activity at a pH below 12.0, 11.5, 11.0, 10.5, 10.0, 9.5, 9.0, 8.5, 8.0, 7.5, 7.0, or 6.5.

In some embodiments, the serine protease polypeptides of the present invention have protease activity at a temperature range from about 10°C to about 90°C, or from about 30°C to about 80°C. In some embodiments, the serine protease polypeptides of the present invention have protease activity at a temperature range of from about 55°C to about 75°C. In some embodiments, the serine protease polypeptides have at least 50%, 60%, 70%, 80% or 90% of maximal protease activity at a temperature of from about 55°C to about 75°C. In some embodiments, the serine proteases have activity at a temperature above 50°C, 55°C, 60°C, 65°C, or 70°C. In some embodiments, the serine proteases have activity at a temperature below 75°C, 80°C, 70°C, 65°C, 60°C, or 55°C.

The serine protease polypeptides of the present invention have at least 80% activity after 20 minutes at 50°C under stressed conditions comprising a Tris/EDTA assay. The stressed conditions can be, for example, those shown in Example 11.

In some embodiments, the serine protease polypeptides of the present invention demonstrate cleaning performance in a cleaning composition. Cleaning compositions often include ingredients harmful to the stability and performance of enzymes, making cleaning compositions a harsh environment for enzymes, e.g. serine proteases, to retain function. Thus, it is not trivial for an enzyme to be put in a cleaning composition and expect enzymatic function (e.g. serine protease activity, such as demonstrated by cleaning performance). In some embodiments, the serine protease polypeptides of the present invention demonstrate cleaning performance in automatic dishwashing (ADW) detergent compositions. In some embodiments, the cleaning performance in automatic dishwashing (ADW) detergent compositions includes cleaning of egg yolk stains. In some embodiments, the serine protease polypeptides of the present invention demonstrate cleaning performance in laundry detergent compositions. In some embodiments, the cleaning performance in laundry detergent compositions includes cleaning of blood/milk/ink stains. In each of the cleaning compositions, the serine protease polypeptides of the present invention demonstrate cleaning performance with or without a bleach component.

A polypeptide of the invention can be subject to various changes, such as one or more amino acid insertions, deletions, and/or substitutions, either conservative or non-conservative, including where such changes do not substantially alter the enzymatic activity of the polypeptide, provided that the polypeptide is as defined in the claims. Similarly, a nucleic acid of the invention can also be subject to various changes, such as one or more substitutions of one or more nucleotides in one or more codons such that a particular codon encodes the same or a different amino acid, resulting in either a silent variation (e.g., when the encoded amino acid is not altered by the nucleotide mutation) or non-silent variation, one or more deletions of one or more nucleic acids (or codons) in the sequence, one or more additions or insertions of one or more nucleic acids (or codons) in the sequence, and/or cleavage of or one or more truncations of one or more nucleic acids (or codons) in the sequence, provided that the polynucleotide is as defined in the claims. Many such changes in the nucleic acid sequence may not substantially alter the enzymatic activity of the resulting encoded polypeptide enzyme compared to the polypeptide enzyme encoded by the original nucleic acid sequence. A nucleic acid sequence of the invention can also be modified to include one or more codons that provide for optimum expression in an expression system (e.g., bacterial expression system), while, if desired, said one or more codons still encode the same amino acid(s).

In some embodiments, the present invention provides a genus of enzyme polypeptides having the desired enzymatic activity (e.g., protease enzyme activity or cleaning performance activity) which comprise sequences having the amino acid substitutions described herein and also which comprise one or more additional amino acid substitutions, such as conservative and non-conservative substitutions, wherein the polypeptide exhibits, maintains, or approximately maintains the desired enzymatic activity (e.g., proteolytic activity, as reflected in the cleaning activity or performance of the polypeptide enzyme of SEQ ID NO:3, SEQ ID NO:4, SEQ ID NO:7, SEQ ID NO:10, SEQ ID NO:11, SEQ ID NO:14, SEQ ID NO:15, SEQ ID NO:22, SEQ ID NO:25, SEQ ID NO:28, SEQ ID NO:31, SEQ ID NO:34, SEQ ID NO:37, SEQ ID NO:40, SEQ ID NO:43, or SEQ ID NO:44), provided that the polypeptides are as defined in the claims. Amino acid substitutions in accordance with the invention may include, but are not limited to, one or more non-conservative substitutions and/or one or more conservative amino acid substitutions. A conservative amino acid residue substitution typically involves exchanging a member within one functional class of amino acid residues for a residue that belongs to the same functional class (conservative amino acid residues are considered functionally homologous or conserved in calculating percent functional homology). A conservative amino acid substitution typically involves the substitution of an amino acid in an amino acid sequence with a functionally similar amino acid. For example, alanine, glycine, serine, and threonine are functionally similar and thus may serve as conservative amino acid substitutions for one another. Aspartic acid and glutamic acid may serve as conservative substitutions for one another. Asparagine and glutamine may serve as conservative substitutions for one another. Arginine, lysine, and histidine may serve as conservative substitutions for one another. Isoleucine, leucine, methionine, and valine may serve as conservative substitutions for one another. Phenylalanine, tyrosine, and tryptophan may serve as conservative substitutions for one another.

Other conservative amino acid substitution groups can be envisioned. For example, amino acids can be grouped by similar function or chemical structure or composition (e.g., acidic, basic, aliphatic, aromatic, sulfur-containing). For instance, an aliphatic grouping may comprise: Glycine (G), Alanine (A), Valine (V), Leucine (L), Isoleucine (I). Other groups containing amino acids that are considered conservative substitutions for one another include: aromatic: Phenylalanine (F), Tyrosine (Y), Tryptophan (W); sulfur-containing: Methionine (M), Cysteine (C); Basic: Arginine (R), Lysine (K), Histidine (H); Acidic: Aspartic acid (D), Glutamic acid (E); non-polar uncharged residues, Cysteine (C), Methionine (M), and Proline (P); hydrophilic uncharged residues: Serine (S), Threonine (T), Asparagine (N), and Glutamine (Q). Additional groupings of amino acids are well-known to those of skill in the art and described in various standard textbooks. Listing of a polypeptide sequence herein, in conjunction with the above substitution groups, provides an express listing of all conservatively substituted polypeptide sequences.

More conservative substitutions exist within the amino acid residue classes described above, which also or alternatively can be suitable. Conservation groups for substitutions that are more conservative include: valine-leucine-isoleucine, phenylalanine-tyrosine, lysine-arginine, alanine-valine, and asparagine-glutamine.

Conservatively substituted variations of a polypeptide sequence of the invention (e.g., variant serine proteases of the invention) include substitutions of a small percentage, sometimes less than 5%, 4%, 3%, 2%, or 1%, or less than 10, 9, 8, 7, 6, 5, 4, 3, 2, or 1 amino acid substitutions of the amino acids of the polypeptide sequence, with a conservatively selected amino acid of the same conservative substitution group, provided that the polypeptide is as defined in the claims.

### III. Nucleic Acids Encoding Serine Proteases

The invention provides isolated, non-naturally occurring, or recombinant nucleic acids (also referred to herein as "polynucleotides"), which may be collectively referred to as "nucleic acids of the invention" or "polynucleotides of the invention", which encode polypeptides of the invention, provided that the polynucleotides are as defined in the claims. Nucleic acids of the invention, including all described below, are useful in recombinant production (e.g., expression) of polypeptides of the invention, typically through expression of a plasmid expression vector comprising a sequence encoding the polypeptide of interest or fragment thereof. As discussed above, polypeptides include serine protease polypeptides having enzymatic activity (e.g., proteolytic activity) which are useful in cleaning applications and cleaning compositions for cleaning an item or a surface (e.g., surface of an item) in need of cleaning.

Nucleic acids of the invention can be generated by using any suitable synthesis, manipulation, and/or isolation techniques, or combinations thereof. For example, a polynucleotide of the invention may be produced using standard nucleic acid synthesis techniques, such as solid-phase synthesis techniques that are well-known to those skilled in the art. In such techniques, fragments of up to 50 or more nucleotide bases are typically synthesized, then joined (e.g., by enzymatic or chemical ligation methods, or polymerase mediated recombination methods) to form essentially any desired continuous nucleic acid sequence. The synthesis of the nucleic acids of the invention can be also facilitated by any suitable method known in the art, including but not limited to chemical synthesis using the classical phosphoramidite method (See e.g., Beaucage et al. Tetrahedron Letters 22:1859-69 [1981]); or the method described by Matthes et al. (See, Matthes et al., EMBO J. 3:801-805 [1984], as is typically practiced in automated synthetic methods. Nucleic acids of the invention also can be produced by using an automatic DNA synthesizer. Customized nucleic acids can be ordered from a variety of commercial sources (e.g., The Midland Certified Reagent Company, the Great American Gene Company, Operon Technologies Inc., and DNA2.0). Other techniques for synthesizing nucleic acids and related principles are known in the art (See e.g., Itakura et al., Ann. Rev. Biochem. 53:323 [1984]; and Itakura et al., Science 198:1056 [1984]).

As indicated above, recombinant DNA techniques useful in modification of nucleic acids are well known in the art. For example, techniques such as restriction endonuclease digestion, ligation, reverse transcription and cDNA production, and polymerase chain reaction (e.g., PCR) are known and readily employed by those of skill in the art. Nucleotides of the invention may also be obtained by screening cDNA libraries using one or more oligonucleotide probes that can hybridize to or PCR-amplify polynucleotides which encode a serine protease polypeptide polypeptide(s) of the invention. Procedures for screening and isolating cDNA clones and PCR amplification procedures are well known to those of skill in the art and described in standard references known to those skilled in the art. Some nucleic acids of the invention can be obtained by altering a naturally occurring polynucleotide backbone (e.g., that encodes an enzyme or parent protease) by, for example, a known mutagenesis procedure (e.g., site-directed mutagenesis, site saturation mutagenesis, and in vitro recombination). A variety of methods are known in the art that are suitable for generating modified polynucleotides of the invention that encode serine protease polypeptides of the invention, including, but not limited to, for example, site-saturation mutagenesis, scanning mutagenesis, insertional mutagenesis, deletion mutagenesis, random mutagenesis, site-directed mutagenesis, and directed-evolution, as well as various other recombinatorial approaches.

### IV. Vectors, Host Cells, and Methods for Producing Serine Proteases

The present invention provides isolated or recombinant vectors, as defined in the claims.

In some embodiments, the invention provides recombinant cells, as defined in the claims, comprising at least one vector (e.g., expression vector or DNA construct) of the invention which comprises at least one nucleic acid or polynucleotide of the invention. Some such recombinant cells are transformed or transfected with such at least one vector. Such cells are typically referred to as host cells. Some such cells comprise bacterial cells, including, but are not limited to Bacillus sp. cells, such as B. subtilis cells. The invention also provides recombinant cells (e.g., recombinant host cells) comprising at least one serine protease polypeptide of the invention.

In some embodiments, the invention provides a vector comprising a nucleic acid or polynucleotide of the invention. In some embodiments, the vector is an expression vector or expression cassette in which a polynucleotide sequence of the invention which encodes a serine protease polypeptide of the invention is operably linked to one or additional nucleic acid segments required for efficient gene expression (e.g., a promoter operably linked to the polynucleotide of the invention which encodes a serine protease polypeptide of the invention). A vector may include a transcription terminator and/or a selection gene, such as an antibiotic resistance gene, that enables continuous cultural maintenance of plasmid-infected host cells by growth in antimicrobial-containing media.

An expression vector may be derived from plasmid or viral DNA, or in alternative embodiments, contains elements of both. Exemplary vectors include, but are not limited to pC194, pJH101, pE194, pHP13 (See, Harwood and Cutting [eds.], Chapter 3, Molecular Biological Methods for Bacillus, John Wiley & Sons [1990]; suitable replicating plasmids for B. subtilis include those listed on p. 92) See also, Perego, Integrational Vectors for Genetic Manipulations in *B. subtilis,* in Sonenshein et al., [eds.] B. subtilis and Other Gram-Positive Bacteria: Biochemistry, Physiology and Molecular Genetics, American Society for Microbiology, Washington, D.C. [1993], pp. 615-624), and p2JM103BBI.

For expression and production of a protein of interest (e.g., serine protease polypeptide) in a cell, at least one expression vector comprising at least one copy of a polynucleotide encoding the serine protease polypeptide, and in some instances comprising multiple copies, is transformed into the cell under conditions suitable for expression of the serine protease. In some embodiments of the present invention, a polynucleotide sequence encoding the serine protease polypeptide (as well as other sequences included in the vector) is integrated into the genome of the host cell, while in other embodiments, a plasmid vector comprising a polynucleotide sequence encoding the serine protease polypeptide remains as autonomous extrachromosomal element within the cell. The invention provides both extrachromosomal nucleic acid elements as well as incoming nucleotide sequences that are integrated into the host cell genome. The vectors described herein are useful for production of the serine protease polypeptides of the invention. In some embodiments, a polynucleotide construct encoding the serine protease polypeptide is present on an integrating vector that enables the integration and optionally the amplification of the polynucleotide encoding the serine protease polypeptide into the host chromosome. Examples of sites for integration are well known to those skilled in the art. In some embodiments, transcription of a polynucleotide encoding a serine protease polypeptide of the invention is effectuated by a promoter that is the wild-type promoter for the selected precursor protease. In some other embodiments, the promoter is heterologous to the precursor protease, but is functional in the host cell. Specifically, examples of suitable promoters for use in bacterial host cells include, but are not limited to, for example, the amyE, amyQ, amyL, pstS, sacB, SPAC, AprE, Veg, HpaII promoters, the promoter of the B. stearothermophilus maltogenic amylase gene, the B. amyloliquefaciens (BAN) amylase gene, the B. subtilis alkaline protease gene, the B. clausii alkaline protease gene the B. pumilis xylosidase gene, the B. thuringiensis cryIIIA, and the B. licheniformis alpha-amylase gene. Additional promoters include, but are not limited to the A4 promoter, as well as phage Lambda PR or PL promoters, and the E. coli lac, trp or tac promoters.

Serine protease polypeptides of the present invention can be produced in host cells of any suitable microorganism, including bacteria and fungi. For example, in some embodiments, serine protease polypeptides of the present invention can be produced in Gram-positive bacteria. In some embodiments, the host cells are *Bacillus* spp., *Streptomyces* spp., *Escherichia* spp., *Aspergillus* spp., *Trichoderma* spp., *Pseudomonas* spp., *Corynebacterium* spp., *Saccharomyces* spp., or *Pichia* spp. In some embodiments, the serine protease polypeptides are produced by *Bacillus* sp. host cells. Examples of *Bacillus* sp. host cells that find use in the production of the serine protease polypeptides of the invention include, but are not limited to *B. licheniformis, B. lentus, B. subtilis, B. amyloliquefaciens, B. lentus, B. sonorensis, B. brevis, B. stearothermophilus, B. alkalophilus, B. coagulans, B. circulans, B. pumilis, B. thuringiensis, B. clausii,* and *B. megaterium,* as well as other organisms within the genus *Bacillus.* In some embodiments, B. subtilis host cells are used for production of serine protease polypeptides. U.S. Patents 5,264,366 and 4,760,025 (RE 34,606) describe various *Bacillus* host strains that can be used for producing serine protease polypeptide of the invention, although other suitable strains can be used.

Several industrial bacterial strains that can be used to produce serine protease polypeptides of the invention include non-recombinant (i.e., wild-type) *Bacillus* sp. strains, as well as variants of naturally-occurring strains and/or recombinant strains. In some embodiments, the host strain is a recombinant strain, wherein a polynucleotide encoding a polypeptide of interest has been introduced into the host. In some embodiments, the host strain is a *B. subtilis* host strain and particularly a recombinant *Bacillus* subtilis host strain. Numerous *B. subtilis* strains are known, including, but not limited to for example, 1A6 (ATCC 39085), 168 (1A01), SB19, W23, Ts85, B637, PB1753 through PB1758, PB3360, JH642, 1A243 (ATCC 39,087), ATCC 21332, ATCC 6051, MI113, DE100 (ATCC 39,094), GX4931, PBT 110, and PEP 211strain (See e.g., Hoch et al., Genetics 73:215-228 [1973]; See also, U.S. Patent Nos. 4,450,235 and 4,302,544, and EP 0134048). The use of B. subtilis as an expression host cells is well known in the art (See e.g., Palva et al., Gene 19:81-87 [1982]; Fahnestock and Fischer, J. Bacteriol., 165:796-804 [1986]; and Wang et al., Gene 69:39-47 [1988]).

In some embodiments, the *Bacillus* host cell is a *Bacillus* sp. that includes a mutation or deletion in at least one of the following genes, degU, degS, degR and degQ. In some embodiments, the mutation is in a degU gene, and in some embodiments the mutation is degU(Hy)32 (See e.g., Msadek et al., J. Bacteriol. 172:824-834 [1990]; and Olmos et al., Mol. Gen. Genet. 253:562-567 [1997]). In some embodiments, the *Bacillus* host comprises a mutation or deletion in scoC4 (See e.g., Caldwell et al., J. Bacteriol. 183:7329-7340 [2001]); spoIIE (See e.g., Arigoni et al., Mol. Microbiol. 31:1407-1415 [1999]); and/or oppA or other genes of the opp operon (See e.g., Perego et al., Mol. Microbiol. 5:173-185 [1991]). Indeed, it is contemplated that any mutation in the opp operon that causes the same phenotype as a mutation in the oppA gene will find use in some embodiments of the altered *Bacillus* strain of the invention. In some embodiments, these mutations occur alone, while in other embodiments, combinations of mutations are present. In some embodiments, an altered *Bacillus* host cell strain that can be used to produce a serine protease polypeptide of the invention is a *Bacillus* host strain that already includes a mutation in one or more of the above-mentioned genes. In addition, *Bacillus* sp. host cells that comprise mutation(s) and/or deletions of endogenous protease genes find use. In some embodiments, the *Bacillus* host cell comprises a deletion of the *aprE* and the *nprE* genes. In other embodiments, the *Bacillus* sp. host cell comprises a deletion of 5 protease genes, while in other embodiments, the *Bacillus* sp. host cell comprises a deletion of 9 protease genes (See e.g., U.S. Pat. Appln. Pub. No. 2005/0202535).

Host cells are transformed with at least one nucleic acid encoding at least one serine protease polypeptide of the invention using any suitable method known in the art. Methods for introducing a nucleic acid (e.g., DNA) into *Bacillus* cells or *E. coli* cells utilizing plasmid DNA constructs or vectors and transforming such plasmid DNA constructs or vectors into such cells are well known. In some embodiments, the plasmids are subsequently isolated from *E. coli* cells and transformed into *Bacillus* cells. However, it is not essential to use intervening microorganisms such as *E. coli,* and in some embodiments, a DNA construct or vector is directly introduced into a *Bacillus* host.

Those of skill in the art are well aware of suitable methods for introducing nucleic acid or polynucleotide sequences of the invention into *Bacillus* cells (See e.g., Ferrari et al., "Genetics," in Harwood et al. [eds.], Bacillus, Plenum Publishing Corp. [1989], pp. 57-72; Saunders et al., J. Bacteriol. 157:718-726 [1984]; Hoch et al., J. Bacteriol. 93:1925 -1937 [1967]; Mann et al., Current Microbiol. 13:131-135 [1986]; Holubova, Folia Microbiol. 30:97 [1985]; Chang et al., Mol. Gen. Genet. 168:11-115 [1979]; Vorobjeva et al., FEMS Microbiol. Lett. 7:261-263 [1980]; Smith et al., Appl. Env. Microbiol. 51:634 [1986]; Fisher et al., Arch. Microbiol. 139:213-217 [1981]; and McDonald, J. Gen. Microbiol. 130:203 [1984]). Indeed, such methods as transformation, including protoplast transformation and congression, transduction, and protoplast fusion are well known and suited for use in the present invention. Methods of transformation are used to introduce a DNA construct or vector comprising a nucleic acid encoding a serine protease polypeptide of the present invention into a host cell. Methods known in the art to transform *Bacillus* cells include such methods as plasmid marker rescue transformation, which involves the uptake of a donor plasmid by competent cells carrying a partially homologous resident plasmid (See, Contente et al., Plasmid 2:555-571 [1979]; Haima et al., Mol. Gen. Genet. 223:185-191 [1990]; Weinrauch et al., J. Bacteriol. 154:1077-1087 [1983]; and Weinrauch et al., J. Bacteriol. 169:1205-1211 [1987]). In this method, the incoming donor plasmid recombines with the homologous region of the resident "helper" plasmid in a process that mimics chromosomal transformation.

In addition to commonly used methods, in some embodiments, host cells are directly transformed with a DNA construct or vector comprising a nucleic acid encoding a serine protease polypeptide of the invention (i.e., an intermediate cell is not used to amplify, or otherwise process, the DNA construct or vector prior to introduction into the host cell). Introduction of the DNA construct or vector of the invention into the host cell includes those physical and chemical methods known in the art to introduce a nucleic acid sequence (e.g., DNA sequence) into a host cell without insertion into a plasmid or vector. Such methods include, but are not limited to calcium chloride precipitation, electroporation, naked DNA, liposomes and the like. In additional embodiments, DNA constructs or vector are co-transformed with a plasmid, without being inserted into the plasmid. In further embodiments, a selective marker is deleted from the altered *Bacillus* strain by methods known in the art (See, Stahl et al., J. Bacteriol. 158:411-418 [1984]; and Palmeros et al., Gene 247:255 -264 [2000]).

In some embodiments, the transformed cells of the present invention are cultured in conventional nutrient media. The suitable specific culture conditions, such as temperature, pH and the like are known to those skilled in the art and are well described in the scientific literature. In some embodiments, the invention provides a culture (e.g., cell culture) comprising at least one serine protease polypeptide or at least one nucleic acid of the invention. Also provided are compositions comprising at least one nucleic acid, vector, or DNA construct of the invention.

In some embodiments, host cells transformed with at least one polynucleotide sequence encoding at least one serine protease polypeptide of the invention are cultured in a suitable nutrient medium under conditions permitting the expression of the present protease, after which the resulting protease is recovered from the culture. The medium used to culture the cells comprises any conventional medium suitable for growing the host cells, such as minimal or complex media containing appropriate supplements. Suitable media are available from commercial suppliers or may be prepared according to published recipes (See e.g., the catalogues of the American Type Culture Collection). In some embodiments, the protease produced by the cells is recovered from the culture medium by conventional procedures, including, but not limited to for example, separating the host cells from the medium by centrifugation or filtration, precipitating the proteinaceous components of the supernatant or filtrate by means of a salt (e.g., ammonium sulfate), chromatographic purification (e.g., ion exchange, gel filtration, affinity, etc.). Any method suitable for recovering or purifying a variant protease finds use in the present invention.

In some embodiments, a serine protease polypeptide produced by a recombinant host cell is secreted into the culture medium. A nucleic acid sequence that encodes a purification facilitating domain may be used to facilitate purification of proteins. A vector or DNA construct comprising a polynucleotide sequence encoding a serine protease polypeptide may further comprise a nucleic acid sequence encoding a purification facilitating domain to facilitate purification of the serine protease polypeptide (See e.g., Kroll et al., DNA Cell Biol. 12:441-53 [1993]). Such purification facilitating domains include, but are not limited to, for example, metal chelating peptides such as histidine-tryptophan modules that allow purification on immobilized metals (See, Porath, Protein Expr. Purif. 3:263-281 [1992]), protein A domains that allow purification on immobilized immunoglobulin, and the domain utilized in the FLAGS extension/affinity purification system. The inclusion of a cleavable linker sequence such as Factor XA or enterokinase (e.g., sequences available from Invitrogen, San Diego, CA) between the purification domain and the heterologous protein also find use to facilitate purification.

Assays for detecting and measuring the enzymatic activity of an enzyme, such as a serine protease polypeptide of the invention, are well known. Various assays for detecting and measuring activity of proteases (e.g., serine protease polypeptides of the invention), are also known to those of ordinary skill in the art. In particular, assays are available for measuring protease activity that are based on the release of acid-soluble peptides from casein or hemoglobin, measured as absorbance at 280 nm or colorimetrically using the Folin method, well known to those skilled in the art. Other exemplary assays involve the solubilization of chromogenic substrates (See e.g., Ward, "Proteinases," in Fogarty (ed.)., Microbial Enzymes and Biotechnology, Applied Science, London, [1983], pp. 251-317). Other exemplary assays include, but are not limited to succinyl-Ala-Ala-Pro-Phe-para nitroanilide assay (suc-AAPF-pNA) and the 2,4,6-trinitrobenzene sulfonate sodium salt assay (TNBS assay). Numerous additional references known to those in the art provide suitable methods (See e.g., Wells et al., Nucleic Acids Res. 11:7911-7925 [1983]; Christianson et al., Anal. Biochem. 223:119 -129 [1994]; and Hsia et al., Anal Biochem. 242:221-227 [1999]).

A variety of methods can be used to determine the level of production of a mature protease (e.g., mature serine protease polypeptides of the present invention) in a host cell. Such methods include, but are not limited to, for example, methods that utilize either polyclonal or monoclonal antibodies specific for the protease. Exemplary methods include, but are not limited to enzyme-linked immunosorbent assays (ELISA), radioimmunoassays (RIA), fluorescent immunoassays (FIA), and fluorescent activated cell sorting (FACS). These and other assays are well known in the art (See e.g., Maddox et al., J. Exp. Med. 158:1211 [1983]).

In some other embodiments, the invention provides methods for making or producing a mature serine protease polypeptide of the invention. A mature serine protease polypeptide does not include a signal peptide or a propeptide sequence. Some methods comprise making or producing a serine protease polypeptide of the invention in a recombinant bacterial host cell, such as for example, a Bacillus sp. cell (e.g., a B. subtilis cell). In some embodiments, the invention provides a method of producing a serine protease polypeptide of the invention, the method comprising cultivating a recombinant host cell comprising a recombinant expression vector comprising a nucleic acid encoding a serine protease polypeptide of the invention under conditions conducive to the production of the serine protease polypeptide. Some such methods further comprise recovering the serine protease polypeptide from the culture.

In some embodiments the invention provides methods of producing a serine protease polypeptide of the invention, the methods comprising: (a) introducing a recombinant expression vector comprising a nucleic acid encoding a serine protease polypeptide of the invention into a population of cells (e.g., bacterial cells, such as B. subtilis cells); and (b) culturing the cells in a culture medium under conditions conducive to produce the serine protease polypeptide encoded by the expression vector. Some such methods further comprise: (c) isolating the serine protease polypeptide from the cells or from the culture medium.

### V. Compositions Comprising Serine Proteases

### A. Fabric and Home Care Products

Unless otherwise noted, all component or composition levels provided herein are made in reference to the active level of that component or composition, and are exclusive of impurities, for example, residual solvents or by-products, which may be present in commercially available sources. Enzyme components weights are based on total active protein. All percentages and ratios are calculated by weight unless otherwise indicated. All percentages and ratios are calculated based on the total composition unless otherwise indicated. Compositions of the invention include cleaning compositions, such as detergent compositions. In the exemplified detergent compositions, the enzymes levels are expressed by pure enzyme by weight of the total composition and unless otherwise specified, the detergent ingredients are expressed by weight of the total compositions.

While not essential for the purposes of the present invention, the non-limiting list of adjuncts illustrated hereinafter are suitable for use in the instant cleaning compositions. In some embodiments, these adjuncts are incorporated for example, to assist or enhance cleaning performance, for treatment of the substrate to be cleaned, or to modify the aesthetics of the cleaning composition as is the case with perfumes, colorants, dyes or the like. It is understood that such adjuncts are in addition to the serine protease polypeptides of the present invention. The precise nature of these additional components, and levels of incorporation thereof, will depend on the physical form of the composition and the nature of the cleaning operation for which it is to be used. Suitable adjunct materials include, but are not limited to, bleach catalysts, other enzymes, enzyme stabilizing systems, chelants, optical brighteners, soil release polymers, dye transfer agents, dispersants, suds suppressors, dyes, perfumes, colorants, filler salts, , photoactivators, fluorescers, fabric conditioners, hydrolyzable surfactants, preservatives, anti-oxidants, anti-shrinkage agents, anti-wrinkle agents, germicides, fungicides, color speckles, silvercare, anti-tarnish and/or anti-corrosion agents, alkalinity sources, solubilizing agents, carriers, processing aids, pigments, and pH control agents, surfactants, builders, chelating agents, dye transfer inhibiting agents, deposition aids, dispersants, additional enzymes, and enzyme stabilizers, catalytic materials, bleach activators, bleach boosters, hydrogen peroxide, sources of hydrogen peroxide, preformed peracids, polymeric dispersing agents, clay soil removal/anti-redeposition agents, brighteners, suds suppressors, dyes, perfumes, structure elasticizing agents, fabric softeners, carriers, hydrotropes, processing aids and/or pigments. In addition to the disclosure below, suitable examples of such other adjuncts and levels of use are found in U.S. Patent Nos. 5,576,282, 6,306,812, 6,326,348, 6,610,642, 6,605,458, 5,705,464, 5,710,115, 5,698,504, 5,695,679, 5,686,014 and 5,646,101. In embodiments in which the cleaning adjunct materials are not compatible with the serine protease polypeptides of the present invention in the cleaning compositions, then suitable methods of keeping the cleaning adjunct materials and the protease(s) separated (i.e., not in contact with each other) until combination of the two components is appropriate are used. Such separation methods include any suitable method known in the art (e.g., gelcaps, encapsulation, tablets, physical separation, etc.). The aforementioned adjunct ingredients may constitute the balance of the cleaning compositions of the present invention.

The cleaning compositions of the present invention are advantageously employed for example, in laundry applications, hard surface cleaning applications, dishwashing applications, including automatic dishwashing and hand dishwashing, as well as cosmetic applications such as dentures, teeth, hair and skin cleaning. The enzymes of the present invention are also suited for use in contact lens cleaning and wound debridement applications. In addition, due to the unique advantages of increased effectiveness in lower temperature solutions, the enzymes of the present invention are ideally suited for laundry applications. Furthermore, the enzymes of the present invention find use in granular and liquid compositions.

The serine protease polypeptides of the present invention also find use in cleaning additive products. In some embodiments, low temperature solution cleaning applications find use. In some embodiments, the present invention provides cleaning additive products including at least one enzyme of the present invention is ideally suited for inclusion in a wash process when additional bleaching effectiveness is desired. Such instances include, but are not limited to low temperature solution cleaning applications. In some embodiments, the additive product is in its simplest form, one or more proteases. In some embodiments, the additive is packaged in dosage form for addition to a cleaning process. In some embodiments, the additive is packaged in dosage form for addition to a cleaning process where a source of peroxygen is employed and increased bleaching effectiveness is desired. Any suitable single dosage unit form finds use with the present invention, including but not limited to pills, tablets, gelcaps, or other single dosage units such as pre-measured powders or liquids. In some embodiments, filler(s) or carrier material(s) are included to increase the volume of such compositions. Suitable filler or carrier materials include, but are not limited to, various salts of sulfate, carbonate and silicate as well as talc, clay and the like. Suitable filler or carrier materials for liquid compositions include, but are not limited to water or low molecular weight primary and secondary alcohols including polyols and diols. Examples of such alcohols include, but are not limited to, methanol, ethanol, propanol and isopropanol. In some embodiments, the compositions contain from about 5% to about 90% of such materials. Acidic fillers find use to reduce pH. Alternatively, in some embodiments, the cleaning additive includes adjunct ingredients, as more fully described below.

The present cleaning compositions and cleaning additives require an effective amount of at least one of the serine protease polypeptides provided herein, alone or in combination with other proteases and/or additional enzymes. The required level of enzyme is achieved by the addition of one or more serine protease polypeptides of the present invention. Typically the present cleaning compositions comprise at least about 0.0001 weight percent, from about 0.0001 to about 10, from about 0.001 to about 1, or from about 0.01 to about 0.1 weight percent of at least one of the serine protease polypeptides of the present invention.

The cleaning compositions herein are typically formulated such that, during use in aqueous cleaning operations, the wash water will have a pH of from about 4.0 to about 11.5, or even from about 5.0 to about 11.5, or even from about 5.0 to about 8.0, or even from about 7.5 to about 10.5. Liquid product formulations are typically formulated to have a pH from about 3.0 to about 9.0 or even from about 3 to about 5. Granular laundry products are typically formulated to have a pH from about 9 to about 11. In some embodiments, the cleaning compositions of the present invention can be formulated to have an alkaline pH under wash conditions, such as a pH of from about 8.0 to about 12.0, or from about 8.5 to about 11.0, or from about 9.0 to about 11.0. In some embodiments, the cleaning compositions of the present invention can be formulated to have a neutral pH under wash conditions, such as a pH of from about 5.0 to about 8.0, or from about 5.5 to about 8.0, or from about 6.0 to about 8.0, or from about 6.0 to about 7.5. In some embodiments, the neutral pH conditions can be measured when the cleaning composition is dissolved 1:100 (wt:wt) in de-ionised water at 20°C., measured using a conventional pH meter. Techniques for controlling pH at recommended usage levels include the use of buffers, alkalis, acids, etc., and are well known to those skilled in the art.

In some embodiments, when the serine protease polypeptide (s) is/are employed in a granular composition or liquid, it is desirable for the serine protease polypeptide to be in the form of an encapsulated particle to protect the serine protease polypeptide from other components of the granular composition during storage. In addition, encapsulation is also a means of controlling the availability of the serine protease polypeptide during the cleaning process. In some embodiments, encapsulation enhances the performance of the serine protease polypeptide (s) and/or additional enzymes. In this regard, the serine protease polypeptides of the present invention are encapsulated with any suitable encapsulating material known in the art. In some embodiments, the encapsulating material typically encapsulates at least part of the serine protease polypeptide (s) of the present invention. Typically, the encapsulating material is water-soluble and/or water-dispersible. In some embodiments, the encapsulating material has a glass transition temperature (Tg) of 0°C or higher. Glass transition temperature is described in more detail in WO 97/11151. The encapsulating material is typically selected from consisting of carbohydrates, natural or synthetic gums, chitin, chitosan, cellulose and cellulose derivatives, silicates, phosphates, borates, polyvinyl alcohol, polyethylene glycol, paraffin waxes, and combinations thereof. When the encapsulating material is a carbohydrate, it is typically selected from monosaccharides, oligosaccharides, polysaccharides, and combinations thereof. In some typical embodiments, the encapsulating material is a starch (See e.g., EP 0 922 499; US 4,977,252; US 5,354,559, and US 5,935,826). In some embodiments, the encapsulating material is a microsphere made from plastic such as thermoplastics, acrylonitrile, methacrylonitrile, polyacrylonitrile, polymethacrylonitrile and mixtures thereof; commercially available microspheres that find use include, but are not limited to those supplied by EXPANCEL® (Stockviksverken, Sweden), and PM 6545, PM 6550, PM 7220, PM 7228, EXTENDOSPHERES®, LUXSIL®, Q-CEL®, and SPHERICEL® (PQ Corp., Valley Forge, PA).

As described herein, the variant proteases of the present invention find particular use in the cleaning industry, including, but not limited to laundry and dish detergents. These applications place enzymes under various environmental stresses. The variant proteases of the present invention provide advantages over many currently used enzymes, due to their stability under various conditions.

Indeed, there are a variety of wash conditions including varying detergent formulations, wash water volumes, wash water temperatures, and lengths of wash time, to which proteases involved in washing are exposed. In addition, detergent formulations used in different geographical areas have different concentrations of their relevant components present in the wash water. For example, European detergents typically have about 4500-5000 ppm of detergent components in the wash water, while Japanese detergents typically have approximately 667 ppm of detergent components in the wash water. In North America, particularly the United States, detergents typically have about 975 ppm of detergent components present in the wash water.

A low detergent concentration system includes detergents where less than about 800 ppm of the detergent components are present in the wash water. Japanese detergents are typically considered low detergent concentration system as they have approximately 667 ppm of detergent components present in the wash water.

A medium detergent concentration includes detergents where between about 800 ppm and about 2000ppm of the detergent components are present in the wash water. North American detergents are generally considered to be medium detergent concentration systems as they have approximately 975 ppm of detergent components present in the wash water. Brazil typically has approximately 1500 ppm of detergent components present in the wash water.

A high detergent concentration system includes detergents where greater than about 2000 ppm of the detergent components are present in the wash water. European detergents are generally considered to be high detergent concentration systems as they have approximately 4500-5000 ppm of detergent components in the wash water.

Latin American detergents are generally high suds phosphate builder detergents and the range of detergents used in Latin America can fall in both the medium and high detergent concentrations as they range from 1500 ppm to 6000 ppm of detergent components in the wash water. As mentioned above, Brazil typically has approximately 1500 ppm of detergent components present in the wash water. However, other high suds phosphate builder detergent geographies, not limited to other Latin American countries, may have high detergent concentration systems up to about 6000 ppm of detergent components present in the wash water.

In light of the foregoing, it is evident that concentrations of detergent compositions in typical wash solutions throughout the world varies from less than about 800 ppm of detergent composition ("low detergent concentration geographies"), for example about 667 ppm in Japan, to between about 800 ppm to about 2000 ppm ("medium detergent concentration geographies"), for example about 975 ppm in U.S. and about 1500 ppm in Brazil, to greater than about 2000 ppm ("high detergent concentration geographies"), for example about 4500 ppm to about 5000 ppm in Europe and about 6000 ppm in high suds phosphate builder geographies.

The concentrations of the typical wash solutions are determined empirically. For example, in the U.S., a typical washing machine holds a volume of about 64.4 L of wash solution. Accordingly, in order to obtain a concentration of about 975 ppm of detergent within the wash solution about 62.79 g of detergent composition must be added to the 64.4 L of wash solution. This amount is the typical amount measured into the wash water by the consumer using the measuring cup provided with the detergent.

As a further example, different geographies use different wash temperatures. The temperature of the wash water in Japan is typically less than that used in Europe. For example, the temperature of the wash water in North America and Japan is typically between about 10 and about 30°C (*e.g.,* about 20°C), whereas the temperature of wash water in Europe is typically between about 30 and about 60°C (*e.g.,* about 40°C). However, in the interest of saving energy, many consumers are switching to using cold water washing. In addition, in some further regions, cold water is typically used for laundry, as well as dish washing applications. In some embodiments, the "cold water washing" of the present invention utilizes "cold water detergent" suitable for washing at temperatures from about 10°C to about 40°C, or from about 20°C to about 30°C, or from about 15°C to about 25°C, as well as all other combinations within the range of about 15°C to about 35°C, and all ranges within 10°C to 40°C.

As a further example, different geographies typically have different water hardness. Water hardness is usually described in terms of the grains per gallon mixed Ca2+/Mg2+. Hardness is a measure of the amount of calcium (Ca2+) and magnesium (Mg2+) in the water. Most water in the United States is hard, but the degree of hardness varies. Moderately hard (60-120 ppm) to hard (121-181 ppm) water has 60 to 181 parts per million (parts per million converted to grains per U.S. gallon is ppm # divided by 17.1 equals grains per gallon) of hardness minerals.

**Table I. Water Hardness**

| **Water** | **Grains per gallon** | **Parts per million** |
|---|---|---|
| Soft | less than 1.0 | less than 17 |
| Slightly hard | 1.0 to 3.5 | 17 to 60 |
| Moderately hard | 3.5 to 7.0 | 60 to 120 |
| Hard | 7.0 to 10.5 | 120 to 180 |
| Very hard | greater than 10.5 | greater than 180 |

European water hardness is typically greater than about 10.5 (for example about 10.5 to about 20.0) grains per gallon mixed Ca2+/Mg2+ (e.g., about 15 grains per gallon mixed Ca2+/Mg2+). North American water hardness is typically greater than Japanese water hardness, but less than European water hardness. For example, North American water hardness can be between about 3 to about 10 grains, about 3 to about 8 grains or about 6 grains. Japanese water hardness is typically lower than North American water hardness, usually less than about 4, for example about 3 grains per gallon mixed Ca2+/Mg2+.

Accordingly, in some embodiments, the present invention provides serine protease polypeptides that show surprising wash performance in at least one set of wash conditions (e.g., water temperature, water hardness, and/or detergent concentration). In some embodiments, the serine protease polypeptides of the present invention are comparable in wash performance to other serine protease polypeptide proteases. In some embodiments of the present invention, the serine protease polypeptides provided herein exhibit enhanced oxidative stability, enhanced thermal stability, enhanced cleaning capabilities under various conditions, and/or enhanced chelator stability. In addition, the serine protease polypeptides of the present invention find use in cleaning compositions that do not include detergents, again either alone or in combination with builders and stabilizers.

In some embodiments of the present invention, the cleaning compositions comprise at least one serine protease polypeptide of the present invention at a level from about 0.00001 % to about 10% by weight of the composition and the balance (e.g., about 99.999% to about 90.0%) comprising cleaning adjunct materials by weight of composition. In some other embodiments of the present invention, the cleaning compositions of the present invention comprises at least one serine protease polypeptide at a level of about 0.0001 % to about 10%, about 0.001% to about 5%, about 0.001% to about 2%, about 0.005% to about 0.5% by weight of the composition and the balance of the cleaning composition (e.g., about 99.9999% to about 90.0%, about 99.999 % to about 98%, about 99.995% to about 99.5% by weight) comprising cleaning adjunct materials.

In some embodiments, the cleaning compositions of the present invention comprise one or more additional detergent enzymes, which provide cleaning performance and/or fabric care and/or dishwashing benefits.

Examples of suitable enzymes include, but are not limited to, additional serine proteases, acyl transferases, alpha-amylases, beta-amylases, alpha-galactosidases, arabinosidases, aryl esterases, beta-galactosidases, carrageenases, catalases, cellobiohydrolases, cellulases, chondroitinases, cutinases, endo-beta-1, 4-glucanases, endo-beta-mannanases, esterases, exo-mannanases, galactanases, glucoamylases, hemicellulases, hyaluronidases, keratinases, laccases, lactases, ligninases, lipases, lipoxygenases, mannanases, metalloproteases, non-serine proteases, oxidases, pectate lyases, pectin acetyl esterases, pectinases, pentosanases, peroxidases, perhydrolases, phenoloxidases, phosphatases, phospholipases, phytases, polygalacturonases, proteases, pullulanases, reductases, rhamnogalacturonases, beta-glucanases, tannases, transglutaminases, xylan acetyl-esterases, xylanases, xyloglucanases, and xylosidases, or any combinations or mixtures thereof. In some embodiments, a combination of enzymes is used (i.e., a "cocktail") comprising conventional applicable enzymes like amylase, lipase, cutinase and/or cellulase in conjunction with protease is used.

In addition to the serine protease polypeptides provided herein, any other suitable protease finds use in the compositions of the present invention. Suitable proteases include those of animal, vegetable or microbial origin. In some embodiments, microbial proteases are used. In some embodiments, chemically or genetically modified mutants are included. In some embodiments, the protease is a serine protease, preferably an alkaline microbial protease or a trypsin-like protease. Examples of alkaline proteases include subtilisins, especially those derived from *Bacillus* (e.g., subtilisin, *lentus, amyloliquefaciens,* subtilisin Carlsberg, subtilisin 309, subtilisin 147 and subtilisin 168). Additional examples include those mutant proteases described in U.S. Pat. Nos. RE 34,606, 5,955,340, 5,700,676, 6,312,936, and 6,482,628. Additional protease examples include, but are not limited to trypsin (e.g., of porcine or bovine origin), and the *Fusarium* protease described in WO 89/06270. In some embodiments, commercially available protease enzymes that find use in the present invention include, but are not limited to MAXATASE®, MAXACAL™, MAXAPEM™, OPTICLEAN®, OPTIMASE®, PROPERASE®, PURAFECT®, PURAFECT® OXP, PURAMAX™, EXCELLASE™, PREFERENZ™ proteases (e.g. P100, P110, P280), EFFECTENZ™ proteases (e.g. P1000, P1050, P2000), EXCELLENZ™ proteases (e.g. P1000), ULTIMASE®, and PURAFAST™ (Genencor); ALCALASE®, SAVINASE®, PRIMASE®, DURAZYM™, POLARZYME®, OVOZYME®, KANNASE®, LIQUANASE®, NEUTRASE®, RELASE® and ESPERASE® (Novozymes); BLAP™ and BLAP™ variants (Henkel Kommanditgesellschaft auf Aktien, Duesseldorf, Germany), and KAP (B. alkalophilus subtilisin; Kao Corp., Tokyo, Japan). Various proteases are described in WO95/23221, WO 92/21760, WO 09/149200, WO 09/149144, WO 09/149145, WO 11/072099, WO 10/056640, WO 10/056653, WO 11/140364, WO 12/151534, U.S. Pat. Publ. No. 2008/0090747, and U.S. Pat. Nos. 5,801,039, 5,340,735, 5,500,364, 5,855,625, US RE 34,606, 5,955,340, 5,700,676, 6,312,936, 6,482,628, 8,530,219, and various other patents. In some further embodiments, metalloproteases find use in the present invention, including but not limited to the metalloproteases described in WO1999014341, WO1999033960, WO1999014342, WO1999034003, WO2007044993, WO2009058303, WO2009058661, WO2014194032, WO2014194034, and WO2014194054. Exemplary metalloproteases include nprE, the recombinant form of neutral metalloprotease expressed in *B. subtilis* (See e.g., WO 07/044993), and PMN, the purified neutral metalloprotease from *B. amyloliquefacients.*

In addition, any suitable lipase finds use in the present invention. Suitable lipases include, but are not limited to those of bacterial or fungal origin. Chemically or genetically modified mutants are encompassed by the present invention. Examples of useful lipases include *Humicola lanuginosa* lipase (See e.g., EP 258 068, and EP 305 216), *Rhizomucor miehei* lipase (See e.g., EP 238 023), *Candida* lipase, such as *C*. *antarctica* lipase (e.g., the *C*. *antarctica* lipase A or B; See e.g., EP 214 761), *Pseudomonas* lipases such as *P. alcaligenes* lipase and P. *pseudoalcaligenes* lipase (See e.g., EP 218 272), *P. cepacia* lipase (See e.g., EP 331 376), P. *stutzeri* lipase (See e.g., GB 1,372,034), *P. fluorescens* lipase, *B. lipase* (e.g., *B. subtilis* lipase [Dartois et al., Biochem. Biophys. Acta 1131:253-260 [1993]); *B. stearothermophilus* lipase [See e.g., JP 64/744992]; and *B. pumilus* lipase [See e.g., WO 91/16422]).

Furthermore, a number of cloned lipases find use in some embodiments of the present invention, including but not limited to Penicillium camembertii lipase (See, Yamaguchi et al., Gene 103:61-67 [1991]), Geotricum candidum lipase (See, Schimada et al., J. Biochem., 106:383-388 [1989]), and various Rhizopus lipases such as R. delemar lipase (See, Hass et al., Gene 109:117-113 [1991]), a R. niveus lipase (Kugimiya et al., Biosci. Biotech. Biochem. 56:716-719 [1992]) and R. oryzae lipase.

Other types of lipase polypeptide enzymes such as cutinases also find use in some embodiments of the present invention, including but not limited to the cutinase derived from Pseudomonas mendocina (See, WO 88/09367), and the cutinase derived from Fusarium solani pisi (See, WO 90/09446).

Additional suitable lipases include lipases such as M1 LIPASE™, LUMA FAST™, and LIPOMAX™ (Genencor); LIPEX®, LIPOLASE® and LIPOLASE® ULTRA (Novozymes); and LIPASE P™ "Amano" (Amano Pharmaceutical Co. Ltd., Japan).

In some embodiments of the present invention, the cleaning compositions of the present invention further comprise lipases at a level from about 0.00001 % to about 10% of additional lipase by weight of the composition and the balance of cleaning adjunct materials by weight of composition. In some other embodiments of the present invention, the cleaning compositions of the present invention also comprise lipases at a level of about 0.0001 % to about 10%, about 0.001% to about 5%, about 0.001% to about 2%, about 0.005% to about 0.5% lipase by weight of the composition.

In some embodiments of the present invention, any suitable amylase finds use in the present invention. In some embodiments, any amylase (e.g., alpha and/or beta) suitable for use in alkaline solutions also find use. Suitable amylases include, but are not limited to those of bacterial or fungal origin. Chemically or genetically modified mutants are included in some embodiments. Amylases that find use in the present invention, include, but are not limited to α-amylases obtained from B. licheniformis (See e.g., GB 1,296,839). Additional suitable amylases include those found in WO9510603, WO9526397, WO9623874, WO9623873, WO9741213, WO9919467, WO0060060, WO0029560, WO9923211, WO9946399, WO0060058, WO0060059, WO9942567, WO0114532, WO02092797, WO0166712, WO0188107, WO0196537, WO0210355, WO9402597, WO0231124, WO9943793, WO9943794, WO2004113551, WO2005001064, WO2005003311, WO0164852, WO2006063594, WO2006066594, WO2006066596, WO2006012899, WO2008092919, WO2008000825, WO2005018336, WO2005066338, WO2009140504, WO2005019443, WO2010091221, WO2010088447, WO0134784, WO2006012902, WO2006031554, WO2006136161, WO2008101894, WO2010059413, WO2011098531, WO2011080352, WO2011080353, WO2011080354, WO2011082425, WO2011082429, WO2011076123, WO2011087836, WO2011076897, WO94183314, WO9535382, WO9909183, WO9826078, WO9902702, WO9743424, WO9929876, WO9100353, WO9605295, WO9630481, WO9710342, WO2008088493, WO2009149419, WO2009061381, WO2009100102, WO2010104675, WO2010117511, and WO2010115021. Commercially available amylases that find use in the present invention include, but are not limited to DURAMYL®, TERMAMYL®, FUNGAMYL®, STAINZYME®, STAINZYME PLUS®, STAINZYME ULTRA®, and BAN™ (Novozymes), as well as POWERASE™, RAPIDASE® and MAXAMYL® P (Genencor).

In some embodiments of the present invention, the cleaning compositions of the present invention further comprise amylases at a level from about 0.00001 % to about 10% of additional amylase by weight of the composition and the balance of cleaning adjunct materials by weight of composition. In some other embodiments of the present invention, the cleaning compositions of the present invention also comprise amylases at a level of about 0.0001 % to about 10%, about 0.001% to about 5%, about 0.001% to about 2%, about 0.005% to about 0.5% amylase by weight of the composition.

In some further embodiments, any suitable cellulase finds used in the cleaning compositions of the present invention. Suitable cellulases include, but are not limited to those of bacterial or fungal origin. Chemically or genetically modified mutants are included in some embodiments. Suitable cellulases include, but are not limited to *Humicola insolens* cellulases (See e.g., U.S. Pat. No. 4,435,307). Especially suitable cellulases are the cellulases having color care benefits (See e.g., EP 0 495 257). Commercially available cellulases that find use in the present include, but are not limited to CELLUZYME®, CAREZYME® (Novozymes), REVITALENZ™ 100 (Danisco US Inc) and KAC-500(B)™ (Kao Corporation). In some embodiments, cellulases are incorporated as portions or fragments of mature wild-type or variant cellulases, wherein a portion of the N-terminus is deleted *(See e.g.,* U.S. Pat. No. 5,874,276). Additional suitable cellulases include those found in WO2005054475, WO2005056787, U.S. Pat. No. 7,449,318, and U.S. Pat. No. 7,833,773. In some embodiments, the cleaning compositions of the present invention further comprise cellulases at a level from about 0.00001 % to about 10% of additional cellulase by weight of the composition and the balance of cleaning adjunct materials by weight of composition. In some other embodiments of the present invention, the cleaning compositions of the present invention also comprise cellulases at a level of about 0.0001 % to about 10%, about 0.001% to about 5%, about 0.001% to about 2%, about 0.005% to about 0.5% cellulase by weight of the composition.

Any mannanase suitable for use in detergent compositions also finds use in the present invention. Suitable mannanases include, but are not limited to those of bacterial or fungal origin. Chemically or genetically modified mutants are included in some embodiments. Various mannanases are known which find use in the present invention *(See e.g.,* U.S. Pat. No. 6,566,114, U.S. Pat. No.6,602,842, and US Patent No. 6,440,991). Commercially available mannanases that find use in the present invention include, but are not limited to MANNASTAR®, PURABRITE™, and MANNAWAY®. In some embodiments, the cleaning compositions of the present invention further comprise mannanases at a level from about 0.00001 % to about 10% of additional mannanase by weight of the composition and the balance of cleaning adjunct materials by weight of composition. In some embodiments of the present invention, the cleaning compositions of the present invention also comprise mannanases at a level of about 0.0001 % to about 10%, about 0.001% to about 5%, about 0.001% to about 2%, about 0.005% to about 0.5% mannanase by weight of the composition.

In some embodiments, peroxidases are used in combination with hydrogen peroxide or a source thereof (e.g., a percarbonate, perborate or persulfate) in the compositions of the present invention. In some alternative embodiments, oxidases are used in combination with oxygen. Both types of enzymes are used for "solution bleaching" (i.e., to prevent transfer of a textile dye from a dyed fabric to another fabric when the fabrics are washed together in a wash liquor), preferably together with an enhancing agent (See e.g., WO 94/12621 and WO 95/01426). Suitable peroxidases/oxidases include, but are not limited to those of plant, bacterial or fungal origin. Chemically or genetically modified mutants are included in some embodiments. In some embodiments, the cleaning compositions of the present invention further comprise peroxidase and/or oxidase enzymes at a level from about 0.00001 % to about 10% of additional peroxidase and/or oxidase by weight of the composition and the balance of cleaning adjunct materials by weight of composition. In some other embodiments of the present invention, the cleaning compositions of the present invention also comprise, peroxidase and/or oxidase enzymes at a level of about 0.0001 % to about 10%, about 0.001% to about 5%, about 0.001% to about 2%, about 0.005% to about 0.5% peroxidase and/or oxidase enzymes by weight of the composition.

In some embodiments, additional enzymes find use, including but not limited to perhydrolases (See e.g., WO 2005056782, WO2007106293, WO2008063400, WO2008106214, and WO2008106215). In addition, in some embodiments, mixtures of the above mentioned enzymes are encompassed herein, in particular one or more additional protease, amylase, lipase, mannanase, and/or at least one cellulase. Indeed, it is contemplated that various mixtures of these enzymes will find use in the present invention. It is also contemplated that the varying levels of the serine protease polypeptide (s) and one or more additional enzymes may both independently range to about 10%, the balance of the cleaning composition being cleaning adjunct materials. The specific selection of cleaning adjunct materials are readily made by considering the surface, item, or fabric to be cleaned, and the desired form of the composition for the cleaning conditions during use (e.g., through the wash detergent use).

Examples of suitable cleaning adjunct materials include, but are not limited to, surfactants, builders, bleaches, bleach activators, bleach catalysts, other enzymes, enzyme stabilizing systems, chelants, optical brighteners, soil release polymers, dye transfer agents, dye transfer inhibiting agents, catalytic materials, hydrogen peroxide, sources of hydrogen peroxide, preformed peracids, polymeric dispersing agents, clay soil removal agents, structure elasticizing agents, dispersants, suds suppressors, dyes, perfumes, colorants, filler salts, hydrotropes, photoactivators, fluorescers, fabric conditioners, fabric softeners, carriers, hydrotropes, processing aids, solvents, pigments, hydrolyzable surfactants, preservatives, anti-oxidants, anti-shrinkage agents, anti-wrinkle agents, germicides, fungicides, color speckles, silvercare, anti-tarnish and/or anti-corrosion agents, alkalinity sources, solubilizing agents, carriers, processing aids, pigments, and pH control agents (*See e.g.,* U.S. Pat. Nos. 6,610,642; 6,605,458; 5,705,464; 5,710,115; 5,698,504; 5,695,679; 5,686,014 and 5,646,101). Embodiments of specific cleaning composition materials are exemplified in detail below. In embodiments in which the cleaning adjunct materials are not compatible with the variant proteases of the present invention in the cleaning compositions, then suitable methods of keeping the cleaning adjunct materials and the protease(s) separated (i.e., not in contact with each other) until combination of the two components is appropriate are used. Such separation methods include any suitable method known in the art (e.g., gelcaps, encapsulation, tablets, physical separation, etc.).

In some embodiments, an effective amount of one or more serine protease polypeptide (s) provided herein is included in compositions useful for cleaning a variety of surfaces in need of proteinaceous stain removal. Such cleaning compositions include cleaning compositions for such applications as cleaning hard surfaces, fabrics, and dishes. Indeed, in some embodiments, the present invention provides fabric cleaning compositions, while in other embodiments, the present invention provides non-fabric cleaning compositions. Notably, the present invention also provides cleaning compositions suitable for personal care, including oral care (including dentrifices, toothpastes, mouthwashes, etc., as well as denture cleaning compositions), skin, and hair cleaning compositions. It is intended that the present invention encompass detergent compositions in any form (i.e., liquid, granular, bar, semi-solid, gels, emulsions, tablets, capsules, etc.).

By way of example, several cleaning compositions wherein the serine protease polypeptides of the present invention find use are described in greater detail below. In some embodiments in which the cleaning compositions of the present invention are formulated as compositions suitable for use in laundry machine washing method(s), the compositions of the present invention preferably contain at least one surfactant and at least one builder compound, as well as one or more cleaning adjunct materials preferably selected from organic polymeric compounds, bleaching agents, additional enzymes, suds suppressors, dispersants, lime-soap dispersants, soil suspension and anti-redeposition agents and corrosion inhibitors. In some embodiments, laundry compositions also contain softening agents (i.e., as additional cleaning adjunct materials). The compositions of the present invention also find use in detergent additive products in solid or liquid form. Such additive products are intended to supplement and/or boost the performance of conventional detergent compositions and can be added at any stage of the cleaning process. In some embodiments, the density of the laundry detergent compositions herein ranges from about 400 to about 1200 g/liter, while in other embodiments, it ranges from about 500 to about 950 g/liter of composition measured at 20°C.

In embodiments formulated as compositions for use in manual dishwashing methods, the compositions of the invention preferably contain at least one surfactant and preferably at least one additional cleaning adjunct material selected from organic polymeric compounds, suds enhancing agents, group II metal ions, solvents, hydrotropes and additional enzymes.

In some embodiments, various cleaning compositions such as those provided in U.S. Pat. No. 6,605,458, find use with the serine protease polypeptides of the present invention. Thus, in some embodiments, the compositions comprising at least one serine protease polypeptide of the present invention is a compact granular fabric cleaning composition, while in other embodiments, the composition is a granular fabric cleaning composition useful in the laundering of colored fabrics, in further embodiments, the composition is a granular fabric cleaning composition which provides softening through the wash capacity, in additional embodiments, the composition is a heavy duty liquid fabric cleaning composition. In some embodiments, the compositions comprising at least one serine protease polypeptide of the present invention are fabric cleaning compositions such as those described in U.S. Pat. Nos. 6,610,642 and 6,376,450. In addition, the serine protease polypeptides of the present invention find use in granular laundry detergent compositions of particular utility under European or Japanese washing conditions (*See e.g.,* U.S. Pat. No. 6,610,642).

In some alternative embodiments, the present invention provides hard surface cleaning compositions comprising at least one serine protease polypeptide provided herein. Thus, in some embodiments, the compositions comprising at least one serine protease polypeptide of the present invention is a hard surface cleaning composition such as those described in U.S. Pat. Nos. 6,610,642, 6,376,450, and 6,376,450.

In yet further embodiments, the present invention provides dishwashing compositions comprising at least one serine protease polypeptide provided herein. Thus, in some embodiments, the compositions comprising at least one serine protease polypeptide of the present invention is a hard surface cleaning composition such as those in U.S. Pat. Nos. 6,610,642 and 6,376,450. In some still further embodiments, the present invention provides dishwashing compositions comprising at least one serine protease polypeptide provided herein. In some further embodiments, the compositions comprising at least one serine protease polypeptide of the present invention comprise oral care compositions such as those in U.S. Pat. No. 6,376,450, and 6,376,450. The formulations and descriptions of the compounds and cleaning adjunct materials contained in the aforementioned US Pat. Nos. 6,376,450, 6,605,458, 6,605,458, and 6,610,642, find use with the serine protease polypeptides provided herein.

The cleaning compositions of the present invention are formulated into any suitable form and prepared by any process chosen by the formulator (*See e.g.,* U.S. Pat. Nos. 5,879,584; 5,691,297; 5,574,005; 5,569,645; 5,565,422; 5,516,448; 5,489,392; and 5,486,303. When a low pH cleaning composition is desired, the pH of such composition is adjusted via the addition of a material such as monoethanolamine or an acidic material such as HCl.

In some embodiments, the cleaning compositions according to the present invention comprise an acidifying particle or an amino carboxylic builder. Examples of an amino carboxylic builder include aminocarboxylic acids, salts and derivatives thereof. In some embodiment, the amino carboxylic builder is an aminopolycarboxylic builder, such as glycine-N,N-diacetic acid or derivative of general formula MOOC-CHR-N (CH2COOM)2 where R is C1-12 alkyl and M is alkali metal. In some embodiments, the amino carboxylic builder can be methylglycine diacetic acid (MGDA), GLDA (glutamic-N,N-diacetic acid), iminodisuccinic acid (IDS), carboxymethyl inulin and salts and derivatives thereof, aspartic acid-N-monoacetic acid (ASMA), aspartic acid-N,N-diacetic acid (ASDA), aspartic acid-N-monopropionic acid (ASMP), iminodisuccinic acid (IDA), N-(2-sulfomethyl) aspartic acid (SMAS), N-(2-sulfoethyl)aspartic acid (SEAS), N-(2-sulfomethyl)glutamic acid (SMGL), N-(2-sulfoethyl) glutamic acid (SEGL), IDS (iminodiacetic acid) and salts and derivatives thereof such as N-methyliminodiacetic acid (MIDA), alpha-alanine-N,N-diacetic acid (alpha-ALDA), serine-N,N-diacetic acid (SEDA), isoserine-N,Ndiacetic acid (ISDA), phenylalanine-N,N-diacetic acid (PHDA), anthranilic acid-N,N-diacetic acid (ANDA), sulfanilic acid-N,N-diacetic acid (SLDA), taurine-N,N-diacetic acid (TUDA) and sulfomethyl-N,N-diacetic acid (SMDA) and alkali metal salts and derivative thereof. In some embodiments, the acidifying particle has a weight geometric mean particle size of from about 400 µ to about 1200 µ and a bulk density of at least 550 g/L. In some embodiments, the acidifying particle comprises at least about 5% of the builder.

In some embodiments, the acidifying particle can comprise any acid, including organic acids and mineral acids. Organic acids can have one or two carboxyls and in some instances up to 15 carbons, especially up to 10 carbons, such as formic, acetic, propionic, capric, oxalic, succinic, adipic, maleic, fumaric, sebacic, malic, lactic, glycolic, tartaric and glyoxylic acids. In some embodiments, the acid is citric acid. Mineral acids include hydrochloric and sulphuric acid. In some instances, the acidifying particle of the invention is a highly active particle comprising a high level of amino carboxylic builder. Sulphuric acid has been found to further contribute to the stability of the final particle.

While not essential for the purposes of the present invention, the non-limiting list of adjuncts illustrated hereinafter are suitable for use in the instant cleaning compositions. In some embodiments, these adjuncts are incorporated for example, to assist or enhance cleaning performance, for treatment of the substrate to be cleaned, or to modify the aesthetics of the cleaning composition as is the case with perfumes, colorants, dyes or the like. It is understood that such adjuncts are in addition to the variant proteases of the present invention. The precise nature of these additional components, and levels of incorporation thereof, will depend on the physical form of the composition and the nature of the cleaning operation for which it is to be used. Suitable adjunct materials include, but are not limited to, surfactants, builders, chelating agents, dye transfer inhibiting agents, deposition aids, dispersants, additional enzymes, and enzyme stabilizers, catalytic materials, bleach activators, bleach boosters, hydrogen peroxide, sources of hydrogen peroxide, preformed peracids, polymeric dispersing agents, clay soil removal/anti-redeposition agents, brighteners, suds suppressors, dyes, perfumes, structure elasticizing agents, fabric softeners, carriers, hydrotropes, processing aids and/or pigments. In addition to the disclosure below, suitable examples of such other adjuncts and levels of use are found in U.S. Patent Nos. 5,576,282, 6,306,812, and 6,326,348. The aforementioned adjunct ingredients may constitute the balance of the cleaning compositions of the present invention.

In some embodiments, the cleaning compositions according to the present invention comprise at least one surfactant and/or a surfactant system wherein the surfactant is selected from nonionic surfactants, anionic surfactants, cationic surfactants, ampholytic surfactants, zwitterionic surfactants, semi-polar nonionic surfactants and mixtures thereof. In some low pH cleaning composition embodiments (*e.g.,* compositions having a neat pH of from about 3 to about 5), the composition typically does not contain alkyl ethoxylated sulfate, as it is believed that such surfactant may be hydrolyzed by such compositions. In some embodiments, the surfactant is present at a level of from about 0.1% to about 60%, while in alternative embodiments the level is from about 1% to about 50%, while in still further embodiments the level is from about 5% to about 40%, by weight of the cleaning composition.

In some embodiments, the cleaning compositions of the present invention comprise one or more detergent builders or builder systems. In some embodiments incorporating at least one builder, the cleaning compositions comprise at least about 1%, from about 3% to about 60% or even from about 5% to about 40% builder by weight of the cleaning composition. Builders include, but are not limited to, the alkali metal, ammonium and alkanolammonium salts of polyphosphates; alkali metal silicates; alkaline earth and alkali metal carbonates; aluminosilicates; polycarboxylate compounds; ether hydroxypolycarboxylates; copolymers of maleic anhydride with ethylene or vinyl methyl ether, 1, 3, 5-trihydroxy benzene-2, 4, 6-trisulphonic acid, and carboxymethyloxysuccinic acid; the various alkali metal, ammonium and substituted ammonium salts of polyacetic acids such as ethylenediamine tetraacetic acid and nitrilotriacetic acid; as well as polycarboxylates such as mellitic acid, succinic acid, citric acid, oxydisuccinic acid, polymaleic acid, benzene 1,3,5-tricarboxylic acid, carboxymethyloxysuccinic acid; and soluble salts thereof. Indeed, it is contemplated that any suitable builder will find use in various embodiments of the present invention.

In some embodiments, the builders form water-soluble hardness ion complexes (*e.g.,* sequestering builders), such as citrates and polyphosphates (*e.g.,* sodium tripolyphosphate and sodium tripolyphospate hexahydrate, potassium tripolyphosphate, and mixed sodium and potassium tripolyphosphate, etc.). It is contemplated that any suitable builder will find use in the present invention, including those known in the art (*See e.g.,* EP 2 100 949).

In some embodiments, builders for use herein include phosphate builders and non-phosphate builders. In some embodiments, the builder is a phosphate builder. In some embodiments, the builder is a non-phosphate builder. If present, builders are used in a level of from 0.1% to 80%, or from 5 to 60%, or from 10 to 50% by weight of the composition. In some embodiments the product comprises a mixture of phosphate and non-phosphate builders. Suitable phosphate builders include mono-phosphates, di-phosphates, tri-polyphosphates or oligomeric-poylphosphates, including the alkali metal salts of these compounds, including the sodium salts. In some embodiments, a builder can be sodium tripolyphosphate (STPP). Additionally, the composition can comprise carbonate and/or citrate, preferably citrate that helps to achieve a neutral pH composition of the invention. Other suitable non-phosphate builders include homopolymers and copolymers of polycarboxylic acids and their partially or completely neutralized salts, monomeric polycarboxylic acids and hydroxycarboxylic acids and their salts. In some embodiments, salts of the above mentioned compounds include the ammonium and/or alkali metal salts, i.e. the lithium, sodium, and potassium salts, including sodium salts. Suitable polycarboxylic acids include acyclic, alicyclic, hetero-cyclic and aromatic carboxylic acids, wherein in some embodiments, they can contain at least two carboxyl groups which are in each case separated from one another by, in some instances, no more than two carbon atoms.

In some embodiments, the cleaning compositions of the present invention contain at least one chelating agent. Suitable chelating agents include, but are not limited to copper, iron and/or manganese chelating agents and mixtures thereof. In embodiments in which at least one chelating agent is used, the cleaning compositions of the present invention comprise from about 0.1% to about 15% or even from about 3.0% to about 10% chelating agent by weight of the subject cleaning composition.

In some still further embodiments, the cleaning compositions provided herein contain at least one deposition aid. Suitable deposition aids include, but are not limited to, polyethylene glycol, polypropylene glycol, polycarboxylate, soil release polymers such as polytelephthalic acid, clays such as kaolinite, montmorillonite, atapulgite, illite, bentonite, halloysite, and mixtures thereof.

As indicated herein, in some embodiments, anti-redeposition agents find use in some embodiments of the present invention. In some embodiments, non-ionic surfactants find use. For example, in automatic dishwashing embodiments, non-ionic surfactants find use for surface modification purposes, in particular for sheeting, to avoid filming and spotting and to improve shine. These non-ionic surfactants also find use in preventing the re-deposition of soils. In some embodiments, the anti-redeposition agent is a non-ionic surfactant as known in the art (*See e.g.,* EP 2 100 949). In some embodiments, the non-ionic surfactant can be ethoxylated nonionic surfactants, epoxy-capped poly(oxyalkylated) alcohols and amine oxides surfactants.

In some embodiments, the cleaning compositions of the present invention include one or more dye transfer inhibiting agents. Suitable polymeric dye transfer inhibiting agents include, but are not limited to, polyvinylpyrrolidone polymers, polyamine N-oxide polymers, copolymers of N-vinylpyrrolidone and N-vinylimidazole, polyvinyloxazolidones and polyvinylimidazoles or mixtures thereof. In embodiments in which at least one dye transfer inhibiting agent is used, the cleaning compositions of the present invention comprise from about 0.0001% to about 10%, from about 0.01% to about 5%, or even from about 0.1% to about 3% by weight of the cleaning composition.

In some embodiments, silicates are included within the compositions of the present invention. In some such embodiments, sodium silicates (e.g., sodium disilicate, sodium metasilicate, and crystalline phyllosilicates) find use. In some embodiments, silicates are present at a level of from about 1% to about 20%. In some embodiments, silicates are present at a level of from about 5% to about 15% by weight of the composition.

In some still additional embodiments, the cleaning compositions of the present invention also contain dispersants. Suitable water-soluble organic materials include, but are not limited to the homo- or co-polymeric acids or their salts, in which the polycarboxylic acid comprises at least two carboxyl radicals separated from each other by not more than two carbon atoms.

In some further embodiments, the enzymes used in the cleaning compositions are stabilized by any suitable technique. In some embodiments, the enzymes employed herein are stabilized by the presence of water-soluble sources of calcium and/or magnesium ions in the finished compositions that provide such ions to the enzymes. In some embodiments, the enzyme stabilizers include oligosaccharides, polysaccharides, and inorganic divalent metal salts, including alkaline earth metals, such as calcium salts, such as calcium formate. It is contemplated that various techniques for enzyme stabilization will find use in the present invention. For example, in some embodiments, the enzymes employed herein are stabilized by the presence of water-soluble sources of zinc (II), calcium (II) and/or magnesium (II) ions in the finished compositions that provide such ions to the enzymes, as well as other metal ions (e.g., barium (II), scandium (II), iron (II), manganese (II), aluminum (III), Tin (II), cobalt (II), copper (II), nickel (II), and oxovanadium (IV). Chlorides and sulfates also find use in some embodiments of the present invention. Examples of suitable oligosaccharides and polysaccharides (e.g., dextrins) are known in the art (See e.g., WO 07/145964). In some embodiments, reversible protease inhibitors also find use, such as boron-containing compounds (e.g., borate, 4-formyl phenyl boronic acid) and/or a tripeptide aldehyde find use to further improve stability, as desired.

In some embodiments, bleaches, bleach activators and/or bleach catalysts are present in the compositions of the present invention. In some embodiments, the cleaning compositions of the present invention comprise inorganic and/or organic bleaching compound(s). Inorganic bleaches include, but are not limited to perhydrate salts (*e.g.,* perborate, percarbonate, perphosphate, persulfate, and persilicate salts). In some embodiments, inorganic perhydrate salts are alkali metal salts. In some embodiments, inorganic perhydrate salts are included as the crystalline solid, without additional protection, although in some other embodiments, the salt is coated. Any suitable salt known in the art finds use in the present invention (*See e.g.,* EP 2 100 949).

In some embodiments, bleach activators are used in the compositions of the present invention. Bleach activators are typically organic peracid precursors that enhance the bleaching action in the course of cleaning at temperatures of 60°C and below. Bleach activators suitable for use herein include compounds which, under perhydrolysis conditions, give aliphatic peroxoycarboxylic acids having preferably from about 1 to about 10 carbon atoms, in particular from about 2 to about 4 carbon atoms, and/or optionally substituted perbenzoic acid. Additional bleach activators are known in the art and find use in the present invention (*See e.g.,* EP 2 100 949).

In addition, in some embodiments and as further described herein, the cleaning compositions of the present invention further comprise at least one bleach catalyst. In some embodiments, the manganese triazacyclononane and related complexes find use, as well as cobalt, copper, manganese, and iron complexes. Additional bleach catalysts find use in the present invention (*See e.g.,* US 4,246,612; 5,227,084; 4,810,410; WO 99/06521; and EP 2 100 949).

In some embodiments, the cleaning compositions of the present invention contain one or more catalytic metal complexes. In some embodiments, a metal-containing bleach catalyst finds use. In some embodiments, the metal bleach catalyst comprises a catalyst system comprising a transition metal cation of defined bleach catalytic activity, (*e.g.,* copper, iron, titanium, ruthenium, tungsten, molybdenum, or manganese cations), an auxiliary metal cation having little or no bleach catalytic activity (*e.g.,* zinc or aluminum cations), and a sequestrate having defined stability constants for the catalytic and auxiliary metal cations, particularly ethylenediaminetetraacetic acid, ethylenediaminetetra (methylenephosphonic acid) and water-soluble salts thereof are used (*See e.g.,* US Patent No. 4,430,243). In some embodiments, the cleaning compositions of the present invention are catalyzed by means of a manganese compound. Such compounds and levels of use are well known in the art (*See e.g.,* US Patent No. 5,576,282). In additional embodiments, cobalt bleach catalysts find use in the cleaning compositions of the present invention. Various cobalt bleach catalysts are known in the art (*See e.g.,* US Patent Nos. 5,597,936 and 5,595,967) and are readily prepared by known procedures.

In some additional embodiments, the cleaning compositions of the present invention include a transition metal complex of a macropolycyclic rigid ligand (MRL). As a practical matter, and not by way of limitation, in some embodiments, the compositions and cleaning processes provided by the present invention are adjusted to provide on the order of at least one part per hundred million of the active MRL species in the aqueous washing medium, and in some embodiments, provide from about 0.005 ppm to about 25 ppm, more preferably from about 0.05 ppm to about 10 ppm, and most preferably from about 0.1 ppm to about 5 ppm, of the MRL in the wash liquor.

In some embodiments, transition-metals in the instant transition-metal bleach catalyst include, but are not limited to manganese, iron and chromium. MRLs also include, but are not limited to special ultra-rigid ligands that are cross-bridged (*e.g.,* 5,12-diethyl-1,5,8,12-tetraazabicyclo[6.6.2]hexadecane). Suitable transition metal MRLs are readily prepared by known procedures (*See e.g.,* WO 2000/32601 and US Patent No. 6,225,464).

In some embodiments, the cleaning compositions of the present invention comprise metal care agents. Metal care agents find use in preventing and/or reducing the tarnishing, corrosion, and/or oxidation of metals, including aluminum, stainless steel, and non-ferrous metals (*e.g.,* silver and copper). Suitable metal care agents include those described in EP 2 100 949, WO 9426860, and WO 94/26859). In some embodiments, the metal care agent is a zinc salt. In some further embodiments, the cleaning compositions of the present invention comprise from about 0.1% to about 5% by weight of one or more metal care agent.

In some embodiments, the cleaning composition is a high density liquid (HDL) composition having a variant serine protease polypeptide protease. The HDL liquid laundry detergent can comprise a detersive surfactant (10%-40%) comprising anionic detersive surfactant (selected from a group of linear or branched or random chain, substituted or unsubstituted alkyl sulphates, alkyl sulphonates, alkyl alkoxylated sulphate, alkyl phosphates, alkyl phosphonates, alkyl carboxylates, and/or mixtures thereof); and optionally non-ionic surfactant (selected from a group of linear or branched or random chain, substituted or unsubstituted alkyl alkoxylated alcohol, for example a C₈-C₁₈ alkyl ethoxylated alcohol and/or C₆-C₁₂ alkyl phenol alkoxylates), optionally wherein the weight ratio of anionic detersive surfactant (with a hydrophilic index (HIc) of from 6.0 to 9) to non-ionic detersive surfactant is greater than 1:1. Suitable detersive surfactants also include cationic detersive surfactants (selected from a group of alkyl pyridinium compounds, alkyl quarternary ammonium compounds, alkyl quarternary phosphonium compounds, alkyl ternary sulphonium compounds, and/or mixtures thereof); zwitterionic and/or amphoteric detersive surfactants (selected from a group of alkanolamine sulpho-betaines); ampholytic surfactants; semi-polar non-ionic surfactants and mixtures thereof.

The composition can comprise optionally, a surfactancy boosting polymer consisting of amphiphilic alkoxylated grease cleaning polymers (selected from a group of alkoxylated polymers having branched hydrophilic and hydrophobic properties, such as alkoxylated polyalkylenimines in the range of 0.05wt%-10wt%) and/or random graft polymers (typically comprising of hydrophilic backbone comprising monomers selected from the group consisting of: unsaturated C₁-C₆ carboxylic acids, ethers, alcohols, aldehydes, ketones, esters, sugar units, alkoxy units, maleic anhydride, saturated polyalcohols such as glycerol, and mixtures thereof; and hydrophobic side chain(s) selected from the group consisting of: C₄-C₂₅ alkyl group, polypropylene, polybutylene, vinyl ester of a saturated C₁-C₆ mono-carboxylic acid, C₁-C₆ alkyl ester of acrylic or methacrylic acid, and mixtures thereof.

The composition can comprise additional polymers such as soil release polymers (include anionically end-capped polyesters, for example SRP1, polymers comprising at least one monomer unit selected from saccharide, dicarboxylic acid, polyol and combinations thereof, in random or block configuration, ethylene terephthalate-based polymers and co-polymers thereof in random or block configuration, for example Repel-o-tex SF, SF-2 and SRP6, Texcare SRA100, SRA300, SRN100, SRN170, SRN240, SRN300 and SRN325, Marloquest SL), anti-redeposition polymers (0.1 wt% to 10wt%, include carboxylate polymers, such as polymers comprising at least one monomer selected from acrylic acid, maleic acid (or maleic anhydride), fumaric acid, itaconic acid, aconitic acid, mesaconic acid, citraconic acid, methylenemalonic acid, and any mixture thereof, vinylpyrrolidone homopolymer, and/or polyethylene glycol, molecular weight in the range of from 500 to 100,000 Da); cellulosic polymer (including those selected from alkyl cellulose, alkyl alkoxyalkyl cellulose, carboxyalkyl cellulose, alkyl carboxyalkyl cellulose examples of which include carboxymethyl cellulose, methyl cellulose, methyl hydroxyethyl cellulose, methyl carboxymethyl cellulose, and mixures thereof) and polymeric carboxylate (such as maleate/acrylate random copolymer or polyacrylate homopolymer).

The composition can further comprise saturated or unsaturated fatty acid, preferably saturated or unsaturated C₁₂-C₂₄ fatty acid (0 wt% to 10 wt%); deposition aids (examples for which include polysaccharides, preferably cellulosic polymers, poly diallyl dimethyl ammonium halides (DADMAC), and co-polymers of DAD MAC with vinyl pyrrolidone, acrylamides, imidazoles, imidazolinium halides, and mixtures thereof, in random or block configuration, cationic guar gum, cationic cellulose such as cationic hydoxyethyl cellulose, cationic starch, cationic polyacylamides, and mixtures thereof.

The composition can further comprise dye transfer inhibiting agents examples of which include manganese phthalocyanine, peroxidases, polyvinylpyrrolidone polymers, polyamine N-oxide polymers, copolymers of N-vinylpyrrolidone and N-vinylimidazole, polyvinyloxazolidones and polyvinylimidazoles and/or mixtures thereof; chelating agents examples of which include ethylene-diamine-tetraacetic acid (EDTA); diethylene triamine penta methylene phosphonic acid (DTPMP); hydroxy-ethane diphosphonic acid (HEDP); ethylenediamine N,N'-disuccinic acid (EDDS); methyl glycine diacetic acid (MGDA); diethylene triamine penta acetic acid (DTPA); propylene diamine tetracetic acid (PDT A); 2-hydroxypyridine-N-oxide (HPNO); or methyl glycine diacetic acid (MGDA); glutamic acid N,N-diacetic acid (N,N-dicarboxymethyl glutamic acid tetrasodium salt (GLDA); nitrilotriacetic acid (NTA); 4,5-dihydroxy-m-benzenedisulfonic acid; citric acid and any salts thereof; N-hydroxyethylethylenediaminetri-acetic acid (HEDTA), triethylenetetraaminehexaacetic acid (TTHA), N-hydroxyethyliminodiacetic acid (HEIDA), dihydroxyethylglycine (DHEG), ethylenediaminetetrapropionic acid (EDTP) and derivatives thereof.

The composition can further comprise enzymes (generally about 0.01 wt% active enzyme to 0.5 wt% active enzyme) selected from proteases; amylases; lipases; cellulases; choline oxidases; peroxidases/oxidases; pectate lyases; mannanases; cutinases; laccases; phospholipases; lysophospholipases; acyltransferase; perhydrolase; arylesterase and any mixture thereof. The composition may comprise an enzyme stabilizer (examples of which include polyols such as propylene glycol or glycerol, sugar or sugar alcohol, lactic acid, reversible protease inhibitor, boric acid, or a boric acid derivative, e.g., an aromatic borate ester, or a phenyl boronic acid derivative such as 4-formylphenyl boronic acid).

The composition can further comprise silicone or fatty-acid based suds suppressors; heuing dyes, calcium and magnesium cations, visual signaling ingredients, anti-foam (0.001 wt% to about 4.0 wt%), and/or structurant/thickener (0.01 wt% to 5 wt%, selected from the group consisting of diglycerides and triglycerides, ethylene glycol distearate, microcrystalline cellulose, cellulose based materials, micro fiber cellulose, biopolymers, xanthan gum, gellan gum, and mixtures thereof).

The composition can be any liquid form, for example a liquid or gel form, or any combination thereof.

In some embodiments, the cleaning compositions of the present invention are provided in unit dose form, including tablets, capsules, sachets, pouches, and multi-compartment pouches. In some embodiments, the unit dose format is designed to provide controlled release of the ingredients within a multi-compartment pouch (or other unit dose format). Suitable unit dose and controlled release formats are known in the art (*See e.g.,* EP 2 100 949, WO 02/102955, US Pat. Nos. 4,765,916 and 4,972,017, and WO 04/111178 for materials suitable for use in unit dose and controlled release formats). In some embodiments, the unit dose form is provided by tablets wrapped with a water-soluble film or water-soluble pouches. Various unit dose formats are provided in EP 2 100 947 and WO2013/165725, and are known in the art.

In some embodiments, the cleaning composition is a high density powder (HDD) composition having a variant serine protease polypeptide protease. The HDD powder laundry detergent can comprise a detersive surfactant including anionic detersive surfactants (*e.g.,* linear or branched or random chain, substituted or unsubstituted alkyl sulphates, alkyl sulphonates, alkyl alkoxylated sulphate, alkyl phosphates, alkyl phosphonates, alkyl carboxylates and/or mixtures thereof), non-ionic detersive surfactant (*e.g.*,linear or branched or random chain, substituted or unsubstituted C8-C18 alkyl ethoxylates, and/or C6-C12 alkyl phenol alkoxylates), cationic detersive surfactants (*e.g.*,alkyl pyridinium compounds, alkyl quaternary ammonium compounds, alkyl quaternary phosphonium compounds, alkyl ternary sulphonium compounds, and mixtures thereof), zwitterionic and/or amphoteric detersive surfactants (*e.g*.,alkanolamine sulpho-betaines); ampholytic surfactants; semi-polar non-ionic surfactants and mixtures thereof; builders (phosphate free builders (*e.g.,* zeolite builders examples of which include zeolite A, zeolite X, zeolite P and zeolite MAP in the range of 0 wt% to less than 10 wt%); phosphate builders (*e.g*.,sodium tri-polyphosphate in the range of 0 wt% to less than 10 wt%); citric acid, citrate salts and nitrilotriacetic acid or salt thereof in the range of less than 15 wt%; silicate salt (sodium or potassium silicate or sodium meta-silicate in the range of 0 wt% to less than 10 wt%, or layered silicate (SKS-6)); carbonate salt (sodium carbonate and/or sodium bicarbonate in the range of 0 wt% to less than 10 wt%); and bleaching agents (including photobleaches *e.g.,* sulfonated zinc phthalocyanines, sulfonated aluminum phthalocyanines, xanthenes dyes, and mixtures thereof); hydrophobic or hydrophilic bleach activators (*e.g.,* dodecanoyl oxybenzene sulfonate, decanoyl oxybenzene sulfonate, decanoyl oxybenzoic acid or salts thereof, 3,5,5-trimethy hexanoyl oxybenzene sulfonate, tetraacetyl ethylene diamine-TAED, nonanoyloxybenzene sulfonate-NOBS, nitrile quats, and mixtures thereof); hydrogen peroxide; sources of hydrogen peroxide (*e.g.*,inorganic perhydrate salts examples of which include mono or tetra hydrate sodium salt of perborate, percarbonate, persulfate, perphosphate, or persilicate); preformed hydrophilic and/or hydrophobic peracids (*e.g*.,percarboxylic acids and salts, percarbonic acids and salts, perimidic acids and salts, and peroxymonosulfuric acids and salts) and mixtures thereof and/or bleach catalyst (*e.g*.,imine bleach boosters examples of which include iminium cations and polyions; iminium zwitterions; modified amines; modified amine oxides; N-sulphonyl imines; N-phosphonyl imines; N-acyl imines; thiadiazole dioxides; perfluoroimines; cyclic sugar ketones and mixtures thereof; metal-containing bleach catalyst (*e.g.,* copper, iron, titanium, ruthenium, tungsten, molybdenum, or manganese cations along with an auxiliary metal cation such as zinc or aluminum and a sequestrate such as ethylenediaminetetraacetic acid, ethylenediaminetetra (methylenephosphonic acid) and water-soluble salts thereof).

The composition can further comprise additional detergent ingredients including perfume microcapsules, starch encapsulated perfume accord, hueing agents, additional polymers including fabric integrity and cationic polymers, dye lock ingredients, fabric-softening agents, brighteners (for example C.I. Fluorescent brighteners), flocculating agents, chelating agents, alkoxylated polyamines, fabric deposition aids, and/or cyclodextrin.

In some embodiments, the cleaning composition is an automatic dishwashing (ADW) detergent composition having a serine protease of the present invention. The ADW detergent composition can comprise two or more non-ionic surfactants selected from a group of ethoxylated non-ionic surfactants, alcohol alkoxylated surfactants, epoxy-capped poly(oxyalkylated) alcohols, or amine oxide surfactants present in amounts from 0 to 10% by weight; builders in the range of 5-60% comprising either phosphate (mono-phosphates, di-phosphates, tri-polyphosphates or oligomeric-poylphosphates, preferred sodium tripolyphosphate-STPP or phosphate-free builders [amino acid based compounds, examples of which include MGDA (methyl-glycine-diacetic acid), and salts and derivatives thereof, GLDA (glutamic-N,Ndiacetic acid) and salts and derivatives thereof, IDS (iminodisuccinic acid) and salts and derivatives thereof, carboxy methyl inulin and salts and derivatives thereof and mixtures thereof, nitrilotriacetic acid (NTA), diethylene triamine penta acetic acid (DTPA), B-alaninediacetic acid (B-ADA) and their salts], homopolymers and copolymers of poly-carboxylic acids and their partially or completely neutralized salts, monomeric polycarboxylic acids and hydroxycarboxylic acids and their salts in the range of 0.5% to 50% by weight; sulfonated/carboxylated polymers (provide dimensional stability to the product) in the range of about 0.1 % to about 50% by weight; drying aids in the range of about 0.1 % to about 10% by weight (selected from polyesters, especially anionic polyesters optionally together with further monomers with 3 to 6 functionalities which are conducive to polycondensation, specifically acid, alcohol or ester functionalities, polycarbonate-, polyurethane- and/or polyurea-polyorganosiloxane compounds or precursor compounds thereof of the reactive cyclic carbonate and urea type); silicates in the range from about 1 % to about 20% by weight (sodium or potassium silicates for example sodium disilicate, sodium meta-silicate and crystalline phyllosilicates); bleach-inorganic (for example perhydrate salts such as perborate, percarbonate, perphosphate, persulfate and persilicate salts) and organic (for example organic peroxyacids including diacyl and tetraacylperoxides, especially diperoxydodecanedioc acid, diperoxytetradecanedioc acid, and diperoxyhexadecanedioc acid); bleach activators- organic peracid precursors in the range from about 0.1 % to about 10% by weight; bleach catalysts (selected from manganese triazacyclononane and related complexes, Co, Cu, Mn and Fe bispyridylamine and related complexes, and pentamine acetate cobalt(III) and related complexes); metal care agents in the range from about 0.1% to 5% by weight (selected from benzatriazoles, metal salts and complexes, and/or silicates); enzymes in the range from about 0.01 to 5.0mg of active enzyme per gram of automatic dishwashing detergent composition (acyl transferases, alpha-amylases, beta-amylases, alpha-galactosidases, arabinosidases, aryl esterases, beta-galactosidases, carrageenases, catalases, cellobiohydrolases, cellulases, chondroitinases, cutinases, endo-beta-1, 4-glucanases, endo-beta-mannanases, esterases, exo-mannanases, galactanases, glucoamylases, hemicellulases, hyaluronidases, keratinases, laccases, lactases, ligninases, lipases, lipoxygenases, mannanases, oxidases, pectate lyases, pectin acetyl esterases, pectinases, pentosanases, peroxidases, phenoloxidases, phosphatases, phospholipases, phytases, polygalacturonases, proteases, pullulanases, reductases, rhamnogalacturonases, beta-glucanases, tannases, transglutaminases, xylan acetyl-esterases, xylanases, xyloglucanases, and xylosidases, and any mixture thereof); and enzyme stabilizer components (selected from oligosaccharides, polysaccharides and inorganic divalent metal salts).

As indicated above, the cleaning compositions of the present invention are formulated into any suitable form and prepared by any process chosen by the formulator, nonlimiting examples of which are described in US Patent Nos. 5,879,584, 5,691,297, 5,574,005, 5,569,645, 5,565,422, 5,516,448, 5,489,392, 5,486,303, 4,515,705, 4,537,706, 4,515,707, 4,550,862, 4,561,998, 4,597,898, 4,968,451, 5,565,145, 5,929,022, 6,294,514 and 6,376,445. In some embodiments in which a low pH cleaning composition is desired, the pH of such composition is adjusted via the addition of an acidic material such as HCl.

The cleaning compositions disclosed herein find use in cleaning a *situs* (*e.g.,* a surface, item, dishware, or fabric). Typically, at least a portion of the situs is contacted with an embodiment of the present cleaning composition, in neat form or diluted in a wash liquor, and then the situs is optionally washed and/or rinsed. For purposes of the present invention, "washing" includes but is not limited to, scrubbing, and mechanical agitation. In some embodiments, the cleaning compositions are typically employed at concentrations of from about 500 ppm to about 15,000 ppm in solution. When the wash solvent is water, the water temperature typically ranges from about 5°C to about 90°C and, when the situs comprises a fabric, the water to fabric mass ratio is typically from about 1:1 to about 30:1.

Representative detergent formulations that beneficially include a serine protease polypeptide of the present invention include the detergent formulations found in WO2013063460, pages 78-152, and in particular the tables of pages 94 to 152. The serine proteases are normally incorporated into the detergent composition at a level of from 0.00001% to 10% of enzyme protein by weight of the composition. In some embodiments, the detergent composition comprises more than 0.0001%, 0.001%, 0.01%, or 0.1% of the serine protease by weight of the composition. In some embodiments, the detergent composition comprises less than 1%, 0.1%, 0.01%, or 0.001% of the serine protease by weight of the composition.

The present invention provides methods for cleaning or washing an item or surface (e.g., hard surface) in need of cleaning, including, but not limited to methods for cleaning or washing a dishware item, a tableware item, a fabric item, a laundry item, personal care item, etc.,, and methods for cleaning or washing a hard or soft surface (e.g., a hard surface of an item).

In some embodiments, the present invention provides a method for cleaning an item, object, or surface in need of cleaning, the method comprising contacting the item or surface (or a portion of the item or surface desired to be cleaned) with at least one serine protease polypeptide of the invention or a composition of the present invention for a sufficient time and/or under conditions suitable and/or effective to clean the item, object, or surface to a desired degree. Some such methods further comprise rinsing the item, object, or surface with water. For some such methods, the cleaning composition is a dishwashing detergent composition and the item or object to be cleaned is a dishware item or tableware item. As used herein, a "dishware item" is an item generally used in serving or eating food. A dishware item can be, but is not limited to for example, a dish, plate, cup, bowl, etc., and the like. As used herein, "tableware" is a broader term that includes, but is not limited to for example, dishes, cutlery, knives, forks, spoons, chopsticks, glassware, pitchers, sauce boats, drinking vessels, serving items, etc. It is intended that "tableware item" includes any of these or similar items for serving or eating food. For some such methods, the cleaning composition is an automatic dishwashing detergent composition or a hand dishwashing detergent composition and the item or object to be cleaned is a dishware or tableware item. For some such methods, the cleaning composition is a laundry detergent composition (*e.g.,* a power laundry detergent composition or a liquid laundry detergent composition), and the item to be cleaned is a fabric item. In some other embodiments, the cleaning composition is a laundry pre-treatment composition.

In some embodiments, the present invention provides methods for cleaning or washing a fabric item optionally in need of cleaning or washing, respectively. In some embodiments, the methods comprise providing a composition comprising the variant protease, including but not limited to fabric or laundry cleaning composition, and a fabric item or laundry item in need of cleaning, and contacting the fabric item or laundry item (or a portion of the item desired to be cleaned) with the composition under conditions sufficient or effective to clean or wash the fabric or laundry item to a desired degree.

In some embodiments, the present invention provides a method for cleaning or washing an item or surface (*e.g.,* hard surface) optionally in need of cleaning, the method comprising providing an item or surface to be cleaned or washed and contacting the item or surface (or a portion of the item or surface desired to be cleaned or washed) with at least one serine protease polypeptide of the invention or a composition of the invention comprising at least one such serine protease polypeptide for a sufficient time and/or under conditions sufficient or effective to clean or wash the item or surface to a desired degree. Such compositions include, but are not limited to for example, a cleaning composition or detergent composition of the invention (*e.g.,* a hand dishwashing detergent composition, hand dishwashing cleaning composition, laundry detergent or fabric detergent or laundry or fabric cleaning composition, liquid laundry detergent, liquid laundry cleaning composition, powder laundry detergent composition, powder laundry cleaning composition, automatic dishwashing detergent composition, laundry booster cleaning or detergent composition, laundry cleaning additive, and laundry pre-spotter composition, etc.). In some embodiments, the method is repeated one or more times, particularly if additional cleaning or washing is desired. For example, in some instance, the method optionally further comprises allowing the item or surface to remain in contact with the at least one variant protease or composition for a period of time sufficient or effective to clean or wash the item or surface to the desired degree. In some embodiments, the methods further comprise rinsing the item or surface with water and/or another liquid. In some embodiments, the methods further comprise contacting the item or surface with at least one variant protease of the invention or a composition of the invention again and allowing the item or surface to remain in contact with the at least one variant protease or composition for a period of time sufficient to clean or wash the item or surface to the desired degree. In some embodiments, the cleaning composition is a dishwashing detergent composition and the item to be cleaned is a dishware or tableware item. In some embodiments of the present methods, the cleaning composition is an automatic dishwashing detergent composition or a hand dishwashing detergent composition and the item to be cleaned is a dishware or tableware item. In some embodiments of the methods, the cleaning composition is a laundry detergent composition and the item to be cleaned is a fabric item.

The present invention also provides methods of cleaning a tableware or dishware item in an automatic dishwashing machine, the method comprising providing an automatic dishwashing machine, placing an amount of an automatic dishwashing composition comprising at least one serine protease polypeptide of the present invention or a composition of the invention sufficient to clean the tableware or dishware item in the machine (*e.g.,* by placing the composition in an appropriate or provided detergent compartment or dispenser in the machine), putting a dishware or tableware item in the machine, and operating the machine so as to clean the tableware or dishware item (*e.g.,* as per the manufacturer's instructions). In some embodiments, the methods include any automatic dishwashing composition described herein, which comprises, but is not limited to at least one serine protease polypeptide provided herein. The amount of automatic dishwashing composition to be used can be readily determined according to the manufacturer's instructions or suggestions and any form of automatic dishwashing composition comprising at least one variant protease of the invention (*e.g.,* liquid, powder, solid, gel, tablet, etc.), including any described herein, may be employed.

The present invention also provides methods for cleaning a surface, item or object optionally in need of cleaning, the method comprises contacting the item or surface (or a portion of the item or surface desired to be cleaned) with at least one serine protease polypeptide of the present invention or a cleaning composition of the invention in neat form or diluted in a wash liquor for a sufficient time and/or under conditions sufficient or effective to clean or wash the item or surface to a desired degree. The surface, item, or object may then be (optionally) washed and/or rinsed if desired.

The present invention also provides methods of cleaning a laundry or fabric item in an washing machine, the method comprising providing an washing machine, placing an amount of a laundry detergent composition comprising at least one serine protease polypeptide enzyme of the invention sufficient to clean the laundry or fabric item in the machine (*e.g.,* by placing the composition in an appropriate or provided detergent compartment or dispenser in the machine), placing the laundry or fabric item in the machine, and operating the machine so as to clean the laundry or fabric item (*e.g.,* as per the manufacturer's instructions). The methods of the present invention include any laundry washing detergent composition described herein, comprising but not limited to at least one of any serine protease polypeptide enzyme provided herein. The amount of laundry detergent composition to be used can be readily determined according to manufacturer's instructions or suggestions and any form of laundry detergent composition comprising at least one variant protease of the invention (*e.g.,* solid, powder, liquid, tablet, gel, etc.), including any described herein, may be employed.

### B. Textile Processing

Also provided are compositions and methods of treating fabrics (*e.g.,* to desize a textile) using a serine protease polypeptide of the present invention. Fabric-treating methods are well known in the art (see, e.g., U.S. Patent No. 6,077,316). For example, the feel and appearance of a fabric can be improved by a method comprising contacting the fabric with a serine protease in a solution. The fabric can be treated with the solution under pressure.

A serine protease of the present invention can be applied during or after the weaving of a textile, or during the desizing stage, or one or more additional fabric processing steps. During the weaving of textiles, the threads are exposed to considerable mechanical strain. Prior to weaving on mechanical looms, warp yarns are often coated with sizing starch or starch derivatives to increase their tensile strength and to prevent breaking. A serine protease of the present invention can be applied during or after the weaving to remove these sizing starch or starch derivatives. After weaving, the serine protease can be used to remove the size coating before further processing the fabric to ensure a homogeneous and wash-proof result.

A serine protease of the present invention can be used alone or with other desizing chemical reagents and/or desizing enzymes to desize fabrics, including cotton-containing fabrics, as detergent additives, *e.g.,* in aqueous compositions. An amylase also can be used in compositions and methods for producing a stonewashed look on indigo-dyed denim fabric and garments. For the manufacture of clothes, the fabric can be cut and sewn into clothes or garments, which are afterwards finished. In particular, for the manufacture of denim jeans, different enzymatic finishing methods have been developed. The finishing of denim garment normally is initiated with an enzymatic desizing step, during which garments are subjected to the action of proteolytic enzymes to provide softness to the fabric and make the cotton more accessible to the subsequent enzymatic finishing steps. The serine protease can be used in methods of finishing denim garments (*e.g.,* a "bio-stoning process"), enzymatic desizing and providing softness to fabrics, and/or finishing process.

### C. Leather and Feather Processing

The serine protease polypeptides described herein find further use in the enzyme aided removal of proteins from animals and their subsequent degradation or disposal, such as feathers, skin, hair, hide, and the like. In some instances, immersion of the animal carcass in a solution comprising a serine protease polypeptide of the present invention can act to protect the skin from damage in comparison to the traditional immersion in scalding water or the defeathering process. In one embodiment, feathers can be sprayed with an isolated serine protease polypeptide of the present invention under conditions suitable for digesting or initiating degradation of the plumage. In some embodiments, a serine protease of the present invention can be used, as above, in combination with an oxidizing agent.

In some embodiments, removal of the oil or fat associated with raw feathers is assisted by using a serine protease polypeptide of the present invention. In some embodiments, the serine protease polypeptides are used in compositions for cleaning the feathers as well as to sanitize and partially dehydrate the fibers. In yet other embodiments, the disclosed serine protease polypeptides find use in recovering protein from plumage. In some other embodiments, the serine protease polypeptides are applied in a wash solution in combination with 95% ethanol or other polar organic solvent with or without a surfactant at about 0.5% (v/v).

### D. Animal Feeds

In a further aspect of the invention, the serine protease polypeptides of the present invention can be used as a component of an animal feed composition, animal feed additive and/or pet food comprising a serine protease and variants thereof. The present invention further relates to a method for preparing such an animal feed composition, animal feed additive composition and/or pet food comprising mixing the serine protease polypeptide with one or more animal feed ingredients and/or animal feed additive ingredients and/or pet food ingredients. Furthermore, the present invention relates to the use of the serine protease polypeptide in the preparation of an animal feed composition and/or animal feed additive composition and/or pet food.

The term "animal" includes all non-ruminant and ruminant animals. In a particular embodiment, the animal is a non-ruminant animal, such as a horse and a mono-gastric animal. Examples of mono-gastric animals include, but are not limited to, pigs and swine, such as piglets, growing pigs, sows; poultry such as turkeys, ducks, chicken, broiler chicks, layers; fish such as salmon, trout, tilapia, catfish and carps; and crustaceans such as shrimps and prawns. In a further embodiment the animal is a ruminant animal including, but not limited to, cattle, young calves, goats, sheep, giraffes, bison, moose, elk, yaks, water buffalo, deer, camels, alpacas, llamas, antelope, pronghorn and nilgai.

In the present context, it is intended that the term "pet food" is understood to mean a food for a household animal such as, but not limited to, dogs, cats, gerbils, hamsters, chinchillas, fancy rats, guinea pigs; avian pets, such as canaries, parakeets, and parrots; reptile pets, such as turtles, lizards and snakes; and aquatic pets, such as tropical fish and frogs.

The terms "animal feed composition," "feedstuff and "fodder" are used interchangeably and can comprise one or more feed materials selected from the group comprising a) cereals, such as small grains (e.g., wheat, barley, rye, oats and combinations thereof) and/or large grains such as maize or sorghum; b) by products from cereals, such as corn gluten meal, Distillers Dried Grain Solubles (DDGS) (particularly corn based Distillers Dried Grain Solubles (cDDGS), wheat bran, wheat middlings, wheat shorts, rice bran, rice hulls, oat hulls, palm kernel, and citrus pulp; c) protein obtained from sources such as soya, sunflower, peanut, lupin, peas, fava beans, cotton, canola, fish meal, dried plasma protein, meat and bone meal, potato protein, whey, copra, sesame; d) oils and fats obtained from vegetable and animal sources; e) minerals and vitamins.

### EXAMPLES

The following examples are provided to demonstrate and illustrate certain preferred embodiments and aspects of the present disclosure and should not be construed as limiting.

In the experimental disclosure which follows, the following abbreviations apply: ADW (automatic dish washing); BMI (blood/milk/ink); BSA (bovine serum albumin); CAPS (*N*-cyclohexyl-3-aminopropanesulfonic acid); CHES (N-cyclohexyl-2-aminoethanesulfonic acid); DMC (dimethyl casein); HDD (heavy duty dry/powder); HDL (heavy duty liquid); HEPES (4-(2-hydroxyethyl)-1-piperazineethanesulfonic acid); MTP (microtiter plate); ND (not done); OD (optical density); PCR (polymerase chain reaction); ppm (parts per million); QS (quantity sufficient); rpm (revolutions per minute); AAPF (succinyl-Ala-Ala-Pro-Phe-p-nitroanilide); TNBSA (2,4,6-trinitrobenzene sulfonic acid); v/v (volume to volume); w/v (weight to volume).

### EXAMPLE 1

### Protein Determination Methods

### Protein Determination by Stain Free Imager Criterion

Protein was quantified by the stain-free Imager Criterion method. This method utilizes stain-free precast PAGE gels, where the intensity of each band depends on the amount of tryptophan residues present in the protein of interest. The Criterion™ TGX (Tris-Glycine extended) Stain-Free™ precast gels for PAGE include unique trihalo compounds. This allows rapid fluorescent detection of proteins with the Gel Doc™ EZ imaging system. The trihalo compounds react with tryptophan residues in a UV-induced reaction to produce fluorescence, which can be easily detected by the Gel Doc EZ imager within gels. Reagents used in the assay: Concentrated (10x) Laemmli Sample Buffer (Kem-En-Tec, Catalogue #42556); either 18 or 26-well Criterion TGX Strain-Free Precast gels (Bio-Rad, Catalogue #567-8124 and 567-8125, respectively); and protein markers "Precision Plus Protein Standards" (Bio-Rad, Catalogue #161- 0363). The assay was carried out as follow: 25µl protein sample and 25µl 0.5M HCL was added to a 96well-PCR plate on ice for 10 min to inactivate the protease and prevent self-hydrolysis. Then, 50µl of the acid protein mix was added to a 50 µL sample buffer containing 0.385 mg DTT in the 96well-PCR plate. The plate was sealed by Microseal 'B' Film from Bio-Rad and was placed in the PCR machine and heated to 70°C for 10 minutes. After that, the chamber was filled by running buffer and the gel cassette was set. Then, 10 µL of each sample together with markers was load in each pocket. After that the electrophoresis was started at 200 V for 35 min. Following electrophoresis, the gel was transferred to the Imager. Image Lab software was used to calculate the intensity of each band. By knowing the protein amount and the tryptophan content of the standard sample, the calibration curve can be made. The amount of experimental sample can be determined by extrapolation of the band intensity and tryptophan numbers to protein concentration. This protein quantification method was employed to prepare the sample BspAG00296 and BspM04033 proteases used in the assays set forth in the Examples.

### N and C-terminal amino acid determination

In preparation for sequence confirmation, a sample of isolated protein may be subjected to a series of chemical treatments in a 10 kDa spinfilter. The sample is denatured and reduced by urea and DTT treatment. A guanidination step was performed to convert lysines to homoarginines to protect lysine side chains from acetylation. Acetylation reaction using iodoacetamide then modifies only the proteins' N-terminal residue. The sample is then mixed with a buffer containing ¹⁸O water and the enzymes trypsin and chymotrypsin are added for digestion. The resulting peptides will contain mixtures of ¹⁸O and ¹⁶O, except for the Carboxyl terminus which will retain the native ¹⁶O. The digestion products were separated and analyzed using a nano-LC system followed by LTQ Orbitrap (Thermo Fisher) high resolution mass spectrometer and the amino acid sequence was deduced from the MS/MS fragment spectrum of the peptides, and the isotopic pattern of the peptides.

In some instances, the protein sample was run on SDS-PAGE gel, as described below, prior to analysis by LC-MS/MS. In preparation for sequence confirmation, including N- and C-terminal determination, a protein band from an SDS-PAGE gel was then subjected to a series of chemical treatments as described below. Between the individual chemical treatment, the gel pieces were washed using distilled water, and ethanol. The protein was reduced/alkylated by DTT/Iodoacetamide treatment. A guanidination step was performed to convert lysines to homoarginines to protect lysine side chains from acetylation. The acetylation reaction using Sulfo-NHS-Acetate only modifies the N-terminal residue. The gel pieces were swelled with a buffer containing ¹⁸O: ¹⁶O water and chymotrypsin or trypsin for protein digestion. Subsequent steps described above for samples not requiring in-gel treatment were followed.

### EXAMPLE 2

### Discovery and Identification of serine protease BspAG00296

*Bacillus* sp. 1M5 (Culture Collection Dupont) was selected as a potential source for enzymes useful in industrial applications. To identify enzymes produced by *Bacillus* sp. 1M5 and the genes that encode these enzymes, the entire genome of *Bacillus* sp. 1M5 was sequenced using Illumina® sequencing by synthesis (SBS) technology. Genome sequencing and assembly of the sequence data was performed by BaseClear (Leiden, The Netherlands). Contigs were annotated by BioXpr (Namur, Belgium). One of the genes identified this way in strain *Bacillus* sp. 1M5 encodes a protein that showed homology to serine proteases of various other bacteria. The sequence of this gene, BspAG00296.n, is depicted in SEQ ID NO:1.
SEQ ID NO:1 sets forth the nucleotide sequence of the *BspAG00296.n* gene: 
ATGAA
GAAGTTCTTATGTCTGTCGGTGTTGATGTTGGTTTATCTGTGTTTCTGGCAATGTG
TTGGCGAATGATGAGGTCAAAAAGGAAGATTATGTTGACGGGCAGTTGATTGTTTC
AGTGGACGCAAGCTTTGACTCAAAAGGGAAGCCGATGCTTCAAGCATTGACAAGCA
CCTCGAAGCTGTTGAATGCAGAATTGAAGAAAAACGGTTTTGAAGTAGCGGATTCG
CTGCTGGAAGTGAAGGGAAATGATTCCGTCGATATTTTCAGCGACAGCTTTAAAGA
GGAGGCAGCAAAAAATACCGGATTTGTTTACCTTGTAGAATATTCTACAGATGCTTA
TGCTTCCATCGATGATGCGAAGAAGGCGCTCGAAAAACAGTTAACGGACATCGGCT
TAAAAGTAAAATATGTCTCTGAAAACTTTACAGTCGAGCTGTCGGCCGAAGCGGCT
GAAGAGGTAATACAGCCGGCAATGCATGCTAATCAGCGCTGGCATTATGAAATGAT
TCGGGCGCCGCAAGCTTGGAATATTACGACCGGCAGCAGGAATGTTCGAATGGCGG
TGCTTGATACAGGAATTGATTCATCACATCCGAACTTAGCAAACCTTGTGAATACAA
GCTTGGGGAGGAGCTTTGTCGGCGGAACGCCTGCTGATGTACACGGACATGGGACT
CATGTTGCCGGTACGATTGCCAGCTACGGCTCCGTATCAGGTGTTATGCAAAACGCT
ACGCTTATTTCCGTAAAAGTATTGGATAACAGCGGCAGCGGCACAATTTATGGCAT
CCAGCAAGGCATTCTGTATGCCGCGAGCATTAACGCCGATGTAATCAACATGTCCTT
GGGAGGCGGCAGCTACAATCAAGGAATGAATGATGCGATTCAGACAGCCGTTAATT
CCGGAACAGTTGTCGTGGCTGCGTCAGGAAACAACGGGGCATCAAGCATTTCCTAC
CCTGCCGCTTACAGCGGAGCGATTGCTGTCGGTTCCGTGACATCCAGCCGGACAAG
ATCAAGCTTCTCCAACTATGGATCAGGCTTAGAGTTAATGGCTCCTGGCTCCAATAT
TTACAGCACATATCCAAACAGCCGGTATGCCACGCTATCCGGAACATCAATGGCAA
CGCCGCATGTTGCCGGGGTCGCCGGGTTAATCCGCTCGGTCAATCCTAATCTTTCCG
CGGCGCAAGTAAGAACGATTTTGCGGAATACGGCTCAATACGCAGGCAGCTCCACG
CAGTACGGCTATGGAATCGTCGATGCGTATGCTGCGGTACTCTCAGCCCGC.
   The preproenzyme encoded by the *BspAG00296.n* gene is depicted in SEQ ID NO:2. At the N-terminus, the protein has a signal peptide with a length of 23 amino acids as predicted by SignalP-NN (Emanuelsson et al., Nature Protocols (2007) 2: 953-971). This signal peptide sequence is underlined and in bold in SEQ ID NO:2. The presence of a signal peptide indicates that this serine protease is a secreted enzyme. The enzyme has a pro sequence which is predicted to be 135 amino acids. The sequence of the predicted, fully processed mature chain (BspAG00296, 274 amino acids) is depicted in SEQ ID NO:3.
SEQ ID NO:2 sets forth the amino acid sequence of the serine protease precursor BspAG00296: **MKKFLCLSVLMLVLSVFSGNVLA***NDEVKKEDYVDGQLIVSVDASFDSKG KPMLQALTSTSKLLNAELKKNGFEVADSLLEVKGNDSVDIFSDSFKEEAAKNTGFVYLVEYST DAYASIDDAKKALEKQLTDIGLKVKYVSENFTVELSAEAAEEVIQPAMHANQRWHYEMIRA* PQAWNITTGSRNVRMAVLDTGIDSSHPNLANLVNTSLGRSFVGGTPADVHGHGTHVAG TIASYGSVSGVMQNATLISVKVLDNSGSGTIYGIQQGILYAASINADVINMSLGGGSYNQ GMNDAIQTAVNSGTVVVAASGNNGASSISYPAAYSGAIAVGSVTSSRTRSSFSNYGSGL ELMAPGSNIYSTYPNSRYATLSGTSMATPHVAGVAGLIRSVNPNLSAAQVRTILRNTAQ YAGSSTQYGYGIVDAYAAVLSAR
SEQ ID NO:3 sets forth the predicted amino acid sequence of the mature protease BspAG00296 (274 amino acids): AMHANQRWHYEMIRAPQAWNITTGSRNVRMAVLDTG IDSSHPNLANLVNTSLGRSFVGGTPADVHGHGTHVAGTIASYGSVSGVMQNATLISVKV LDNSGSGTIYGIQQGILYAASINADVINMSLGGGSYNQGMNDAIQTAVNSGTVVVAASG NNGASSISYPAAYSGAIAVGSVTSSRTRSSFSNYGSGLELMAPGSNIYSTYPNSRYATLS GTSMATPHVAGVAGLIRSVNPNLSAAQVRTILRNTAQYAGSSTQYGYGIVDAYAAVLS AR.

### EXAMPLE 3

### Heterologous expression of BspAG00296

BspAG00296 protease was produced in *B. subtilis* using an expression cassette consisting of the *B. subtilis aprE* promoter, the *B. subtilis aprE* signal peptide sequence, the native BspAG00296 protease pro-peptide, the mature BspAG00296 protease and a BPN' terminator. This cassette was cloned into the pHYT replicating shuttle vector and transformed into a suitable *B. subtilis* strain. The pHYT vector was derived from pHY300PLK (Takara) by adding a terminator after the tetracycline resistance gene using the *Bst*EII and *Eco*RI sites (terminator sequence, GGTTACCTTGAATGTATATAAACATTCTCAAAGGGATTTCTAAT AAAAAACGCTCGGTTGCCGCCGGGCGTTTTTTATGCATCGATGGAATTC) (SEQ ID NO:45). The *Hind*III site in pHY300PLK was also removed using a linker cloned into the BamHI and HindIII sites (new linker sequence, GGATCCTGACTGCCTGAGCTT) (SEQ ID NO:46).

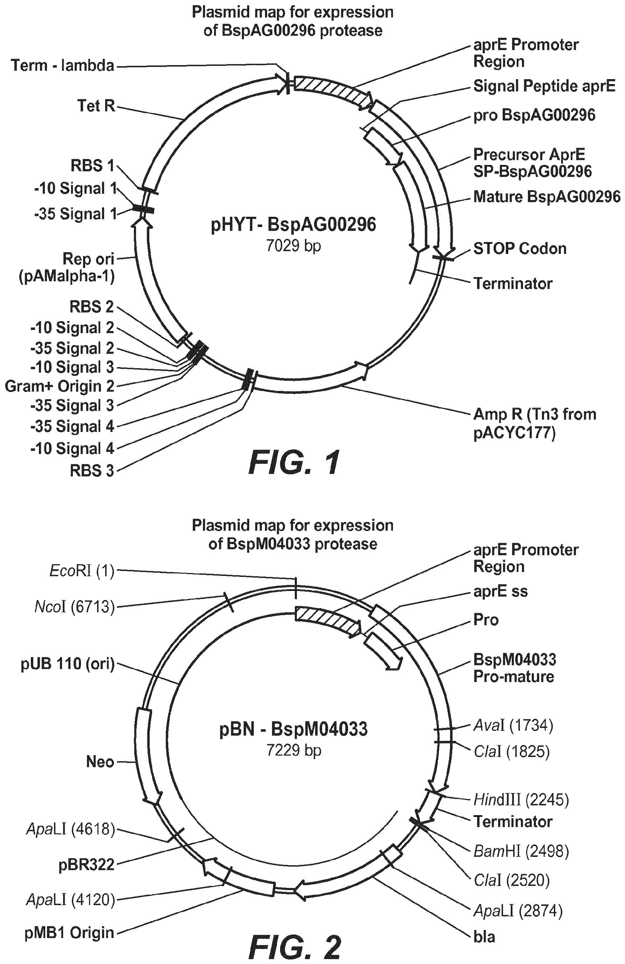

A map of the pHYT vector containing the BspAG00296 gene (pHYT- BspAG00296) is shown in Figure 1.

To produce BspAG00296, a *B. subtilis* transformant containing pHYT- BspAG00296 was cultivated in an enriched semi-defined media based on MOPs buffer, with urea as major nitrogen source, glucose as the main carbon source, and supplemented with 1% soytone for robust cell growth. The media was supplemented with 25ppm tetracycline. After incubation (2 days at 32°C), BspAG00296 protease was detected in the growth medium. After centrifugation and filtration, culture supernatants with BspAG00296 protease were used for assays and purification.

Samples of BspAG00296 protein were analyzed as described in Example 1. The sequence of the most prominent protein (approximately 28 kDa) was determined to correspond to sequence listed in SEQ ID NO:4.

SEQ ID NO:4 sets forth the amino acid sequence of the predominant form of mature protease BspAG00296 (273 residues):MHANQRWHYEMIRAPQAWNITTGSRNVRMAVLDTGIDSSHPNLANLVNTSLGRSFVGGTPADVHGHGTHVAGTIASYGSVSGVMQNATLISVKVLDNSGSGTIYGIQQGILYAASINADVINMSLGGGSYNQGMNDAIQTAVNSGTVVVAASGNNGASSISYPAAYSGAIAVGSVTSSRTRSSFSNYGSGLELMAPGSNIYSTYPNSRYATLSGTSMATPHVAGVAGLIRSVNPNLSAAQVRTILRNTAQYAGSSTQYGYGIVDAYAAVLSAR.

### COMPARATIVE EXAMPLE 4

### Discovery and Identification of serine protease BspM04033

*Bacillus sp.* WDG290 (Culture Collection Dupont) was selected as a potential source for enzymes useful in industrial applications. To identify enzymes produced by *Bacillus sp.* WDG290 and the genes that encode these enzymes, the entire genome of *Bacillus sp.* WDG290 was sequenced using Illumina® sequencing by synthesis (SBS) technology. Genome sequencing and assembly of the sequence data was performed by BaseClear (Leiden, The Netherlands). Contigs were annotated by BioXpr (Namur, Belgium). One of the genes identified this way in strain *Bacillus sp.* WDG290 encodes a protein that showed homology to serine proteases of various other bacteria. The sequence of this gene, *BspM04033.n,* is depicted in SEQ ID NO:5.
SEQ ID NO:5 sets forth the nucleotide sequence of the *BspM04033.n* gene: ATGGAGGAGAAAAATGTGAAAAAAAGTGCAGTTTGGGTCCTTATGACGGTGTTGGTTTTCAGTCTGTTTTTAAATCCTGCCGGAATTGGCGCGCAGGCCTCTGATGCAGCTTCAGAAAAAGATGACACTGCCTACATAGAGGGGCAGTTGATTGTATCGGTAAAGAGCAGTGACGTTTCAGTGAAGGGAATCGAAGGGGTAAACAAGAAGATCATGGGCGATGTCCTGAGAGAACGGGGATTCGCCATAACGGATTCTATTATGGGACTCGGCGATCCTGCTGAAGTGAATGCCTTTACGAACCAGGAGTTCAGTGAATCCGTCGTGAAGAATATGGGGCTCGTTTACCTTGCAGAATACGATGTGTCTGTTTATGCATCAGTAGAAGAAGCGAAACGGGAGCTGGCCGAAGCGCTCAAAGAGAACGGAATGGAAATCAGACACATCTCGAAGAACTATGAAATGCACGCGATCGGGGAACCTGCCGATGTCTCTCCCCAGATGCACCCGAACCAGCAGTGGCATTACAACATGATTAATGCACCGCAGGCGTGGGGGACAACGACAGGCTCCTCAAGTGTCATTCAGGCTGTGCTTGATACGGGGATTGACCACAATCATCAGAGTCTCGCAAACTTAGTAAACACAAGTCTCGGACAGAGCTTTGTGGGCGGAAGTACGATGGATGTTCAAGGGCACGGAACGCACGTTGCCGGTACGATTGCAAGCTACGGTTCTGTGTCCGGCGTGATGCACAATGCTACGCTCGTACCGGTTAAAGTGCTGAATGACAGTGGATCAGGGTCACTTTTCGGCATTACGCAGGGAATCCTGTATTCAGCTGATATCGGGGCCGACGTGATCAACATGTCTCTTGGCGGCGGCGGTTACAACCAGAGTATGGCAGAAGCTGCACAGACAGCGGTAAATGCCGGTTCGATTGTAATTGCGGCAAGCGGAAATGACGGAGCGGGCAGTATTTCGTATCCGGCAGCGTACAGCAGCGTCATTGCGGTTGGGTCTGTAACCTCGACAGGTGCCCGTTCCAACTTCTCAAACTACGGCAGCGGACTTGAACTGATGGCACCTGGTTCAAATATTTACAGCACCGTACCGAATAACGGCTATGCCACATTCTCGGGTACGTCGATGGCATCCCCGCATGCAGCAGGTGTTGCCGGTCTGATGAGAGCGGTCAATCCGAATCTATCGGTATCGAATGCCAGATCGATTATGCAGAACACGGCTCAGTATGCCGGAAGCCCGACTTTCTACGGGTACGGGATCGTTGACGCGAACGCAGCGGTTCAGCAGGCATCAGGGGGAAGCGGCGGTCCTTCCAATATTACTGAAACGAGTATATCCACTGACCGTTTCTATGTGCAGCGAGGTCAGAACGTGACGTCAACTGCTCAGGTTACGAATGAAAACGGACAGGGTCTTGCCAACGCGACGGTGACCTTCACCATCACCCGTCCAAACGGATCAACGCTTACGAATACAGCAACGACCAACAGTTCCGGTTTCGCCTCATGGACGGTCGGCACATCCGGTGCCACCGCAACAGGCACCTATTCAGTAGAAGCATCATCTTCTCTTCAGGGGTATCAGGGAAGTTCCGCTTCAACGAGTTTCTTTGTTTAC.
   The pre-proenzyme encoded by the *BspM04033.n* gene is depicted in SEQ ID NO:6. At the N-terminus, the protein has a signal peptide with a length of 33 amino acids as predicted by SignalP-NN (Emanuelsson et al., Nature Protocols (2007) 2: 953-971). This signal peptide sequence is underlined and in bold in SEQ ID NO:6. The presence of a signal peptide indicates that this serine protease is a secreted enzyme. The enzyme has a pro sequence which is predicted to be 133 amino acids (This prediction is based on the pro-mature junction in a *Paenibacillus* subtilisin: WO2012175708, SEQ ID NO:6). The sequence of the predicted, processed mature chain (BspM04033, 382 amino acids) is depicted in SEQ ID NO:7.
SEQ ID NO:6 sets forth the amino acid sequence of the serine protease precursor BspM04033: **MEEKNVKKSAVWVLMTVLVFSLFLNPAGIGAQA***SDAASEKDDTAYIEGQLIVSVKSSDVSVKGIEGVNKKIMGDVLRERGFAITDSIMGLGDPAEVNAFTNQEFSESVVKNMGLVYLAEYDVSVYASVEEAKRELAEALKENGMEIRHISKNYEMHAIGEPADVSP*QMHPNQQWHYNMINAPQAWGTTTGSSSVIQAVLDTGIDHNHQSLANLVNTSLGQSFVGGSTMDVQGHGTHVAGTIASYGSVSGVMHNATLVPVKVLNDSGSGSLFGITQGILYSADIGADVINMSLGGGGYNQSMAEAAQTAVNAGSIVIAASGNDGAGSISYPAAYSSVIAVGSVTSTGARSNFSNYGSGLELMAPGSNIYSTVPNNGYATFSGTSMASPHAAGVAGLMRAVNPNLSVSNARSIMQNTAQYAGSPTFYGYGIVDANAAVQQASGGSGGPSNITETSISTDRFYVQRGQNVTSTAQVTNENGQGLANATVTFTITRPNGSTLTNTATTNSSGFASWTVGTSGATATGTYSVEASSSLQGYQGSSASTSFFVY.
SEQ ID NO:7 sets forth the amino acid sequence of the predicted mature protease BspM04033 (382 amino acids): QMHPNQQWHYNMINAPQAWGTTTGSSSVIQAVLDTGIDHNHQSLANLVNTSLGQSFVGGSTMDVQGHGTHVAGTIASYGSVSGVMHNATLVPVKVLNDSGSGSLFGITQGILYSADIGADVINMSLGGGGYNQSMAEAAQTAVNAGSIVIAASGNDGAGSISYPAAYSSVIAVGSVTSTGARSNFSNYGSGLELMAPGSNIYSTVPNNGYATFSGTSMASPHAAGVAGLMRAVNPNLSVSNARSIMQNTAQYAGSPTFYGYGIVDANAAVQQASGGSGGPSNITETSISTDRFYVQRGQNVTSTAQVTNENGQGLANATVTFTITRPNGSTLTNTATTNSSGFASWTVGTSGATATGTYSVEASSSLQGYQGSSASTSFFVY.

### COMPARATIVE EXAMPLE 5

### Heterologous expression of BspM04033

BspM04033 protease was produced in *B. subtilis* using an expression cassette consisting of the *B. subtilis aprE* promoter, the *B. subtilis aprE* signal peptide sequence, the native BspM04033 protease pro-peptide, the mature BspM04033 protease and a BPN' terminator. This cassette was cloned into the pBN based replicating shuttle vector (Babe' et al. (1998), Biotechnol. Appl. Biochem. 27: 117-124) and a suitable strain of *B. subtilis* was transformed using the plasmid.

A map of the pBN vector containing the BspM04033 gene (pBN- BspM04033) is shown in Figure 2.

The nucleotide pro-mature sequence of the BspM04033 gene in plasmid pBN-BspM04033 is depicted in SEQ ID NO:8: TCTGATGCAGCTTCAGAAAAAGATGACACTGCCTACATAGAGGGGCAGTTGATTGTATCGGTAAAGAGCAGTGACGTTTCAGTGAAGGGAATCGAAGGGGTAAACAAGAAGATCATGGGCGATGTCCTGAGAGAACGGGGATTCGCCATAACGGATTCTATTATGGGACTCGGCGATCCTGCTGAAGTGAATGCCTTTACGAACCAGGAGTTCAGTGAATCCGTCGTGAAGAATATGGGGCTCGTTTACCTTGCAGAATACGATGTGTCTGTTTATGCATCAGTAGAAGAAGCGAAACGGGAGCTGGCCGAAGCGCTCAAAGAGAACGGAATGGAAATCAGACACATCTCGAAGAACTATGAAATGCACGCGATCGGGGAACCTGCCGATGTCTCTCCCCAGATGCACCCGAACCAGCAGTGGCATTACAACATGATTAATGCACCGCAGGCGTGGGGGACAACGACAGGCTCCTCAAGTGTCATTCAGGCTGTGCTTGATACGGGGATTGACCACAATCATCAGAGTCTCGCAAACTTAGTAAACACAAGTCTCGGACAGAGCTTTGTGGGCGGAAGTACGATGGATGTTCAAGGGCACGGAACGCACGTTGCCGGTACGATTGCAAGCTACGGTTCTGTGTCCGGCGTGATGCACAATGCTACGCTCGTACCGGTTAAAGTGCTGAATGACAGTGGATCAGGGTCACTTTTCGGCATTACGCAGGGAATCCTGTATTCAGCTGATATCGGGGCCGACGTGATCAACATGTCTCTTGGCGGCGGCGGTTACAACCAGAGTATGGCAGAAGCTGCACAGACAGCGGTAAATGCCGGTTCGATTGTAATTGCGGCAAGCGGAAATGACGGAGCGGGCAGTATTTCGTATCCGGCAGCGTACAGCAGCGTCATTGCGGTTGGGTCTGTAACCTCGACAGGTGCCCGTTCCAACTTCTCAAACTACGGCAGCGGACTTGAACTGATGGCACCTGGTTCAAATATTTACAGCACCGTACCGAATAACGGCTATGCCACATTCTCGGGTACGTCGATGGCATCCCCGCATGCAGCAGGTGTTGCCGGTCTGATGAGAGCGGTCAATCCGAATCTATCGGTATCGAATGCCAGATCGATTATGCAGAACACGGCTCAGTATGCCGGAAGCCCGACTTTCTACGGGTACGGGATCGTTGACGCGAACGCAGCGGTTCAGCAGGCATCAGGGGGAAGCGGCGGTCCTTCCAATATTACTGAAACGAGTATATCCACTGACCGTTTCTATGTGCAGCGAGGTCAGAACGTGACGTCAACTGCTCAGGTTACGAATGAAAACGGACAGGGTCTTGCCAACGCGACGGTGACCTTCACCATCACCCGTCCAAACGGATCAACGCTTACGAATACAGCAACGACCAACAGTTCCGGTTTCGCCTCATGGACGGTCGGCACATCCGGTGCCACCGCAACAGGCACCTATTCAGTAGAAGCATCATCTTCTCTTCAGGGGTATCAGGGAAGTTCCGCTTCAACGAGTTTCTTTGTTTAC.

The amino acid sequence of the BspM04033 precursor protein expressed from plasmid pBN-BspM04033 is depicted in SEQ ID NO:9 with the predicted pro-peptide is shown in underlined text: SDAASEKDDTAYIEGOLIVSVKSSDVSVKGIEGVNKKIMGDVLRERGFAITDSIMGLGDPAEVNAFTNQEFSESVVKNMGLVYLAEYDVSVYASYEEAKRELAEALKENGMEIRHISKNYEMHAIGEPADVSPQMHPNQQWHYNMINAPQAWGTTTGSSSVIQA VLDTGIDHNHQSLANLVNTSLGQSFVGGSTMDVQGHGTHVAGTIASYGSVSGVMHNATLVPVKVLNDSGSGSLFGITQGILYSADIGADVINMSLGGGGYNQSMAEAAQTAVNAGSIVIAASGNDGAGSISYPAAYSSVIAVGSVTSTGARSNFSNYGSGLELMAPGSNIYSTVPNNGYATFSGTSMASPHAAGVAGLMRAVNPNLSVSNARSIMQNTAQYAGSPTFYGYGIVDANAAVQQASGGSGGPSNITETSISTDRFYVQRGQNVTSTAQVTNENGQGLANATVTFTITRPNGSTLTNTATTNSSGFASWTVGTSGATATGTYSVEASSSLQGYQGSSASTSFFVY.

To produce BspM04033, a *B. subtilis* transformant containing pBN-BspM04033 was cultured in 15 ml Falcon tubes for 16 hours in TSB (broth) with 10 ppm neomycin, and 300 µl of this pre-culture was added to a 500 mL flask filled with 30 mL of cultivation media (described below) supplemented with 10 ppm neomycin. The flasks were incubated for 48 hours at 32°C with constant rotational mixing at 180 rpm. Cultures were harvested by centrifugation at 14500 rpm for 20 minutes in conical tubes. The culture supernatants were used for assays. The cultivation media was an enriched semi-defined media based on MOPs buffer, with urea as major nitrogen source, glucose as the main carbon source, and supplemented with 1% soytone for robust cell growth.

Samples of BspM04033 protein were analyzed as described in Example 1. The samples contained two predominant forms of the enzyme, one approximately 44 kDa and the other approximately 28 kDa. The larger protein corresponds to the full length processed protease region devoid of pro sequence, and the subsequent residue, Q1 (Gln) as shown in SEQ ID NO:10. SEQ ID NO:10 shows the observed full length BspM04033 protein expressed from plasmid pBN-BspM04033 (381 amino acids): MHPNQQWHYNMINAPQAWGTTTGSSSVIQAVLDTGIDHNHQSLANLVNTSLGQSFVGGSTMDVQGHGTHVAGTIASYGSVSGVMHNATLVPVKVLNDSGSGSLFGITQGILYSADIGADVINMSLGGGGYNQSMAEAAQTAVNAGSIVIAASGNDGAGSISYPAAYSSVIAVGSVTSTGARSNFSNYGSGLELMAPGSNIYSTVPNNGYATFSGTSMASPHAAGVAGLMRAVNPNLSVSNARSIMQNTAQYAGSPTFYGYGIVDANAAVQQASGGSGGPSNITETSISTDRFYVQRGQNVTSTAQVTNENGQGLANATVTFTITRPNGSTLTNTATTNSSGFASWTVGTSGATATGTYSVEASSSLQGYQGSSASTSFFVY.

The sequence of the most prominent protein sample upon sample storage (approximately 28 kDa) was determined to correspond to a C-terminal truncated form, sequence listed in SEQ ID:11. This polypeptide is devoid of the Gln1 that follows the predicted pro region, and is consistent in length with a Peptidase S8 family domain.

SEQ ID NO:11 sets forth the amino acid sequence of the predominant form of protease BspM04033 observed (276 amino acids): MHPNQQWHYNMINAPQAWGTTTGSSS VIQAVLDTGIDHNHQSLANLVNTSLGQSFVGGSTMDVQGHGTHVAGTIASYGSVSGVMHNATLVPVKVLNDSGSGSLFGITQGILYSADIGADVINMSLGGGGYNQSMAEAAQTAVNAGSIVIAASGNDGAGSISYPAAYSSVIAVGSVTSTGARSNFSNYGSGLELMAPGSNIYSTVPNNGYATFSGTSMASPHAAGVAGLMRAVNPNLSVSNARSIMQNTAQYAGSPTFYGYGIVDANAAVQQASGGS.

### COMPARATIVE EXAMPLE 6

### Discovery and Identification of serine protease BspW01765

*Bacillus sp.* SWT211 (Dupont Culture Collection) was selected as a potential source for enzymes useful in industrial applications. To identify enzymes produced by *Bacillus sp.* SWT211 and the genes that encode these enzymes, the entire genome of *Bacillus sp.* SWT211 was sequenced using Illumina® sequencing by synthesis (SBS) technology. Genome sequencing, assembly and annotation of the sequence data was performed by BaseClear (Leiden, The Netherlands). One of genes identified this way in SWT211 encodes a protein that showed homology to serine proteases of various other bacteria. The sequence of this gene, *BspW01765.n,* is depicted in SEQ ID NO:12.
SEQ ID NO:12 sets forth the nucleotide sequence of the *BspW01765.n* gene: 
ATGAA
GAAGTTATTTACCTTGTTTTTATTGACACTTGTAATGCTTGTGGGTTATTTCTGTA
AATGTCATGGCAGATAATGAGGAAGAAAAAGAAGACCATAAGTACATTGAAGGTC
AATTAATCGTATCGGTAGAACCGGATGCAAATGATAACTCAATAGGACAAATGAAT
ATCACCTCAGATAAATTACAAAATAACTCCTCTCTAAAGAATAAAGGATTTAAAAT
AGCAGATTCTTTATTGGAAAACAATACTCCTGGTGTTCAAAGTATATTCAGCAGTAG
CTTTGTACAAGATGCTGCGAAAAGAACAGGGCTCGTTTACCTCATAGAATATTCCCC
AGAAAAATTTGAATCCATTCAGGCAGCAAAAAAAGACCTTGAAAAACCTTAACAG
AACTTGGATTTAATGTGAGATATGTTTCAGAAAACTTTGTTGTTGAGCTTTTAGAGA
CAGAAGCTACCTCAGATACTGGTGAAGATATCATCACGCCATTTATGCACAGTAAT
CAAGAATGGCATTACGGCATGATTAATGCCCCTGATGCTTGGGGTATTACTACAGGT
GACAGTAATGTAACAATAGCAGTATTGGATACTGGAATAGATTCTAGCCATTCAAG
TTTAAGTAACTTAGTAGATACTAGTCTTGGAAGAAGCTATGTTGGTGGTTCTCCAGA
GGATGTTCAAGGTCATGGAACGCACGTAGCAGGTACGATAGCAAGCTATGGTGCAG
TATCGGGTGTCATGCAGGATGCAACACTCATTTCTGTCAAAGTTTTAGGTGATGATG
GAAGTGGGTCAATGTATGGCATACAACAAGGAGTTTTATATGCTACAAGTATTGGT
GCAGACGTCATTAATATGTCTTTAGGCGGAGGCGGTTATAATCAAGGTTTCAATGAT
GCTATTGATACAGCAGTTGCGAATGGATCAGTTGTAATTGCTGCTTCTGGTAATGAT
 
GGTAGAGCTTCTATTTCCTATCCAGCAGCTTATGATGGAGCAATTGCAGTTGGGTCA
GTAACTTCTAGTGGTAATCGCTCAAACTTCTCTAACTATGGAAGTGGTCTTGAGTTA
ATGGCACCAGGATCAAGTATCTACAGCACCTATCCTAATGGTCAGTACAGAACGTT
ATCAGGTACATCTATGGCAGCTCCACATGCTGCAGGTGTTGCAGGACTAGTACGGG
CAGTAAATCCGAACTTGTCAGTAGCAGAAGTGAGAAACATATTAGCGGATACAGCA
CAATATGCAGGTAGTTCTCATCAGTATGGAAACGGTATTGTAGATGCTTTTGCAGCG
GTTCAAGCAGCAGGTGGATCTGGTGGAACACCATCACCTGGTGTTACGAATACAGT
TGTTTCAACAGATAAAAGTGTTTATGAGCGTGGTGAGCAAGTAACGATGACAACAA
CTGTTACAGATGAAGGCGGTAATGCTCTTCAAGACGCTACAGTTAATTACACAATTA
CACGTCCAAATGGATCTACTGTAACAAATACAACAACTACAAATTCAAATGGAATT
GCAACGTGGATAATTGGATCTAATTCACAAACTGCTTTAGGGACTTACGATGTGAC
GGCAGAAACTAGTCTATCAGGCTATCAAACTAGCTCTGATACTACTTCCTTTAGCTT
CTCTGATCAAGCACAGACCCAACAAACAGTAACGGATGTTTCAACGAATAGTAGCT
ATTATGCACGTGGTCAGAATGTAACCATATCAGCTGAAGTGAAGGATCAAGATGGA
GAGGCCCTATCAAATGCTACGGTTTCTTTTACAATTATCAGACCAAATGGAAGTACG
TTGACGAATACAGCTACAACTAATAGCGCAGGTGTGGCCACTTGGACTGTATCAAC
GAGTAGTGGAACTGCAAGAGGGACATATGAAGTAACTGCAGAGTCTTCTTACTCTA
CTTATGATGGAAGTTCAGATACCACAATCTTTTATGTTTAT.
   The preproenzyme encoded by the *BspW01765.n* gene is depicted in SEQ ID NO:13. At the N-terminus, the protein has a signal peptide with a length of 25 amino acids as predicted by SignalP-NN (Emanuelsson et al., Nature Protocols (2007) 2: 953-971). This signal peptide sequence is underlined and in bold in SEQ ID NO:13. The presence of a signal peptide indicates that this serine protease is a secreted enzyme. The enzyme has a pro sequence which is predicted (based on the pro-mature junction in *Bacillus bogoriensis* protease: see WO2012175708, SEQ ID NO:4) to be 142 amino acids (*in italics* in SEQ ID NO:13). The sequence of the predicted, processed mature chain (BspW01765, 488 amino acids) is depicted in SEQ ID NO:14. The mature chain of BspW01765 consists of a Peptidase S8 family domain at the N-terminus and a domain with an unknown function at the C-terminus. The sequence of the catalytic, peptidase domain of BspW01765 protease is predicted to be 270 amino acids and is depicted in SEQ ID NO:15.
SEQ ID NO:13 sets forth the predicted amino acid sequence of the serine protease precursor BspW01765: **MKKLFTLFLLTLVMLVGLFSVNVMA***DNEEEKEDHKYIEGQLIVSVEPDANDNSIGQMNITSDKLQNNSSLKNKGFKIADSLLENNTPGVQSIFSSSFVQDAAKRTGLVYLIEYSPEKFESIQAAKKDLEKTLTELGFNVRYVSENFVVELLETEATSDTGEDIITPF*MHSN QEWHYGMINAPDAWGITTGDSNVTIAVLDTGIDSSHSSLSNLVDTSLGRSYVGGSPEDVQGHGTHVAGTIASYGAVSGVMQDATLISVKVLGDDGSGSMYGIQQGVLYATSIGADVINMSLGGGGYNQGFNDAIDTAVANGSVVIAASGNDGRASISYPAAYDGAIAVGSVTSSGNRSNFSNYGSGLELMAPGSSIYSTYPNGQYRTLSGTSMAAPHAAGVAGLVRAVNPNLSVAEVRNILADTAQYAGSSHQYGNGIVDAFAAVQAAGGSGGTPSPGVTNTVVSTDKSVYERGEQVTMTTTVTDEGGNALQDATVNYTITRPNGSTVTNTTTTNSNGIATWIIGSNSQTALGTYDVTAETSLSGYQTSSDTTSFSFSDQAQTQQTVTDVSTNSSYYARGQNVTISAEVKDQDGEALSNATVSFTIIRPNGSTLTNTATTNSAGVATWTVSTSSGTARGTYEVTAESSYSTYDGSSDTTIFYVY.
SEQ ID NO:14 sets forth the predicted amino acid sequence of the mature protease BspW01765 (382 amino acids): MHSNQEWHYGMINAPDAWGITTGDSNVTIAVLDTGIDSSHSSLSNLVDTSLGRSYVGGSPEDVQGHGTHVAGTIASYGAVSGVMQDATLISVKVLGDDGSGSMYGIQQGVLYATSIGADVINMSLGGGGYNQGFNDAIDTAVANGSVVIAASGNDGRASISYPAAYDGAIAVGSVTSSGNRSNFSNYGSGLELMAPGSSIYSTYPNGQYRTLSGTSMAAPHAAGVAGLVRAVNPNLSVAEVRNILADTAQYAGSSHQYGNGIVDAFAAVQAAGGSGGTPSPGVTNTVVSTDKSVYERGEQVTMTTTVTDEGGNALQDATVNYTITRPNGSTVTNTTTTNSNGIATWIIGSNSQTALGTYDVTAETSLSGYQTSSDTTSFSFSDQAQTQQTVTDVSTNSSYYARGQNVTISAEVKDQDGEALSNATVSFTIIRPNGSTLTNTATTNSAGVATWTVSTSSGTARGTYEVTAESSYSTYDGSSDTTIFYVY.
SEQ ID NO:15 sets forth the predicted amino acid sequence of the Peptidase S8 family domain of BspW01765 protease (270 amino acids): MHSNQEWHYGMINAPDAWGITTGDSNVTIAVLDTGIDSSHSSLSNLVDTSLGRSYVGGSPEDVQGHGTHVAGTIASYGAVSGVMQDATLISVKVLGDDGSGSMYGIQQGVLYATSIGADVINMSLGGGGYNQGFNDAIDTAVANGSVVIAASGNDGRASISYPAAYDGAIAVGSVTSSGNRSNFSNYGSGLELMAPGSSIYSTYPNGQYRTLSGTSMAAPHAAGVAGLVRAVNPNLSVAEVRNILADTAQYAGSSHQYGNGIVDAFAAVQ.

### EXAMPLE 7

### Protease activity of BspAG00296 and BspM04033

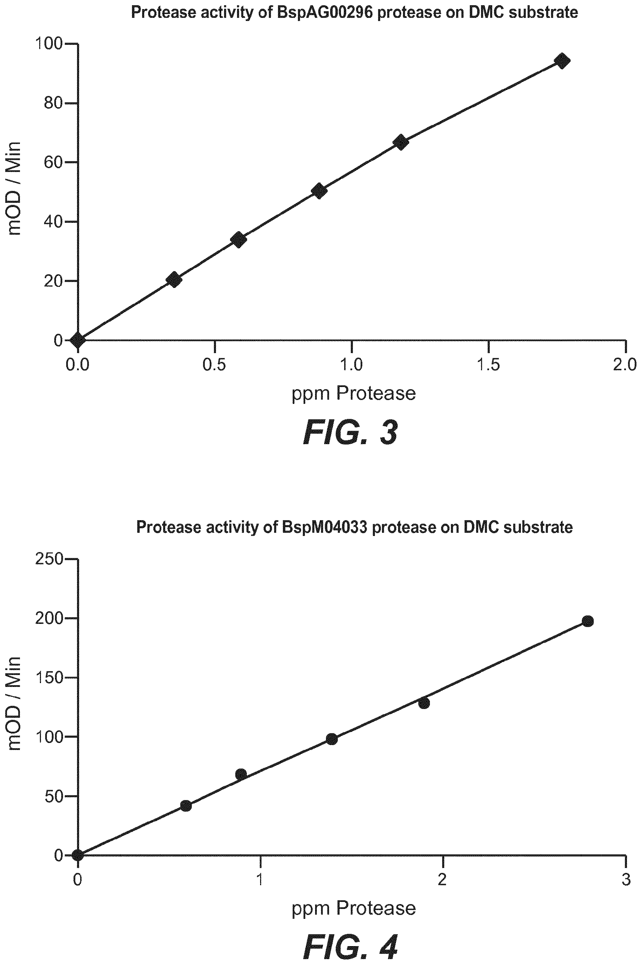

The protease activities of BspAG00296, BspM04033 proteases were tested by measuring the hydrolysis of dimethyl casein (DMC) substrate. The reagent solutions used for the DMC assay were: 2.5% Dimethylcasein (DMC, Sigma) in 100 mM Sodium Carbonate pH 9.5, 0.075% TNBSA (2,4,6-trinitrobenzene sulfonic acid, Thermo Scientific) in Reagent A. Reagent A: 45.4 g Na₂B₄O₇.10H₂0 (Merck) in 15 mL 4N NaOH to reach a final volume of 1000 mL in MQ water, Dilution Solution: 10 mM NaCl, 0.1 mM CaCl₂, 0.005% Tween-80. Protease supernatants were diluted in dilution solution to appropriate concentration for the assay. A 96-well microtiter plate (MTP) was filled with 95µl DMC substrate followed by the addition of 5µl diluted protease supernatant. 100µL of TNBSA in reagent A was then added with slow mixing. Activity was measured at 405 nm over 5 minutes using a SpectraMax plate reader in kinetic mode at RT. The absorbance of a blank containing no protease was subtracted from values. The activity was expressed as mOD/min. The protease activity curve for BspAG00296 is shown in Figure 3 and for BspM04033 is shown in Figure 4. Using the DMC assay, the specific activity of BspAG00296 protease was found to be 56 mOD/min/ppm, and of BspM04033 protease was found to be 71 mOD/min/ppm. The specific activities of GG36 and BPN' proteases were found to be 54 and 23 mOD/min/ppm, respectively under the same assay conditions.

### EXAMPLE 8

### pH profile of BspAG00296 and BspM04033 protease

The pH dependence of proteolytic activity of BspAG00296 and BspM04033 proteases was studied using azo-casein as substrate in a 50mM Acetate/Bis-Tris/HEPES/CHES buffer including 50 mM CaCl₂. The activity was measured at pH between 4 to 12 with 1 pH unit increments. One Protaxyme AK tablet (Megazyme, Ireland) was added to a glass test tube together with 1.9 mL of appropriate buffer and a magnet, followed by gentle hydration at 40°C for 5 min in a temperature controlled water bath fitted with magnetic stirrer. A 100 microliters sample of freshly prepared protease (diluted in deionised water to appropriate concentration for the assay) was added to the prehydrated substrate and reaction was carried out at 40°C for 10 min. To terminate the reaction, 10 mL of a 2 % w/v Tris buffer, pH 12 was added, solution was mixed, and the sample was immediately filtered through a Whatman No. 1 filter. The supernatant was collected, and the absorbance at 590 nm of the supernatant was measured to quantify the product of the reaction. The absorbance from a buffer-only control was subtracted, and the resulting values were converted to percentages of relative activity, by defining the activity at the optimal pH as 100%. BspAG00296 was determined to maintain ≥50% of activity over the pH range of 6-12, and BspM04033 was determined to maintain ≥50% of activity over the pH range of 7-12, under the conditions of this assay.

### EXAMPLE 9

### Temperature profile of BspAG00296 and BspM04033 protease

The temperature dependence of proteolytic activity of BspM04033 protease was studied using azo-casein as substrate in a 50mM Acetate/Bis-Tris/HEPES/CHES buffer including 50 mM CaCl₂ at pH 9. The activity was measured at temperatures between 30°C and 80°C with 10°C increments. One Protaxyme AK tablet (Megazyme, Ireland) was added to a glass test tube together with 1.9 mL of appropriate buffer and a magnet, followed by gentle hydration at set temperatures for 5 min in a temperature controlled water bath fitted with magnetic stirrer. A 100µl sample of freshly prepared protease (diluted in deionised water to appropriate concentration for the assay) was added to the prehydrated substrate and reaction was carried out at temperatures between 30°C and 80°C for 10 min. To terminate the reaction, 10 mL of a 2 % w/v Tris buffer pH 12 was added and solution was mixed and filtered immediately through a Whatman No. 1 filter. The supernatant was collected and the absorbance at 590 nm of the supernatant was measured to quantify the product of the reaction. The absorbance from a buffer-only control was subtracted from each sample reading, and the resulting values were converted to percentages of relative activity, by defining the activity at the optimal temperature at 100%. BspAG00296 was determined to retain ≥50% activity over a range of 55-75°C and BspM04033 was determined to retain ≥50% activity over a range of 55-80°C, under the conditions of this assay.

### EXAMPLE 10

### Cleaning performance of BspAG00296 and BspM04033

The cleaning performance of BspAG00296 and BspM04033 proteases was tested on BMI (blood/milk/ink on cotton) microswatches (EMPA-116, Center for Testmaterials, The Netherlands) for laundry based applications, and on egg yolk (egg yolk on polyacryl fabric, aged and colored with carbon black dye) microswatches (PAS-38, Center for Testmaterials, The Netherlands) for dish based applications. MTPs (Corning 3641) containing pre-punched (to fit on MTP) and pre-rinsed swatches, were filled with detergent prior to enzyme addition. Commercial detergents were heat-inactivated to remove enzyme and dosed as described in Table 1.

Heavy duty liquid (HDL) laundry detergents were inactivated by heating to 95°C for 4 hours in a water bath. Heavy duty dry (HDD) laundry detergents were inactivated by preparing a 10 % w/v solution and heating for 4 hours at 95°C. After heating the HDD and HDL detergents for 4 hours, protease activity was non-existent. Following inactivation treatment, protease activity was assayed using N-suc-AAPF-pNA substrate. The reagent solutions used for the AAPF hydrolysis assay were: 100 mM Tris/HCl pH 8.6, containing 0.005% TWEEN®-80 (Tris dilution buffer); 100 mM Tris buffer pH 8.6, containing 10 mM CaCl₂ and 0.005% TWEEN®-80 (Tris/Ca buffer); and 160 mM suc-AAPF-pNA in DMSO (suc-AAPF-pNA stock solution) (Sigma: S-7388). To prepare a substrate working solution, 1 ml suc-AAPF-pNA stock solution was added to 100 ml Tris/Ca buffer and mixed well. An enzyme sample was added to a MTP plate (Costar 9017) containing 1 mg/suc-AAPF-pNA working solution and assayed for activity at 405 nm over 3 minutes using a SpectraMax plate reader in kinetic mode at RT. The protease activity was expressed as mOD·min¹.

Washing solutions with the Final Detergent Wash concentrations (g/L) described in Table 1 were made up and used in the cleaning performance assay.

**Table 1: List of detergent conditions used for performance assays**

| **Detergent*** | **Type** | **Final Detergent Wash Conc, (g/L)** | **Hardness Conc. (ppm)** | **Buffer** | **Set pH** |
|---|---|---|---|---|---|
| OMO color | HDD | 5.3 | 250 | 2 mM NaCO₃ | 10.6 |
| Kirkland Ultra | HDD | 1.09 | 150 | 2 mM NaCO₃ | 10.6 |
| OMO Klein & Krachtig | HDL | 2.8 | 250 | 5 mM sodium HEPES | 8.2 |
| Kirkland Ultra | HDL | 0.71 | 150 | 5 mM sodium HEPES | 8.2 |
| GSM-B 10.5 | ADW | 3 | 374 | Unbuffered | as is ∼10.5 |
| GSM-B 9 | ADW | 3 | 374 | Unbuffered,1M citrate added to adjust pH | 9 |

| | | | | | |
|---|---|---|---|---|---|
| *Detergent sources: Kirkland Ultra HDD and HDL (Sun Products) were purchased from local supermarket in US in 2012. OMO color HDD and OMO Klein & Krachtig (Unilever) were purchased from local supermarkets in The Netherlands in 2013. GSM-B was purchased from WFK Testgewebe GmbH, Germany, www.testgewebe.de, composition is given in Table 2. | | | | | |

**Table 2: Composition of GSM-B pH10.5 ADW detergent**

| **GSM-B Phosphate-Free Detergent** | |
|---|---|
| **Component** | **Wt %** |
| Sodium citrate dehydrate | 30.0 |
| Maleic acid/ acrylic acid copolymer sodium Salt (SOKALAN® CP5; BASF) | 12.0 |
| Sodium perborate monohydrate | 5.0 |
| TAED | 2.0 |
| Sodium disilicate: Protil A (Cognis) | 25.0 |
| Linear fatty alcohol ethoxylate | 2.0 |
| Sodium carbonate anhydrous | add to 100 |

For cleaning assays, 10 uL of protease diluted in dilution buffer: 10 mM NaCl, 0.1 mM CaCl₂, 0.005% Tween-80 was added to a detergent-filled microswatch plate to reach a final volume of 200 uL, with 0.04 to 10 ppm final enzyme concentration. Laundry cleaning assays with HDL or HDD formulas were carried out at 25°C for 15 minutes, while automatic dish (ADW) assays were carried out at 40°C for 30 minutes.

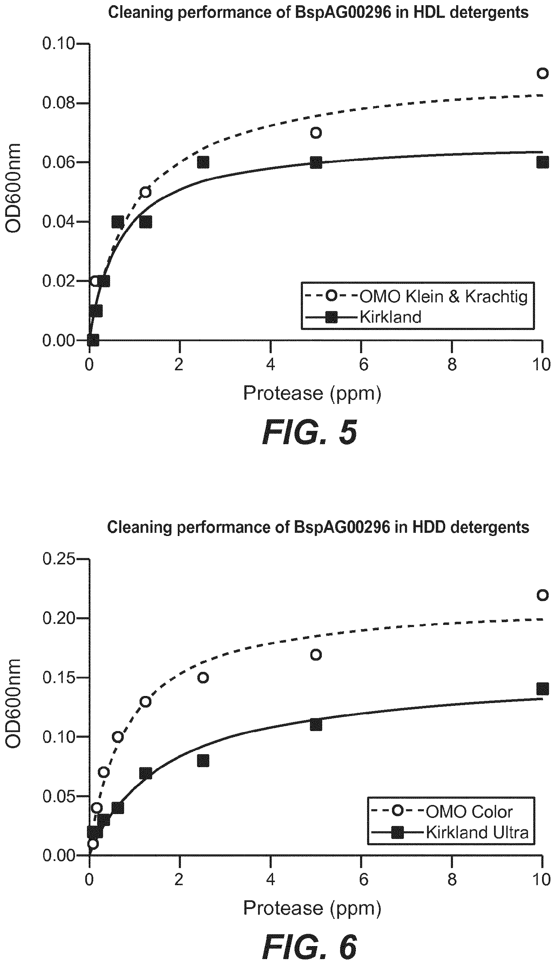

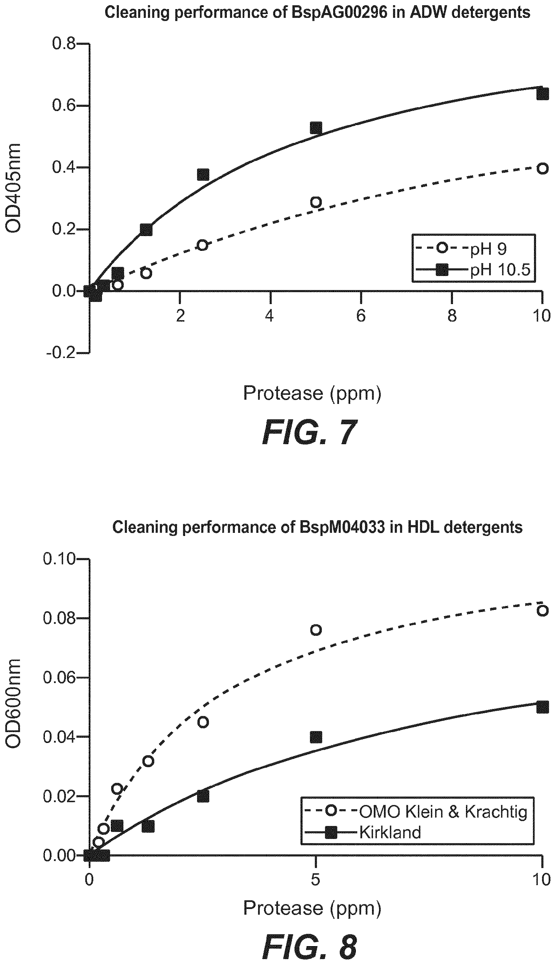

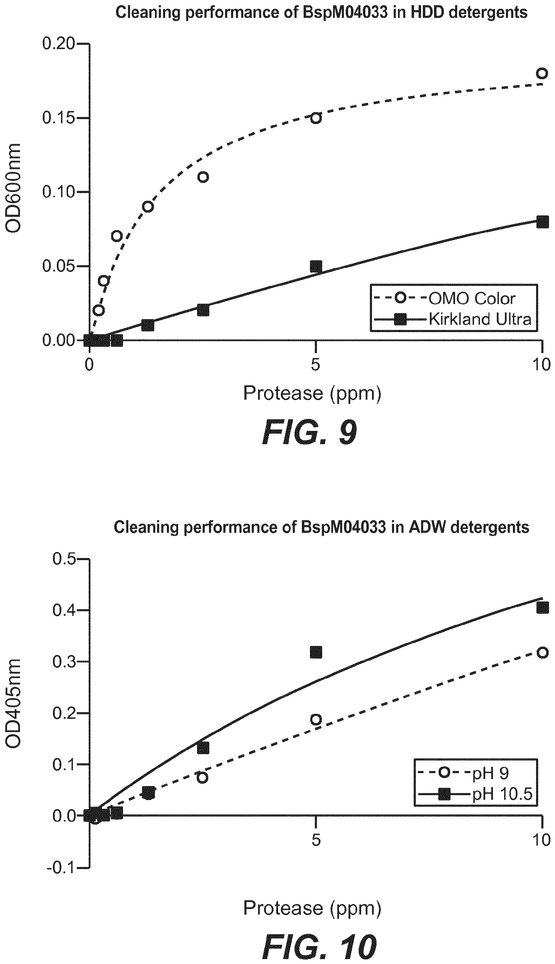

Following incubation, 100 uL of supernatant was transferred to a fresh MTP (Kisker G080-F) and absorbance was read at 600 nm for EMPA-116 swatches, or at 405 nm for PAS-38 swatches, using the SpectraMax plate reader. The absorbance from a buffer-only control was subtracted and the resulting OD values at 600nm (for HDL and HDD detergents) and 405nm (for ADW detergents) were plotted as a function of protease concentration. The data was fitted to Langmuir equation. The cleaning performance of BspAG00296 and BspM04033 is shown graphically in Figures 5-7 and Figures 8-10, respectively.

### EXAMPLE 11

### Stability evaluation of proteases

The stability of BspAG00296, BspM04033, *B. sp.* NN018132 (WO2012175708-002) Full length sequence (SEQ ID NO:16) and truncated form (SEQ ID NO:17), GG36 (SEQ ID NO:18), and FNA (SEQ ID NO:19) proteases was determined under various conditions.
SEQ ID NO:16 sets forth the sequence of full length *B. sp.* NN018132 protease: 
LMHNNQRWHYEMINAPQAWGITTGSSNVRIAVLDTGIDANHPNLRNLVDTSLGRSFVG
GGTGDVQGHGTHVAGTIASYGSVSGVMQNARLIPVKVLGDNGSGSMYGIQQGILYAAS
INADVINMSLGGGGYDSGMNNAINTAVSSGTLVIAASGNDGRGSISYPAAYSNAIAVGS
VTSNRTRSNFSNYGSGLELMAPGSNIYSTYPNGQFRTLSGTSMATPHVAGVAGLIKSAN
PNLSVTQVRNILRDTAQYAGSSNQYGYGIVNAYAAVQAAGGAVSYETNTSVSTNQST
YYRGNNVTMTAIVTDQNNSRLQGATVNFTITRPNGTTVTNATTTNSSGVATWTIGSNSS
TAVGTYQVRAQTTYPNYQSSSATTSFRLQ.
SEQ ID NO:17 sets forth the sequence of the truncated *B. sp.* NN018132 protease: 
MHNNQRWHYEMINAPQAWGITTGSSNVRIAVLDTGIDANHPNLRNLVDTSLGRSFV
GGGTGDVQGHGTHVAGTIASYGSVSGVMQNARLIPVKVLGDNGSGSMYGIQQGILYA
ASINADVINMSLGGGGYDSGMNNAINTAVSSGTLVIAASGNDGRGSISYPAAYSNAIAV
GSVTSNRTRSNFSNYGSGLELMAPGSNIYSTYPNGQFRTLSGTSMATPHVAGVAGLIKS
ANPNLSVTQVRNILRDTAQYAGSSNQYGYGIVNAYAAVQAAGG.
SEQ ID NO:18 sets forth the sequence of GG36 protease: AQSVPWGISRVQAPAAHNRGLTGSGVKVAVLDTGISTHPDLNIRGGASFVPGEPSTQDGNGHGTHVAGTIAALNNSIGVLGVAPSAELYAVKVLGASGSGSVSSIAQGLEWAGNNGMHVANLSLGSPSPSATLEQAVNSATSRGVLVVAASGNSGAGSISYPARYANAMAVGATDQNNNRASFSQYGAGLDIVAPGVNVQSTYPGSTYASLNGTSMATPHVAGAAALVKQKNPSWSNVQIRNHLKNTATSLGSTNLYGSGLVNAEAATR.
SEQ ID NO:19 sets forth the sequence of FNA protease (BPN'Y217L): AQSVPYGVSQIKAPALHSQGYTGSNVKVAVIDSGIDSSHPDLKVAGGASMVPSETNPFQDNNSHGT HVAGTVAALNNSIGVLGVAPSASLYAVKVLGADGSGQYSWIINGIEWAIANNMDVINMSLGGPSGSAALKAAVDKAVASGVVVVAAAGNEGTSGSSSTVGYPGKYPSVIAVGAVDSSNQRASFSSVGPELDVMAPGVSIQSTLPGNKYGALNGTSMASPHVAGAAALILSKHPNWTNTQVRSSLENTTTKLGDSFYYGKGLINVQAAAQ.

Stability was tested under three stress conditions shown below by measuring the residual proteolytic activity following incubation at set temperatures.
1. LAS/EDTA: 0.02% LAS, 2.1mM EDTA in 50mM HEPES pH8, 0.005% Tween 80
2. Tris/EDTA: 50mM Tris, 1 mM EDTA, pH 9, 0.005% Tween 80
3. OMO HDL: 10% OMO Klein & Krachtig (protease inactivated prior to use)

For stressed conditions, diluted enzyme sample was mixed in stress buffers/detergent in a 96-well PCR plate and incubated at 30°C, 40°C, 50°C, 60°C and 75°C for 20 minutes using a Tetrad2 Thermocycler. For the unstressed condition, enzyme was assayed immediately after mixing with stress media to establish a baseline (initial activity). Protease activity under stressed and unstressed conditions was measured by either the hydrolysis of AAPF-pNA (for OMO HDL) or DMC (for LAS/EDTA and Tris/EDTA) substrate assays described previously. Percent residual activities were calculated by taking a ratio of the stressed to unstressed activity at each temperature and multiplying it by 100. The percent remaining activity for each protease is shown on Tables 3-5 for each condition run at the various temperatures.

| **Table3: Stability of proteases in LAS/EDTA** | | | | | | |
|---|---|---|---|---|---|---|
| | | **Incubation temperature** | | | | |
| **Enzyme** | **Unstressed** | **30°C** | **40°C** | **50°C** | **60°C** | **75°C** |
| BspAG00296 | 100 | >90 | 54 | 11 | 11 | 9 |
| BspM04033 | 100 | >90 | >90 | >90 | 66 | 23 |
| *B. sp.* NN018132 | 100 | 64 | 10 | 8 | 8 | 9 |
| GG36 | 100 | 78 | 16 | 1 | 1 | 1 |
| FNA | 100 | >90 | 84 | 7 | 4 | 1 |

| **Table 4: Stability of proteases in Tris/EDTA** | | | | | | |
|---|---|---|---|---|---|---|
| | | **Incubation temperature** | | | | |
| **Enzyme** | **Unstressed** | **30°C** | **40°C** | **50°C** | **60°C** | **75°C** |
| BspAG00296 | 100 | >90 | >90 | >90 | 81 | 17 |
| BspM04033 | 100 | >90 | >90 | >90 | 42 | 26 |
| *B. sp.* NN018132 | 100 | >90 | >90 | >90 | 20 | 21 |
| GG36 | 100 | >90 | 87 | 8 | 6 | 6 |
| FNA | 100 | >90 | >90 | 24 | 19 | 20 |

| **Table 5: Stability of proteases in OMO HDL** | | | | | | |
|---|---|---|---|---|---|---|
| | | **Incubation temperature** | | | | |
| **Enzyme** | **Unstressed** | **30°C** | **40°C** | **50°C** | **60°C** | **75°C** |
| BspAG00296 | 100 | 89 | 85 | 10 | 2 | 1 |
| BspM04033 | 100 | >90 | >90 | >90 | 15 | 0 |
| *B. sp.* NN018132 | 100 | 75 | 45 | 0 | 0 | 0 |
| GG36 | 100 | 85 | 81 | 35 | 3 | 2 |
| FNA | 100 | >90 | >90 | 71 | 1 | 1 |

### COMPARATIVE EXAMPLE 12

### Identification of Additional B. sp. Serine Proteases

Additional subtilisins were identified by sequencing the genomes of other *B. spp. B. sp.* SWT81 (BspAA02831), *B. sp.* SWT4 (SWT4_1110112), *B. sp.* SWT22 (SWT22_1181566), *B. sp.* SWT32 (SWT32_1214607), *B. sp.* SWT40 (SWT40_1237842), *B. sp.* SWT41 (SWT41_1431481), *B. sp.* SWT77 (SWT77_1339394), and *B. sp.* SWT123 (SWT123_1418561) obtained from the DuPont Culture Collection. Genome sequencing, assembly and annotation were essentially as described in Example 2. All genomes encoded proteins homologous to BspAG00296 and BspM04033.

The amino acid sequence of the preproenzyme form of BspAA02831 is depicted in SEQ ID NO:20. The predicted signal peptide sequence is underlined and the pro sequence is in italics. The sequence of the predicted, fully processed mature chain (BspAA02831, 381 amino acids) is in bold.

SEQ ID NO:20 sets forth the amino acid sequence of the preproenzyme form of BspAA02831: MKKWLGMSAVVVLMVLSLFTGSGFA*NESKGKNNGDYIEGQLVISIEDQSEFSIQSTNNIINKDQVLENKGFEIVDSLLGQSDPNEIQAFNHDFTATVVNEMGMVYLVEYDVKKYKSIDKAKKELEKTMKDLGLEVRYVSENFVMHAMEEVTAEDVSIA***MHNNQRWHYEMINAPQAWNITTGSRNVRIAVLDTGIDANHPNLRNLVNTSLGRSFVGGGTGDVQGHGTHVAGTIASYGSVSGVMQNATLIPVKVLGDNGSGSMYGIQQGILYAASVNSDVINMSLGGGGYSQGMDDAIRTAVSSGTIVVAATGNDSRGSISYPAAYSGAIAVGSVTSNRTRSSFSNYGQGLELMAPGSNIYSTYPNGQFRTLSGTSMATPHVAGVAGLIRAANPNISVSEARSILQNTAQYAGSFNQYGYGIVDANAAVRAARGQSQQPSYETNTTVSTNASSYRRGQSVTVRADVVDQDGRALANSTVQFTITRPNGTTVTNTATTNNSGVATWTIATSSSTARGTYGVQAATSLSGYEGSTATTSFSVN.**

The amino acid sequence of the proenzyme form of BspAA02831 is depicted in SEQ ID NO:21. The pro sequence is in italics. The sequence of the predicted, fully processed mature chain (BspAA02831, 381 amino acids) is in bold.

SEQ ID NO:21 sets forth the amino acid sequence of the proenzyme form of BspAA02831:

NESKGKNNGDYIEGQLVISIEDQSEFSIQSTNNIINKDQVLENKGFEIVDSLLGQ

SDPNEIQAFNHDFTATVVNEMGMVYLVEYDVKKYKSIDKAKKELEKTMKDLGLEVRYVSENF
VMHAMEEVTAEDVSIAMHNNQRWHYEMINAPQAWNITTGSRNVRIAVLDTGIDANH
PNLRNLVNTSLGRSFVGGGTGDVQGHGTHVAGTIASYGSVSGVMQNATLIPVKVL
GDNGSGSMYGIQQGILYAASVNSDVINMSLGGGGYSQGMDDAIRTAVSSGTIVVAA
TGNDSRGSISYPAAYSGAIAVGSVTSNRTRSSFSNYGQGLELMAPGSNIYSTYPNGQ
FRTLSGTSMATPHVAGVAGLIRAANPNISVSEARSILQNTAQYAGSFNQYGYGIVDA
NAAVRAARGQSQQPSYETNTTVSTNASSYRRGQSVTVRADVVDQDGRALANSTVQ
FTITRPNGTTVTNTATTNNSGVATWTIATSSSTARGTYGVQAATSLSGYEGSTATTS
FSVN.

The sequence of the predicted, fully processed mature chain (BspAA02831, 381 amino acids) is depicted in SEQ ID NO:22:

MHNNQRWHYEMINAPQAWNITTGSRNVRIAV
LDTGIDANHPNLRNLVNTSLGRSFVGGGTGDVQGHGTHVAGTIASYGSVSGVMQNATL
IPVKVLGDNGSGSMYGIQQGILYAASVNSDVINMSLGGGGYSQGMDDAIRTAVSSGTIV
VAATGNDSRGSISYPAAYSGAIAVGSVTSNRTRSSFSNYGQGLELMAPGSNIYSTYPNG
QFRTLSGTSMATPHVAGVAGLIRAANPNISVSEARSILQNTAQYAGSFNQYGYGIVDAN
AAVRAARGQSQQPSYETNTTVSTNASSYRRGQSVTVRADVVDQDGRALANSTVQFTIT
RPNGTTVTNTATTNNSGVATWTIATSSSTARGTYGVQAATSLSGYEGSTATTSFSVN.

The amino acid sequence of the preproenzyme form of SWT4 is depicted in SEQ ID NO:23. The predicted signal peptide sequence is underlined and the pro sequence is in italics. The sequence of the predicted, fully processed mature chain (SWT4, 381 amino acids) is in bold.

SEQ ID NO:23 sets forth the amino acid sequence of the preproenzyme form of SWT4: VKKSAVWNLMTVLVFSLFLNPAGIGAQA*SDAASEKDDTAYIEGQLIVSVKSSDVSVKGIEGVNKKIMGDVLRERGFAITDSIMGLGDPGEVNAFTNQEFSESWKNMGLVYLAEYDVSVYASVEEAKRALAEALKENGMEIRHISKNYEMHAIGELADVSPQ***MHPNQQWHYNMINAPQAWGTTTGSSSVIQAVLDTGIDHNHQSLANLVNTSLGQSFVGGSTMDVQGHGTHVAGTIASYGSVSGVMHNATLVPVKVLNDSGSGSLFGITQGILYSADIGADVINMSLGGGGYNQSMAEAAQTAVNAGSIVIAASGNDGAGSVSYPAAYSSVIAVGSVTSTGARSNFSNYGSGLELMAPGSNIYSTVPNNGYATFSGTSMASPHAAGVAGLMRAVNPNLSVSNARSIMQNTAQYAGSPTFYGYGIVDANAAVQQASGGSGDPSNITETSISTDRFYVQRGQNVTSTAQVTNENGQGLANATVTFTITRPNGSTLTNTATTNSSGFASWTVGTSGATATGTYSVEASSSLQGYQGSSASTSFFVY.**

The amino acid sequence of the proenzyme form of SWT4 is depicted in SEQ ID NO:24. The pro sequence is in italics. The sequence of the predicted, fully processed mature chain (SWT4, 381 amino acids) is in bold.

SEQ ID NO:24 sets forth the amino acid sequence of the proenzyme form of SWT4:

SDAASEKDDTAYIEGQLIVSVKSSDVSVKGIEGVNKKIMGDVLRERGFAITDSIMGLGDPGEV
N
AFTNQEFSESVVKNMGLVYLAEYDVSVYASVEEAKRALAEALKENGMEIRHISKNYEMHAIGE
LADVSPQMHPNQQWHYNMINAPQAWGTTTGSSSVIQAVLDTGIDHNHQSLANLVN
TSLGQSFVGGSTMDVQGHGTHVAGTIASYGSVSGVMHNATLVPVKVLNDSGSGSL
FGITQGILYSADIGADVINMSLGGGGYNQSMAEAAQTAVNAGSIVIAASGNDGAGS
VSYPAAYSSVIAVGSVTSTGARSNFSNYGSGLELMAPGSNIYSTVPNNGYATFSGTS
MASPHAAGVAGLMRAVNPNLSVSNARSIMQNTAQYAGSPTFYGYGIVDANAAVQQ
ASGGSGDPSNITETSISTDRFYVQRGQNVTSTAQVTNENGQGLANATVTFTITRPNG
STLTNTATTNSSGFASWTVGTSGATATGTYSVEASSSLQGYQGSSASTSFFVY.

The sequence of the predicted, fully processed mature chain (SWT4, 381 amino acids) is depicted in SEQ ID NO:25: MHPNQQWHYNMINAPQAWGTTTGSSSVIQAVLDTGIDHNHQSLANLVNTSLGQSFVGGSTMDVQGHGTHVAGTIASYGSVSGVMHNATLVPVKVLNDSGSGSLFGITQGILYSADIGADVINMSLGGGGYNQSMAEAAQTAVNAGSIVIAASGNDGAGSVSYPAAYSSVIAVGSVTSTGARSNFSNYGSGLELMAPGSNIYSTVPNNGYATFSGTSMASPHAAGVAGLMRAVNPNLSVSNARSIMQNTAQYAGSPTFYGYGIVDANAAVQQASGGSGDPSNITETSISTDRFYVQRGQNVTSTAQVTNENGQGLANATVTFTITRPNGSTLTNTATTNSSGFASWTVGTSGATATGTYSVEASSSLQGYQGSSASTSFFVY.

The amino acid sequence of the preproenzyme form of SWT22 is depicted in SEQ ID NO:26. The predicted signal peptide sequence is underlined and the pro sequence is in italics. The sequence of the predicted, fully processed mature chain (SWT22, 488 amino acids) is in bold.

SEQ ID NO:26 sets forth the amino acid sequence of the preproenzyme form of SWT22:

MKKLLTLSILTLAMLVGFFSVNVFADNEVQKKEDHKYIDGQLIVSVEMDGKENSL
KGQLNSTTELLQDNAELKKKGFAVSDSLLEEKTADSQSVFSDSFVEKAAKKTGFVYLMEYST
DEYDSIKTAMKELEKTLNELGLKVRYVSENFVVELLETDAVAEADENKIAPLMHRNQEWHY
GMINAPDAWGITTGSSNVRMAVLDTGIDSSHPSLRNLVDTSLGRSYVGGNPEDRQG
HGTHVAGTIASYGNVSGVMQNASLISVKVLGDDGSGSTYGIQQGVLYAASINSDVI
NMSLGGGGYSQGFSDAIDTAVANGTVVIAASGNDGRASISYPAAYDGAIAVGSVTSS

GSRSNFSNYGNGLELMAPGSSIYSTYPNGQYRTLSGTSMAAPHAAGVAGLVRAVDP
SLSVSQVRGILADTAQYAGSSHQYGNGIVDAYAAVQAAGGSGGAPAPSETNTSVST
NGSVFERGDDVTMTASVTDDNGNGLQGAAVNFTITRPNGSTVTNTATTNSSGNAT
WTIGSNSQTALGTYEVTAETTLSGYESSSDTTSFSFSNQAQTHQTVTDVSTNSNYYA
RGQNVTVSAEVRDQDGAVLSNATVSFTITRPNGSTVTNTGATNSAGVATWTVSTSG
ATATGTYQVTAETTLTNYDGSSDSTSFYVY.

The amino acid sequence of the proenzyme form of SWT22 is depicted in SEQ ID NO:27. The pro sequence is in italics. The sequence of the predicted, fully processed mature chain (SWT22, 488 amino acids) is in bold.

SEQ ID NO:27 sets forth the amino acid sequence of the proenzyme form of SWT22:

DNEVQKKEDHKYIDGQLIVSVEMDGKENSLKGQLNSTTELLQDNAELKKKGFAVSDSLLEEK
TADSQSVFSDSFVEKAAKKTGFVYLMEYSTDEYDSIKTAMKELEKTLNELGLKVRYVSENFVV
ELLETDAVAEADENKIAPLMHRNQEWHYGMINAPDAWGITTGSSNVRMAVLDTGIDS
SHPSLRNLVDTSLGRSYVGGNPEDRQGHGTHVAGTIASYGNVSGVMQNASLISVKV
LGDDGSGSTYGIQQGVLYAASINSDVINMSLGGGGYSQGFSDAIDTAVANGTVVIA
ASGNDGRASISYPAAYDGAIAVGSVTSSGSRSNFSNYGNGLELMAPGSSIYSTYPNG
QYRTLSGTSMAAPHAAGVAGLVRAVDPSLSVSQVRGILADTAQYAGSSHQYGNGI
VDAYAAVQAAGGSGGAPAPSETNTSVSTNGSVFERGDDVTMTASVTDDNGNGLQG
AAVNFTITRPNGSTVTNTATTNSSGNATWTIGSNSQTALGTYEVTAETTLSGYESSS
DTTSFSFSNQAQTHQTVTDVSTNSNYYARGQNVTVSAEVRDQDGAVLSNATVSFTI
TRPNGSTVTNTGATNSAGVATWTVSTSGATATGTYQVTAETTLTNYDGSSDSTSFY
VY.

The sequence of the predicted, fully processed mature chain (SWT22, 488 amino acids) is depicted in SEQ ID NO:28: MHRNQEWHYGMINAPDAWGITTGSSNVRMAVLDTGIDSSHPSLRNLVDTSLGRSYVGGNPEDRQGHGTHVAGTIASYGNVSGVMQNASLISVKVLGDDGSGSTYGIQQGVLYAASINSDVINMSLGGGGYSQGFSDAIDTAVANGTVVIAASGNDGRASISYPAAYDGAIAVGSVTSSGSRSNFSNYGNGLELMAPGSSIYSTYPNGQYRTLSGTSMAAPHAAGVAGLVRAVDPSLSVSQVRGILADTAQYAGSSHQYGNGIVDAYAAVQAAGGSGGAPAPSETNTSVSTNGSVFERGDDVTMTASVTDDNGNGLQGAAVNFTITRPNGSTVTNTATTNSSGNATWTIGSNSQTALGTYEVTAETTLSGYESSSDTTSFSFSNQAQTHQTVTDVSTNSNYYARGQNVTVSAEVRDQDGAVLSNATVSFTITRPNGSTVTNTGATNSAGVATWTVSTSGATATGTYQVTAETTLTNYDGSSDSTSFYVY.

The amino acid sequence of the preproenzyme form of SWT32 is depicted in SEQ ID NO:29. The predicted signal peptide sequence is underlined and the pro sequence is in italics. The sequence of the predicted, fully processed mature chain (SWT32, 381 amino acids) is in bold.

SEQ ID NO:29 sets forth the amino acid sequence of the preproenzyme form of SWT32:

VKKSAVWVLMTVLVFSLFLNPAGIGAQASDAASEKDDTAYIEGQLIVSVKSSDVSV
KGIEGLNKKIMGNVLRERGFAITDSIMGLGDPAEVNAFTNQEFSESVVKNMGLVYLAEYDVS
VYASVEEAKRALAEALKENGMEIRHISKNYEMHAIGEPADVSPQMHPNQQWHYNMINAP
QAWGTTTGSSSVIQAVLDTGIDHNHQSLANLVNTSLGQSFVGGSTMDVQGHGTHV
AGTIASYGSVSGVMHNATLVPVKVLNDSGSGSLFGITQGILYSADIGADVINMSLGG
GGYNQSMAEAAQTAVNAGSIVIAASGNDGAGSISYPAAYSSVIAVGSVTSTGARSNF
SNYGSGLELMAPGSNIYSTVPNNGYATFSGTSMASPHAAGVAGLMRAVNPNLSVSD
ARSIMQNTAQYAGSPTFYGYGIVDANAAVQQASGGSGGPSNITETSISTDRFYVQR
GQNVTSTAQVTNENGQGLANATVTFTITRPNGSTLTNTATTNGSGFASWTVGTSG
ATATGTYSVEASSSLQGYQGSSASTSFFVY.

The amino acid sequence of the proenzyme form of SWT32 is depicted in SEQ ID NO:30. The pro sequence is in italics. The sequence of the predicted, fully processed mature chain (SWT32, 381 amino acids) is in bold.

SEQ ID NO:30 sets forth the amino acid sequence of the proenzyme form of SWT32:

SDAASEKDDTAYIEGQLIVSVKSSDVSVKGIEGLNKKIMGNVLRERGFAITDSIMGLGDPAEVN
AFTNQEFSESVVKNMGLVYLAEYDVSVYASVEEAKRALAEALKENGMEIRHISKNYEMHAIGE
PADVSPQMHPNQQWHYNMINAPQAWGTTTGSSSVIQAVLDTGIDHNHQSLANLVN
TSLGQSFVGGSTMDVQGHGTHVAGTIASYGSVSGVMHNATLVPVKVLNDSGSGSL
FGITQGILYSADIGADVINMSLGGGGYNQSMAEAAQTAVNAGSIVIAASGNDGAGSI
SYPAAYSSVIAVGSVTSTGARSNFSNYGSGLELMAPGSNIYSTVPNNGYATFSGTSM
ASPHAAGVAGLMRAVNPNLSVSDARSIMQNTAQYAGSPTFYGYGIVDANAAVQQA
SGGSGGPSNITETSISTDRFYVQRGQNVTSTAQVTNENGQGLANATVTFTITRPNGS
TLTNTATTNGSGFASWTVGTSGATATGTYSVEASSSLQGYQGSSASTSFFVY.

The sequence of the predicted, fully processed mature chain (SWT32, 381 amino acids) is depicted in SEQ ID NO:31: MHPNQQWHYNMINAPQAWGTTTGSSSVIQAVLDTGIDHNHQSLANLVNTSLGQSFVGGSTMDVQGHGTHVAGTIASYGSVSGVMHNATLVPVKVLNDSGSGSLFGITQGILYSADIGADVINMSLGGGGYNQSMAEAAQTAVNAGSIVIAA SGNDGAGSISYPAAYSSVIAVGSVTSTGARSNFSNYGSGLELMAPGSNIYSTVPNNGYATFSGTSMASPHAAGVAGLMRAVNPNLSVSDARSIMQNTAQYAGSPTFYGYGIVDANAAVQQASGGSGGPSNITETSISTDRFYVQRGQNVTSTAQVTNENGQGLANATVTFTITRPNGSTLTNTATTNGSGFASWTVGTSGATATGTYSVEASSSLQGYQGSSASTSFFVY.

The amino acid sequence of the preproenzyme form of SWT40 is depicted in SEQ ID NO:32. The predicted signal peptide sequence is underlined and the pro sequence is in italics. The sequence of the predicted, fully processed mature chain (SWT40, 381 amino acids) is in bold.

SEQ ID NO:32 sets forth the amino acid sequence of the preproenzyme form of SWT40: MKKWLGMSAVVVLMVFSMFTGAGFA*NESKGKNNGDYIEGQLVISIEDQSQFSIQATNNIINKDEVLENNGFEIVDSLLGQNDPNEIQAYNHDFTATVVNEMGLVYLVEYDVKKYKSIDKAKKELEKTMKDLGLEVRYVSENFVMHAMEEVTAEEVSIA***MHNNQRWHYEMINAPQAWNVTTGSRNVRIAVLDTGIDANHPNLRNLVNTSLGRSFVGGGTGDVQGHGTHVAGTIASYGSVSGVMQNATLIPVKVLGDNGSGSMYGIQQGILYAASVNSDVINMSLGGGGYSQGMDDAIRTAVSSGTIVVAATGNDSRGSISYPAAYSGAIAVGSVTSNRTRSSFSNYGQGLELMAPGSNIYSTYPNGQFRTLSGTSMATPHVAGVAGLIRAANPNISVAEARSILQNTAQYAGSFNQYGYGIVDANAAVRAARGQTEQPRYETNTTVSTNASTYRRGQSVTVRADVVDQDGRALANSTVQFTITRPNGTTVTNTATTNSSGVATWTIGTSSSTARGTYGVQAATSLSGYEGSTATTSFVVN.**

The amino acid sequence of the proenzyme form of SWT40 is depicted in SEQ ID NO:33. The pro sequence is in italics. The sequence of the predicted, fully processed mature chain (SWT40, 381 amino acids) is in bold.

SEQ ID NO:33 sets forth the amino acid sequence of the proenzyme form of SWT40:

NESKGKNNGDYIEGQLVISIEDQSQFSIQATNNIINKDEVLENNGFEIVDSLLGQNDPNEIQAY
NHDFTATVVNEMGLVYLVEYDVKKYKSIDKAKKELEKTMKDLGLEVRYVSENFVMHAMEEV
TAEEVSIAMHNNQRWHYEMINAPQAWNVTTGSRNVRIAVLDTGIDANHPNLRNLVN
TSLGRSFVGGGTGDVQGHGTHVAGTIASYGSVSGVMQNATLIPVKVLGDNGSGSM
YGIQQGILYAASVNSDVINMSLGGGGYSQGMDDAIRTAVSSGTIVVAATGNDSRGSI
SYPAAYSGAIAVGSVTSNRTRSSFSNYGQGLELMAPGSNIYSTYPNGQFRTLSGTSM
ATPHVAGVAGLIRAANPNISVAEARSILQNTAQYAGSFNQYGYGIVDANAAVRAAR
GQTEQPRYETNTTVSTNASTYRRGQSVTVRADVVDQDGRALANSTVQFTITRPNG
TTVTNTATTNSSGVATWTIGTSSSTARGTYGVQAATSLSGYEGSTATTSFVVN.

The sequence of the predicted, fully processed mature chain (SWT40, 381 amino acids) is depicted in SEQ ID NO:34: MHNNQRWHYEMINAPQAWNVTTGSRNVRIAVLDTGIDANHPNLRNLVNTSLGRSFVGGGTGDVQGHGTHVAGTIASYGSVSGVMQNATLIPVKVLGDNGSGSMYGIQQGILYAASVNSDVINMSLGGGGYSQGMDDAIRTAVSSGTIVVAATGNDSRGSISYPAAYSGAIAVGSVTSNRTRSSFSNYGQGLELMAPGSNIYSTYPNGQFRTLSGTSMATPHVAGVAGLIRAANPNISVAEARSILQNTAQYAGSFNQYGYGIVDANAAVRAARGQTEQPRYETNTTVSTNASTYRRGQSVTVRADVVDQDGRALANSTVQFTITRPNGTTVTNTATTNSSGVATWTIGTSSSTARGTYGVQAATSLSGYEGSTATTSFVVN.

The amino acid sequence of the preproenzyme form of SWT41 is depicted in SEQ ID NO:35. The predicted signal peptide sequence is underlined and the pro sequence is in italics. The sequence of the predicted, fully processed mature chain (SWT41, 381 amino acids) is in bold.

SEQ ID NO:35 sets forth the amino acid sequence of the preproenzyme form of SWT41: VKKSAVWVLMTVLVFSLFLNPAGIGAQA*SDAASEKDDTAYIEGQLIVSVKSSDVSVKGIEGLNKKIMGNVLRERGFAITDSIMGLGDPAEVNAFTNQEFSESWKNMGLVYLAEYDVSVYASVEEAKRALAEALKENGMEIRHISKNYEMHAIGEPADVSPQ***MHPNQQWHYNMINAPQAWGTTTGSSSVIQAVLDTGIDHNHQSLANLVNTSLGQSFVGGSTMDVQGHGTHVAGTIASYGSVSGVMHNATLVPVKVLNDSGSGSLFGITQGILYSADIGADVINMSLGGGGYNQSMAEAAQTAVNAGSIVIAASGNDGAGSISYPAAYSSVIAVGSVTSTGARSNFSNYGSGLELMAPGSNIYSTVPNNGYATFSGTSMASPHAAGVAGLMRAVNPNLSVSDARSIMQNTAQYAGSPTFYGYGIVDANAAVQQASGGSGGPSNITETSISTDRFYVQRGQNVTSTAQVTNENGQGLANATVTFTITRPNGSTLTNTATTNGSGFASWTVGTSGATATGTYSVEASSSLQGYQGSSASTSFFVY.**

The amino acid sequence of the proenzyme form of SWT41 is depicted in SEQ ID NO:36. The pro sequence is in italics. The sequence of the predicted, fully processed mature chain (SWT41, 381 amino acids) is in bold.

SEQ ID NO:36 sets forth the amino acid sequence of the proenzyme form of SWT41:

SDAASEKDDTAYIEGQLIVSVKSSDVSVKGIEGLNKKIMGNVLRERGFAITDSIMGLGDPAEVN
AFTNQEFSESVVKNMGLVYLAEYDVSVYASVEEAKRALAEALKENGMEIRHISKNYEMHAIGE
PADVSPQMHPNQQWHYNMINAPQAWGTTTGSSSVIQAVLDTGIDHNHQSLANLVN
TSLGQSFVGGSTMDVQGHGTHVAGTIASYGSVSGVMHNATLVPVKVLNDSGSGSL
FGITQGILYSADIGADVINMSLGGGGYNQSMAEAAQTAVNAGSIVIAASGNDGAGSI
SYPAAYSSVIAVGSVTSTGARSNFSNYGSGLELMAPGSNIYSTVPNNGYATFSGTSM

ASPHAAGVAGLMRAVNPNLSVSDARSIMQNTAQYAGSPTFYGYGIVDANAAVQQA
SGGSGGPSNITETSISTDRFYVQRGQNVTSTAQVTNENGQGLANATVTFTITRPNGS
TLTNTATTNGSGFASWTVGTSGATATGTYSVEASSSLQGYQGSSASTSFFVY.

The sequence of the predicted, fully processed mature chain (SWT41, 381 amino acids) is depicted in SEQ ID NO:37: MHPNQQWHYNMINAPQAWGTTTGSSSVIQAVLDTGIDHNHQSLANLVNTSLGQSFVGGSTMDVQGHGTHVAGTIASYGSVSGVMHNATLVPVKVLNDSGSGSLFGITQGILYSADIGADVINMSLGGGGYNQSMAEAAQTAVNAGSIVIAASGNDGAGSISYPAAYSSVIAVGSVTSTGARSNFSNYGSGLELMAPGSNIYSTVPNNGYATFSGTSMASPHAAGVAGLMRAVNPNLSVSDARSIMQNTAQYAGSPTFYGYGIVDANAAVQQASGGSGGPSNITETSISTDRFYVQRGQNVTSTAQVTNENGQGLANATVTFTITRPNGSTLTNTATTNGSGFASWTVGTSGATATGTYSVEASSSLQGYQGSSASTSFFVY.

The amino acid sequence of the preproenzyme form of SWT77 is depicted in SEQ ID NO:38. The predicted signal peptide sequence is underlined and the pro sequence is in italics. The sequence of the predicted, fully processed mature chain (SWT77, 381 amino acids) is in bold.

SEQ ID NO:38 sets forth the amino acid sequence of the preproenzyme form of SWT77:

LKKSAVWVLMTVLVFSLFLNPAGIGAQASDAASGKEEAAYIEGQLIVSVKASDASV
KGIEGV NQKVMGNELRERGFAITDSIMGLGDPAEVNAFTNQEFSESVVRNMGLVYLAEYDVS
VYKSSDEAKRSLAEALKENGMEIRHISENYEMHAIGEPADVSPQMHPNQQWHYNMINAPQ
AWETTTGSSSVIQAVLDTGIDHNHQSLANLVNTSLGQSFVGGSTMDVQGHGTHVA
GTIASYGSVSGVMHNATLVPVKVLNDSGSGSLFGITQGILYSADIGADVINMSLGGG
GYNQSMAEAAQTAVDAGSIVIAASGNDGAGSISYPAAYSSVIAVGSVTSTGARSNFS
NYGSGLELMAPGSNIYSTVPNNGYATFSGTSMAAPHAAGVAGLMRAVNSNLSVSD
ARSIMQNTAQYAGSPTFYGYGIVDANAAVQQASGGSGGPSNITETSISTDRYYVQR
GQNVTSTAQVTNENGQALANATVTFTITRPNGSTLTNTATTNSSGVASWTVGTSGG
TATGTYSVEASSSLQGYQGSSASTSFFVY.

The amino acid sequence of the proenzyme form of SWT77 is depicted in SEQ ID NO:39. The pro sequence is in italics. The sequence of the predicted, fully processed mature chain (SWT77, 381 amino acids) is in bold.

SEQ ID NO:39 sets forth the amino acid sequence of the proenzyme form of SWT77:

SDAASGKEEAAYIEGQLIVSVKASDASVKGIEGVNQKVMGNELRERGFAITDSIMGLGDPAEV
NAFTNQEFSESVVRNMGLVYLAEYDVSVYKSSDEAKRSLAEALKENGMEIRHISENYEMHAIG

EPADVSPQMHPNQQWHYNMINAPQAWETTTGSSSVIQAVLDTGIDHNHQSLANLV
NTSLGQSFVGGSTMDVQGHGTHVAGTIASYGSVSGVMHNATLVPVKVLNDSGSGS
LFGITQGILYSADIGADVINMSLGGGGYNQSMAEAAQTAVDAGSIVIAASGNDGAG
SISYPAAYSSVIAVGSVTSTGARSNFSNYGSGLELMAPGSNIYSTVPNNGYATFSGTS
MAAPHAAGVAGLMRAVNSNLSVSDARSIMQNTAQYAGSPTFYGYGIVDANAAVQ
QASGGSGGPSNITETSISTDRYYVQRGQNVTSTAQVTNENGQALANATVTFTITRPN
GSTLTNTATTNSSGVASWTVGTSGGTATGTYSVEASSSLQGYQGSSASTSFFVY.

The sequence of the predicted, fully processed mature chain (SWT77, 381 amino acids) is depicted in SEQ ID NO:40: MHPNQQWHYNMINAPQAWETTTGSSSVIQAVLDTGIDHNHQSLANLVNTSLGQSFVGGSTMDVQGHGTHVAGTIASYGSVSGVMHNATLVPVKVLNDSGSGSLFGITQGILYSADIGADVINMSLGGGGYNQSMAEAAQTAVDAGSIVIAASGNDGAGSISYPAAYSSVIAVGSVTSTGARSNFSNYGSGLELMAPGSNIYSTVPNNGYATFSGTSMAAPHAAGVAGLMRAVNSNLSVSDARSIMQNTAQYAGSPTFYGYGIVDANAAVQQASGGSGGPSNITETSISTDRYYVQRGQNVTSTAQVTNENGQALANATVTFTITRPNGSTLTNTATTNSSGVASWTVGTSGGTATGTYSVEASSSLQGYQGSSASTSFFVY.

The amino acid sequence of the preproenzyme form of SWT123 is depicted in SEQ ID NO:41. The predicted signal peptide sequence is underlined and the pro sequence is in italics. The sequence of the predicted, fully processed mature chain (SWT123, 488 amino acids) is in bold.

SEQ ID NO:41 sets forth the amino acid sequence of the preproenzyme form of SWT123: MKKLLTLFLLTLVMLVGLFSVNVMA*DNEDQKYIEGQLIVSVETNVGGYSITGLMNNTSEILQDNATLRNKGFHVADTLLENNAAGVQSVFSSNFVEETAKRTGLVYLMEYSPEDYESIQEAKNDLENTLKELGLKVRYVSENFVVELFETETPSNTDEENIISPF***MHSNQEWHYGMINAPDAWGITTGSSNVRIAILDTGIDSSHPSLRNLVDTGLGRSYVGGSPEDVQGHGTHVAGTIASYGAVSGVMQDATLISVKVLGDDGSGSMYGIQQGVLYAASVGADVINMSLGGGGYNQGFSDAIDTAVANGTVVIAASGNDGRASISYPAAYDGAIAVGSVTSSGNRSNFSNYGSGLELMAPGSSIYSTYPNGQYRTLSGTSMAAPHAAGVAGLVRAVNPNLSVAEVRSILADTAQYAGSTYQYGNGIVDAFAAVQAAGGSGGTPSPGVTNTVVSTDKSVYERGDQVTMTATVTDEDGNALQGASVNYTITRPNGSDVTNTATTNTNGIATWTIGSNSQTAIGTYDVTAESSLSGYESSTDTTSFRFSDQAQSQQTVTDVSTNSSYYARGQNVTISAEVTDQDGAALSNATVSFTITRPNGSTLTNTATTNSAGVASWTVSTSSGTARGTYEVTAESTYSTYEGSSDTTSFYVY.**

The amino acid sequence of the proenzyme form of SWT123 is depicted in SEQ ID NO:42. The pro sequence is in italics. The sequence of the predicted, fully processed mature chain (SWT123, 488 amino acids) is in bold.

SEQ ID NO:42 sets forth the amino acid sequence of the proenzyme form of SWT123: *DNEDQKYIEGQLIVSVETNVGGYSITGLMNNTSEILQDNATLRNKGFHVADTLLENNAAGVQSVFSSNFVEETAKRTGLVYLMEYSPEDYESIQEAKNDLENTLKELGLKVRYVSENFVVELFETETPSNTDEENIISPF***MHSNQEWHYGMINAPDAWGITTGSSNVRIAILDTGIDSSHPSLRNLVDTGLGRSYVGGSPEDVQGHGTHVAGTIASYGAVSGVMQDATLISVKVLGDDGSGSMYGIQQGVLYAASVGADVINMSLGGGGYNQGFSDAIDTAVANGTVVIAASGNDGRASISYPAAYDGAIAVGSVTSSGNRSNFSNYGSGLELMAPGSSIYSTYPNGQYRTLSGTSMAAPHAAGVAGLVRAVNPNLSVAEVRSILADTAQYAGSTYQYGNGIVDAFAAVQAAGGSGGTPSPGVTNTVVSTDKSVYERGDQVTMTATVTDEDGNALQGASVNYTITRPNGSDVTNTATTNTNGIATWTIGSNSQTAIGTYDVTAESSLSGYESSTDTTSFRFSDQAQSQQTVTDVSTNSSYYARGQNVTISAEVTDQDGAALSNATVSFTITRPNGSTLTNTATTNSAGVASWTVSTSSGTARGTYEVTAESTYSTYEGSSDTTSFYVY.**

The sequence of the predicted, fully processed mature chain (SWT123, 488 amino acids) is depicted in SEQ ID NO:43: MHSNQEWHYGMINAPDAWGITTGSSNVRIAILDTGIDSSHPSLRNLVDTGLGRSYVGGSPEDVQGHGTHVAGTIASYGAVSGVMQDATLISVKVLGDDGSGSMYGIQQGVLYAASVGADVINMSLGGGGYNQGFSDAIDTAVANGTVVIAASGNDGRASISYPAAYDGAIAVGSVTSSGNRSNFSNYGSGLELMAPGSSIYSTYPNGQYRTLSGTSMAAPHAAGVAGLVRAVNPNLSVAEVRSILADTAQYAGSTYQYGNGIVDAFAAVQAAGGSGGTPSPGVTNTVVSTDKSVYERGDQVTMTATVTDEDGNALQGASVNYTITRPNGSDVTNTATTNTNGIATWTIGSNSQTAIGTYDVTAESSLSGYESSTDTTSFRFSDQAQSQQTVTDVSTNSSYYARGQNVTISAEVTDQDGAALSNATVSFTITRPNGSTLTNTATTNSAGVASWTVSTSSGTARGTYEVTAESTYSTYEGSSDTTSFYVY.

### COMPARATIVE EXAMPLE 13

### Identification of Homologous Proteases

The amino acid sequences of the predicted mature forms of BspAG00296 (SEQ ID NO:3, 274 amino acids), BspM04033 (SEQ ID NO:11, 276 amino acids), and SWT77 (SEQ ID NO:40, 381 amino acids) were subjected to a BLAST search (Altschul et al., Nucleic Acids Res, 25:3389-402, 1997) against the NCBI non-redundant protein database. A similar search was run against the Genome Quest Patent database with search parameters set to default values using SEQ ID NO:3, SEQ ID NO:7, and SEQ ID NO:40, respectively as the query sequences. Subsets of the search results are shown in Tables 6 and 7 for BspAG00296; Tables 8 and 9 for BspM04033; and Tables 10 and 11 for SWT77. Percent identity (PID) for both search sets was defined as the number of identical residues divided by the number of aligned residues in the pairwise alignment. The column labeled "Sequence Length" refers to the length (in amino acids) of the protein sequences associated with the listed Accession Nos., while the column labeled "Aligned Length" refers to the length (in amino acids) of the aligned protein sequence used for the PID calculation.

| **Table 6: List of sequences with percent identity to BspAG00296 (SEQ ID NO:3) protein identified from the NCBI non-redundant protein database** | | | | |
|---|---|---|---|---|
| **Accession #** | **PID** | **Organism** | **Sequence Length** | **Alignment Length** |
| WP_026675114.1 | 82 | *B. bogoriensis* | 539 | 273 |
| WP_010283106 | 77 | *B. timonensis* | 544 | 273 |
| WP_006679321 | 77 | *P. dendritformis* | 578 | 273 |
| WP_025025887.1 | 75 | *B. mannanilyticus* | 550 | 272 |
| WP_026080796.1 | 54 | *B. licheniformis* | 378 | 252 |
| CAJ70731 | 53 | *B. licheniformis* | 379 | 252 |
| BAA06157 | 50 | *B. sp. Sendai* | 382 | 267 |
| AAA22212 | 50 | *B. alcalophilus* | 380 | 267 |
| WP_006636716 | 50 | *B. sonorensis* | 378 | 252 |
| AAC43581 | 50 | *B. sp SprD* | 379 | 259 |
| P29599 | 50 | *B. lentus* | 269 | 267 |
| ABI26631.1 | 46 | *B clausii* | 361 | 264 |
| WP_010333625 | 49 | *B mojavensis* | 381 | 267 |
| BAN09118 | 49 | *B subtilis* | 381 | 267 |
| WP_012957236.1 | 48 | *B pseudofirmus* | 374 | 253 |
| CAA74536 | 48 | *B subtilis str 168* | 381 | 267 |
| WP_010329279 | 48 | *B vallismortis* | 381 | 267 |
| BAD21128 | 48 | *B. sp _KSM-LD1* | 377 | 269 |
| AAC43580 | 47 | *B. sp. SprC* | 378 | 273 |
| BAD 11988 | 47 | *B. sp. KSM-LD1* | 376 | 273 |
| WP_003327717.1 | 47 | *B. atrophaeus* | 382 | 267 |
| ADN04910 | 48 | *B. circulans* | 275 | 267 |
| AFP23380.1 | 47 | *B. lehensis* | 276 | 267 |
| WP_007497196 | 48 | *B. stratosphericus* | 383 | 267 |
| ADK11996 | 48 | *B. pumilus* | 383 | 267 |
| CAA24990 | 46 | *B. amyloliquefaciens* | 376 | 264 |
| WP_022553591.1 | 46 | *B. methylotrophicus* | 382 | 264 |
| ABY25856 | 46 | *G. stearothermophilus* | 382 | 264 |

| **Table 7: List of sequences with percent identity to BspAG00296 (SEQ ID NO:3) protein identified from the Genome Quest database** | | | | |
|---|---|---|---|---|
| **Patent ID #** | **PID** | **Organism** | **Sequence Length** | **Alignment Length** |
| WO2012175708-0004 | 82.85 | *B. bogoriensis* | 541 | 274 |
| WO2012175708-0002 | 82.35 | *B. sp. NN018132* | 548 | 274 |
| WO2012175708-0006 | 77.66 | *P. dendritiformis* | 578 | 273 |
| DE10260903 | 55.16 | *B. licheniformis synthetic* | 379 | 252 |
| JP2002533080-0001 | 54.55 | *B. licheniformis* | 275 | 253 |
| JP1991072876-0004 | 54.37 | *B. licheniformis* | 274 | 252 |
| US5719021-0004 | 54.37 | *B. licheniformis* | 350 | 252 |
| WO2011014278-0109 | 54.37 | *B. licheniformis* | 274 | 252 |
| US6274365-0007 | 54.37 | *B. licheniformis* | 274 | 252 |
| US6908991-0004 | 54.37 | *B. sp.* | 274 | 252 |
| EP0405901-0008 | 54.37 | *B. sp.* | 274 | 252 |
| US7449187-0007 | 50.19 | *B. sp.* | 268 | 267 |
| WO2010123754-0051 | 50.19 | *B. sp.* | 269 | 267 |
| US20110045572-0034 | 50.19 | *B. sp.* | 269 | 267 |

| **Table 8: List of sequences with percent identity to BspM04033 (SEQ ID NO:11) protein identified from the NCBI non-redundant protein database** | | | | |
|---|---|---|---|---|
| **Accession #** | **PID** | **Organism** | **Sequence Length** | **Alignment Length** |
| WP_010283106 | 77 | *B. timonensis* | 544 | 275 |
| WP_026675114.1 | 75 | *B. bogoriensis* | 539 | 276 |
| WP_025025887.1 | 73 | *B. mannanilyticus* | 550 | 276 |
| WP_006679321 | 71 | *P. dendritiformis* | 578 | 275 |
| AEU12640.1 | 48 | *B. licheniformis* | 379 | 255 |
| WP_024712963.1 | 48 | *B. tequilensis* | 894 | 226 |
| WP_014113314.1 | 48 | *B. subtilis subsp. spizizenii TU-B-10* | 894 | 226 |
| WP_014730854.1 | 48 | *Mesotoga prima MesG1.Ag.4.2* | 503 | 267 |
| WP_010329314.1 | 48 | *B. vallismortis* | 893 | 226 |
| AAC43580 | 47 | *B. sp. SprC* | 378 | 274 |
| CAJ70731 | 47 | *B. licheniformis* | 379 | 269 |
| WP_012957236.1 | 47 | *B. pseudofirmus* | 374 | 251 |
| AAC43581 | 46 | *B. sp. SprD* | 379 | 270 |
| BAD11988 | 46 | *B. sp. KSM-LD1 SA protease* | 376 | 274 |
| P27693.1 | 46 | *B. alcalophilus* | 380 | 269 |
| BAD21128 | 46 | *B. sp_KSM_LD1* | 377 | 270 |
| P29599 | 44 | *B. lentus Savinase* | 269 | 269 |
| P41362.1 | 45 | *B. clausii* | 380 | 269 |
| BAA06157 | 45 | *B. sp. Sendai* | 382 | 269 |
| ADN04910 | 44 | *B. circulans* | 275 | 267 |
| AFP23380.1 | 43 | *B. lehensis* | 276 | 267 |
| WP_007497196 | 43 | *B. stratosphericus* | 383 | 267 |
| ADK11996 | 43 | *B. pumilus* | 383 | 267 |
| WP_006636716 | 43 | *B. sonorensis* | 378 | 269 |
| WP_003327717.1 | 42 | *B. atrophaeus* | 382 | 271 |

| **Table 8: List of sequences with percent identity to BspM04033 (SEQ ID NO:11) protein identified from the NCBI non-redundant protein database** | | | | |
|---|---|---|---|---|
| **Accession #** | **PID** | **Organism** | **Sequence Length** | **Alignment Length** |
| WP_017417394.1 | 41 | *B. amyloliquefaciens* | 382 | 271 |
| WP_010333625 | 41 | *B. mojavensis* | 381 | 271 |
| WP_032721270.1 | 41 | *B. subtilis* | 381 | 271 |
| WP_015252429.1 | 41 | *B. subtilis subsp. subtilis str. BSP1* | 381 | 271 |
| WP_022553591.1 | 41 | *B. methylotrophicus* | 382 | 271 |
| ABY25856 | 41 | *G. stearothermophilus* | 382 | 271 |

| **Table 9: List of sequences with percent identity to BspM04033 (SEQ ID NO:11) protein identified from the Genome Quest database** | | | | |
|---|---|---|---|---|
| **Patent ID #** | **PID** | **Organism** | **Sequence Length** | **Alignment Length** |
| WO2012175708-0004 | 75.2 | *B. bogoriensis* | 541 | 274 |
| WO2012175708-0002 | 74.6 | *B. sp. NN018132* | 548 | 276 |
| WO2012175708-0006 | 71.6 | *P. dendritiformis* | 578 | 275 |
| WO9628566 | 47.9 | *Synthetic* | 274 | 269 |
| WO9406915-0001 | 47.8 | *empty* | 275 | 274 |
| CN102676561-0002 | 47.2 | *Bacillus licheniformis* | 350 | 269 |
| US20090011489-0005 | 47.2 | *B. licheniformis* | 274 | 269 |
| CN101215534-0002 | 47.2 | *B. licheniformis YP1A* | 379 | 269 |
| WO2011014278-0116 | 47.1 | *B. licheniformis* | 269 | 263 |
| WO2009005647-0008 | 47.1 | *B. licheniformis* | 374 | 263 |
| US5275945-0002 | 47.1 | *empty* | 377 | 274 |
| WO2011014278-0109 | 46.8 | *B. licheniformis* | 274 | 269 |
| US8168417-5227 | 46.8 | *B. licheniformis* | 379 | 269 |
| US20030049619-0011 | 46.8 | *B. licheniformis* | 379 | 269 |
| EP2166076-0002 | 46.5 | *B. licheniformis* | 274 | 269 |
| EP1921148-0026 | 46.2 | *B. licheniformis* | 378 | 273 |

| **Table 10: List of sequences with percent identity to SWT77 (SEQ ID NO:40) protein identified from the NCBI non-redundant protein database** | | | | |
|---|---|---|---|---|
| **Accession #** | **PID** | **Organism** | **Sequence Length** | **Alignment Length** |
| WP_026675114.1 | 70.8 | *B. bogoriensis* | 539 | 380 |
| WP_010283106.1 | 69.8 | *B. timonensis* | 544 | 387 |
| WP_025025887.1 | 69.8 | *B. mannanilyticus* | 550 | 380 |
| WP_006679321.1 | 58.5 | *P. dendritiformis C454* | 578 | 381 |
| WP_014113314.1 | 48.2 | *B. subtilis subsp. spizizenii TU-B-10* | 894 | 226 |
| AAT75303.1 | 48.2 | *B. mojavensis* | 379 | 226 |
| WP_024712963.1 | 48.2 | *B. tequilensis* | 894 | 226 |
| WP_010329314.1 | 47.8 | *B. vallismortis* | 893 | 226 |
| AEU12640.1 | 47.8 | *B. licheniformis* | 379 | 255 |
| AAC43580.1 | 47.4 | *B. sp.SprC* | 378 | 274 |
| WP_012957236.1 | 46.2 | *B. pseudofirmus OF4* | 374 | 251 |
| AAC43581.1 | 45.6 | *B. sp. SprD* | 379 | 270 |
| BAD11988.2 | 45.6 | *B. sp. KSM-LD1 SA* | 376 | 274 |
| AIC95824.1 | 45.4 | *B. lehensis* | 378 | 269 |
| BAD21128.1 | 45.2 | *B. sp. KSM-LD1 SB* | 377 | 270 |
| BAA06157.1 | 44.2 | *B. alcalophilus* | 382 | 269 |
| P29599.1 | 43.9 | *B. lentus* | 269 | 269 |
| WP_006636716.1 | 43.5 | *B. sonorensis* | 378 | 255 |
| AAX14553.1 | 43.1 | *B. pumilus* | 381 | 267 |
| WP_033016381.1 | 42.1 | *G. stearothermophilus* | 351 | 273 |

| **Table 11: List of sequences with percent identity to SWT77 (SEQ ID NO:40) protein identified from the Genome Quest database** | | | | |
|---|---|---|---|---|
| **Patent ID #** | **PID** | **Organism** | **Sequence Length** | **Alignment Length** |
| WO2012175708-0004 | 69.74 | *B. bogoriensis* | 541 | 380 |
| WO2012175708-0002 | 69.05 | *B. sp. NN018132* | 548 | 378 |
| WO2012175708-0006 | 58.53 | *P. dendritiformis* | 578 | 381 |
| CN102703482-0002 | 49.2 | *B. licheniformis; YP1A CCTCC M207021 Synthetic* | 379 | 250 |
| WO9628566 | 49.02 | *Synthetic* | 274 | 255 |
| WO2005124012-0020 | 48.24 | *B. licheniformis Synthetic* | 274 | 255 |
| WO03062380-0005 | 48.03 | *B. licheniformis* | 273 | 254 |
| WO2005124012-0014 | 47.84 | *B. licheniformis Synthetic* | 274 | 255 |
| WO9406915-0001 | 47.45 | *B. sp.* | 275 | 274 |
| US5275945-0002 | 46.35 | *B. sp.* | 377 | 274 |

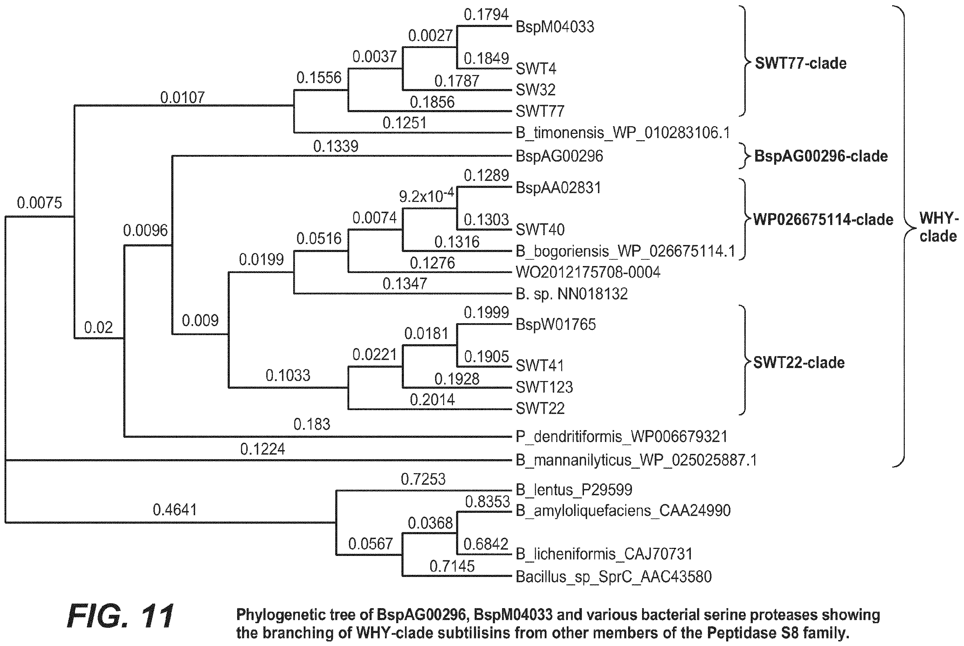

A phylogenetic tree for amino acid sequences of the following subtilisins was built: BspAG00296 (SEQ ID NO:4), BspM04033 (SEQ ID NO:11), BspW01765 (SEQ ID NO:15), BspAA02831 (SEQ ID NO:22), SWT4 (SEQ ID NO:25), SWT22 (SEQ ID NO:28), SWT32 (SEQ ID NO:31), SWT40 (SEQ ID NO:34), SWT41 (SEQ ID NO:37), SWT77 (SEQ ID NO:40), SWT123 (SEQ ID NO:43), *B. amyloliquefaciens* (NCBI Accession No: CAA24990), *B. lentus* (NCBI Accession NO: P29599), *B. sp.* SprC (NCBI Accession No: AAC43580), *B. licheniformis* (NCBI Accession No: CAJ70731), *B. sp.* NN018132 (SEQ ID NO:17) and *B. bogoriensis* (SEQ ID NO:4 from WO2012175708A2), *B. bogoriensis* (NCBI Accession NO: WP026675114.1, *B. timonensis* (NCBI Accession No: WP010283106), and *P. dendritiformis* (NCBI Accession NO: WP006679321). The sequences were entered in the Vector NTI Advance suite and a Guide Tree was created using the Neighbor Joining (NJ) method (Saitou and Nei, Mol Biol Evol, 4:406-425, 1987). The tree construction was calculated using the following parameters: Kimura's correction for sequence distance and ignoring positions with gaps. AlignX displays the calculated distance values in parenthesis following the molecule name displayed on the phylogenetic tree shown in Figure 11. The BspAG00296, BspM04033, BspW01765, BspAA02831, SWT4, SWT22, SWT32, SWT40, SWT41, SWT77, SWT123, *B. sp.* NN018132 (SEQ ID NO:17) and *B. bogoriensis* (SEQ ID NO:4 from WO2012175708A2), *B. bogoriensis* (NCBI Accession NO: WP026675114.1, *B. Timonensis* (NCBI Accession NO: WP010283106), and *P. dendritiformis* (NCBI Accession NO: WP006679321) subtilisins all cluster in the same region (as shown in Figure 11) to form the WHY-clade. The BspM04033, SWT4, SWT32, and SWT77 subtilisins all cluster in the same sub-region (as shown in Figure 11) to form the SWT77-clade. The BspAG00296 subtilisin clusters in the sub-region (as shown in Figure 11) to form the BspAG00296-clade. The BspAA02831, SWT40, and WP026675114 subtilisins all cluster in the same sub-region (as shown in Figure 11) to form the WP026675114-clade. The BspW01765, SWT41, SWT123, and SWT22 subtilisins all cluster in the same sub-region (as shown in Figure 11) to form the SWT22-clade.

### EXAMPLE 14

### Unique features of WHY-clade subtilisins

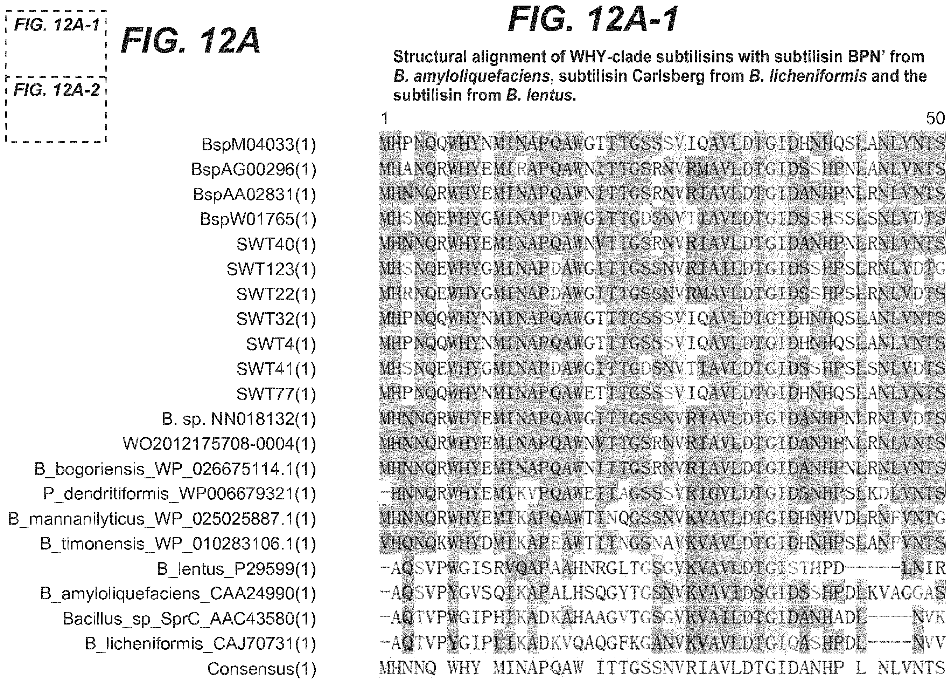

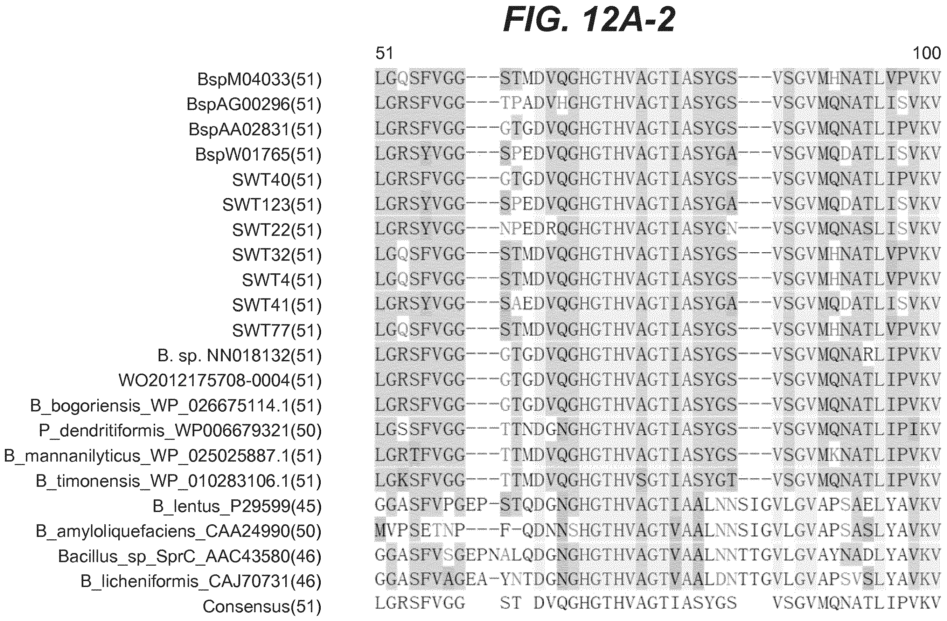

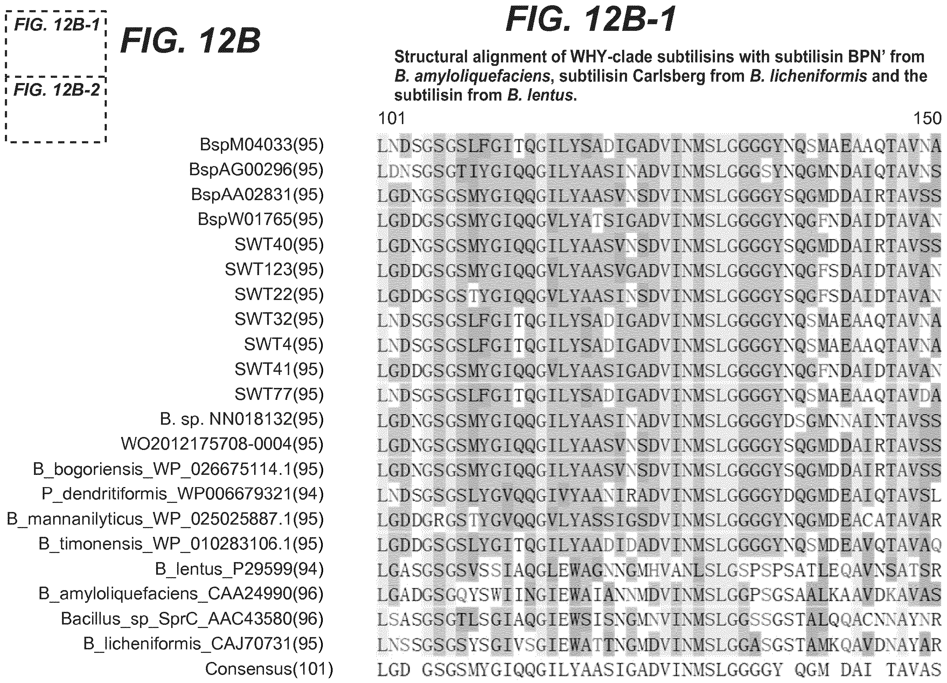

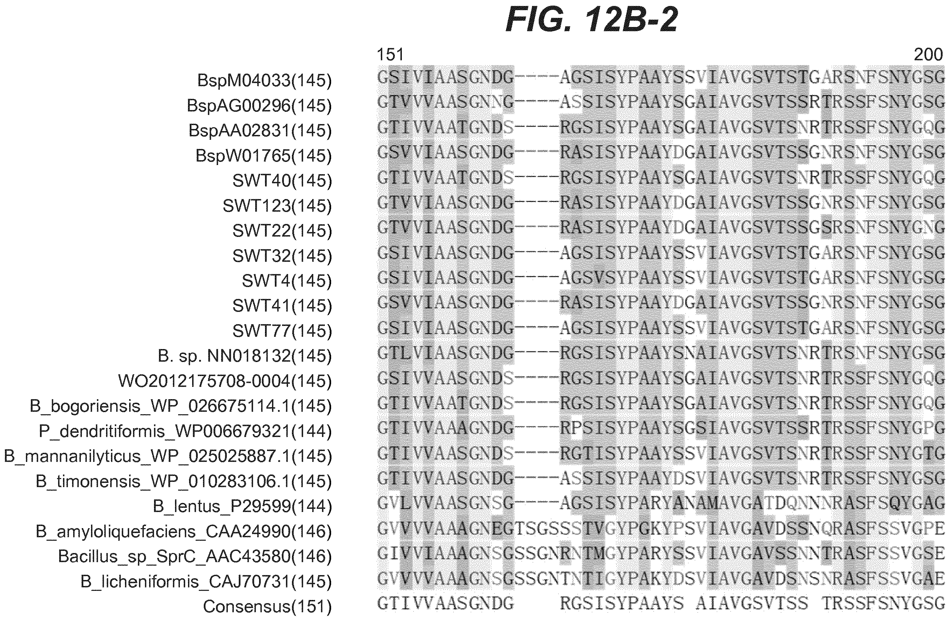

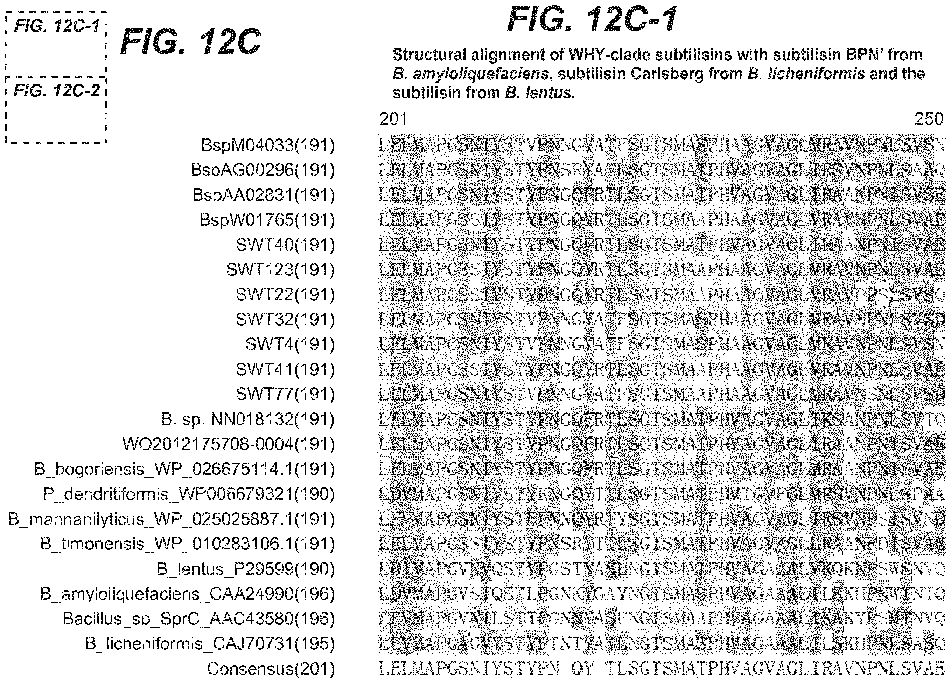

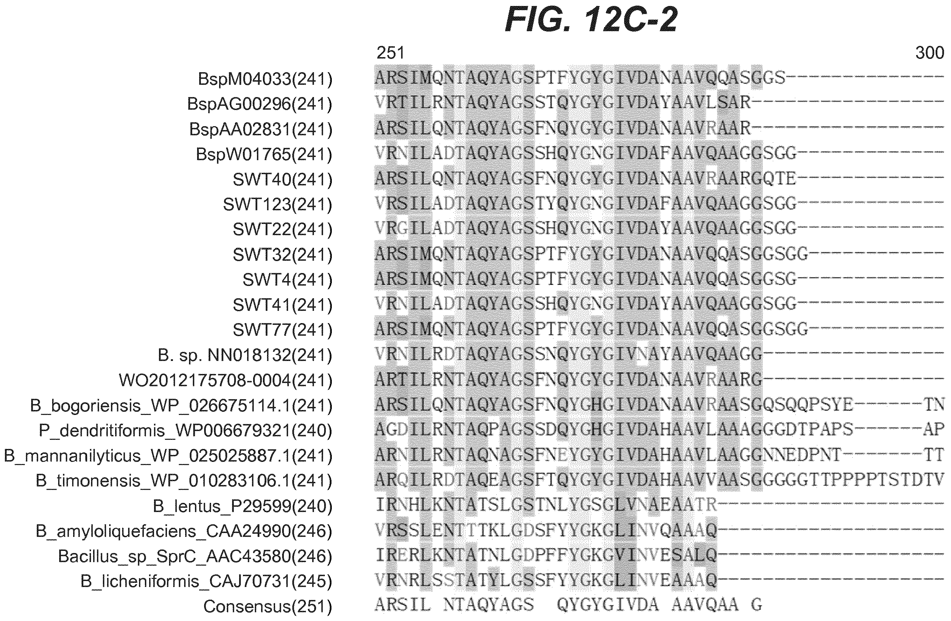

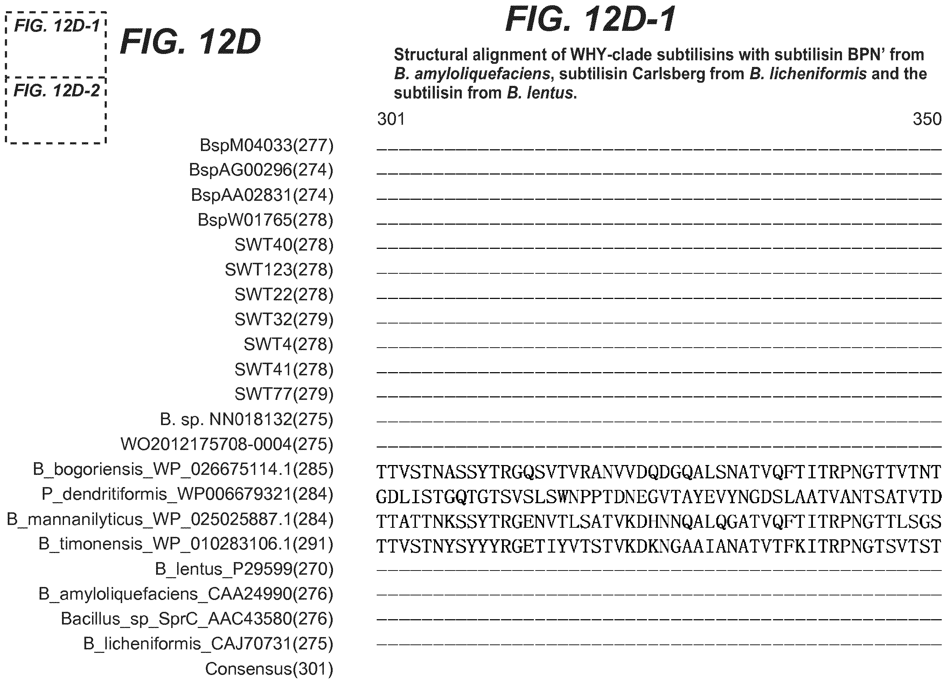

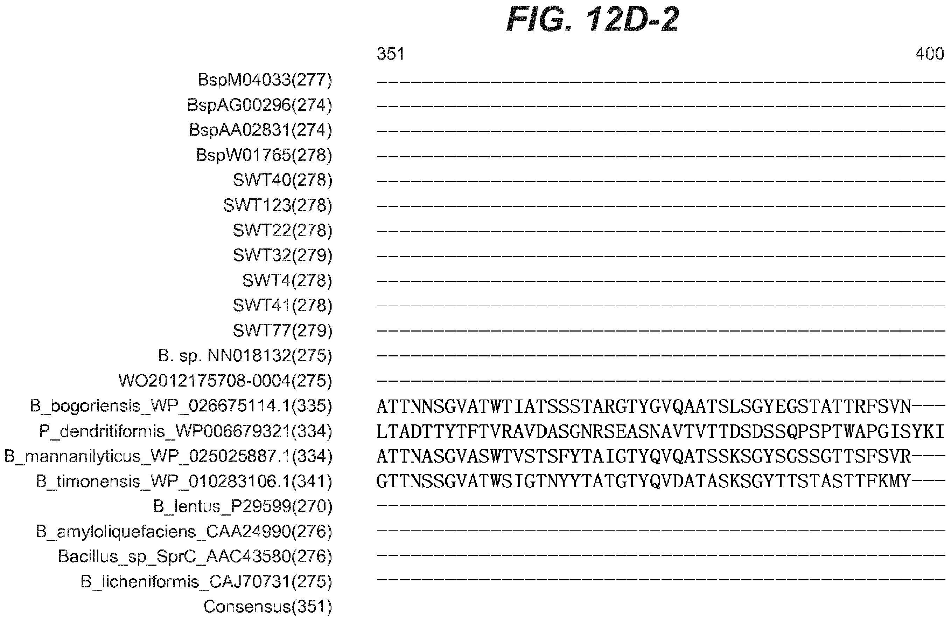

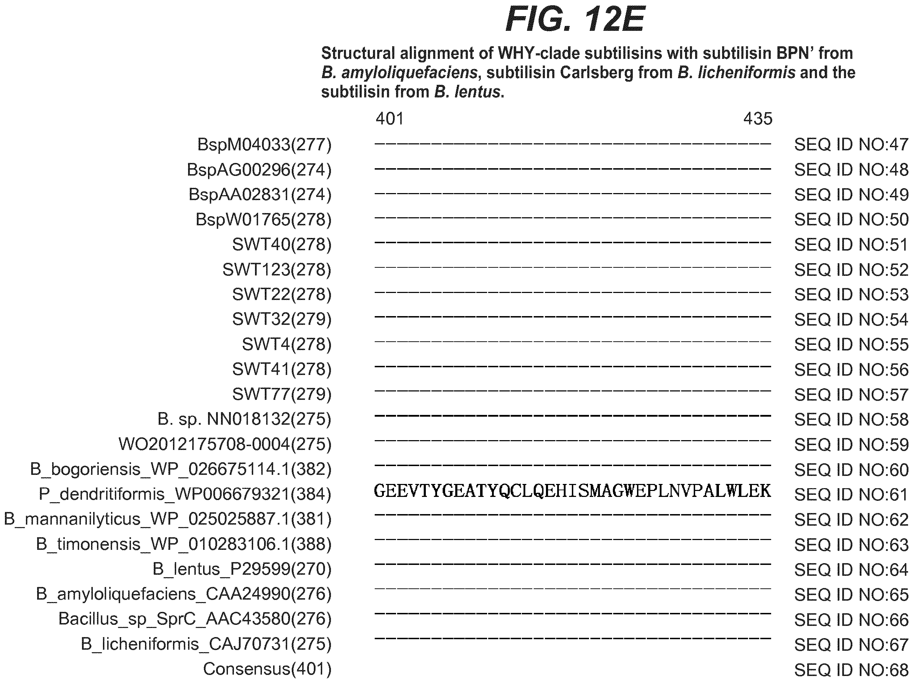

A structure based alignment of the following proteases BspAG00296 (SEQ ID NO:4), BspM04033 (SEQ ID NO:11), BspW01765 (SEQ ID NO:15), BspAA02831 (SEQ ID NO:22), SWT4 (SEQ ID NO:25), SWT22 (SEQ ID NO:28), SWT32 (SEQ ID NO:31), SWT40 (SEQ ID NO:34), SWT41 (SEQ ID NO:37), SWT77 (SEQ ID NO:40), SWT123 (SEQ ID NO:43), BPN' subtilisin from *B. amyloliquefaciens* (pdb entry 2STI), Carlsberg from *B. licheniformis* (pdb entry 1CSE), *B. lentus* subtilisin (pdb entry 1JEA), *B. sp.* NN018132 (SEQ ID NO:17), *B. bogoriensis* (WO2012175708-004), *B. bogoriensis* (NCBI Accession No: WP026675114), *B. mannanilyticus* (NCBI Accession No: WP025025887), *B. timonensis* (NCBI Accession No: WP010283106), and *P*. *dendritiformis* (NCBI Accession No: WP006679321) was performed using the "align" option in the Molecular Operating Environment (MOE) software (Chemical Computing Group, Montreal, Quebec, Canada) to look for structural similarities, and is set forth in Figure 12A-1 - 12E. The alignment applies conserved structural motifs as an additional guide to conventional sequence alignment. This alignment was performed using standard program defaults present in the 2012.10 distribution of MOE.

As shown in Figure 12A-1 - 12E, the structural alignment of subtilisins BspAG00296, BspM04033, BspW01765, BspAA02831, SWT4, SWT22, SWT32, SWT40, SWT41, SWT77, SWT123, *B. sp.* NN018132 (SEQ ID NO:17), *B. bogoriensis* (WO2012175708-004), *B. bogoriensis* (NCBI Accession No: WP026675114), *B. mannanilyticus* (NCBI Accession No: WP025025887), *B. timonensis* (NCBI Accession No: WP010283106), and *P. dendritiformis* (NCBI Accession NO: WP006679321) sequences show a common pattern of one insertion and two deletions relative to the sequences of subtilisins: BPN' from *B. amyloliquefaciens,* Carlsberg from *B. licheniformis* and subtilisin from *B. lentus,* for which three dimensional structures are available (pdb entries 2ST1, 1CSE and 1JEA, respectively). The numbering of residues in the 1JEA and 1CSE structures is with respect to subtilisin BPN'; while the numbering of residues for BspM04033 and all other proteases shown is the consecutive linear sequence.

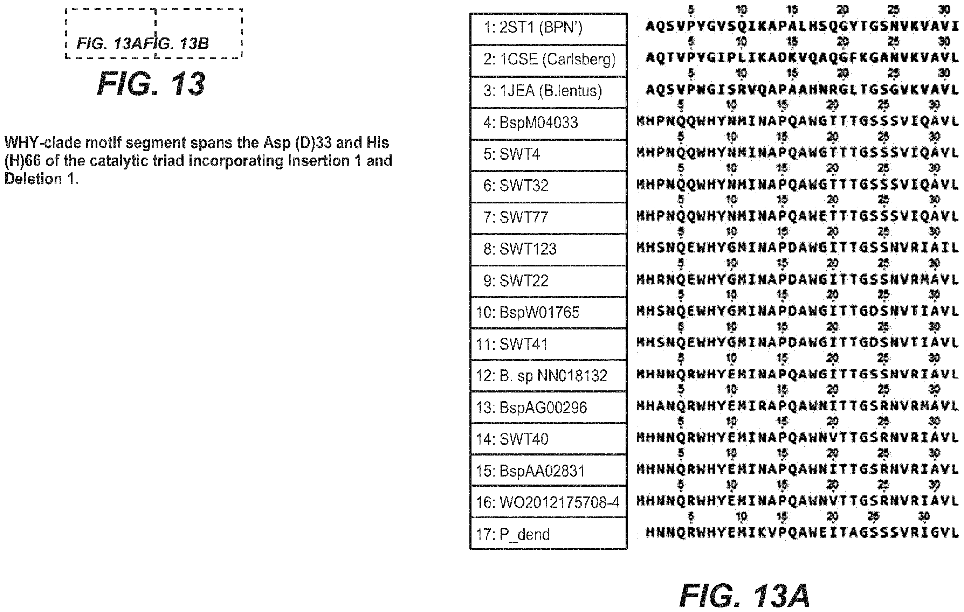

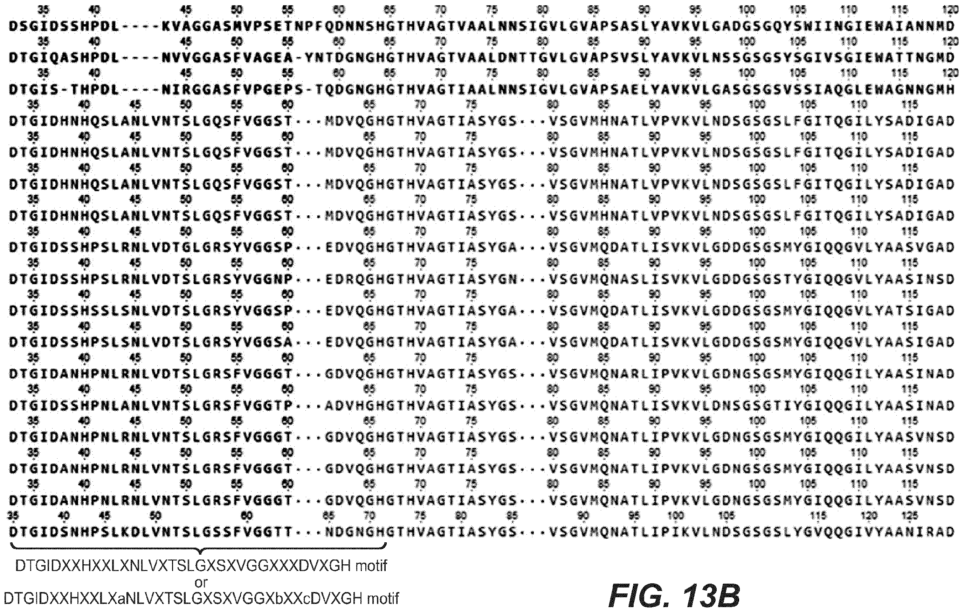

These WHY-clade subtilisins share sufficient features to create a clade, subsequently termed WHY-clade, where the term WHY derives from the complete conserved residues WHY near the N-terminus (W residue position 7 in BspM04033 and other members of this clade). In addition, the WHY-clade subtilisins share a common deletion with the *B. lentus* subtilisin and thus its structure will be used as a reference to understand the probable consequences of the differentiating characteristics of the WHY-clade subtilisins. With the exception of *P. dendritiformis, B. manannilyticus, B. timonensis* subtilisins, all other members of this clade have conserved residues NLV at positions corresponding to 45-47 in BspM04033 within the motif shown on Figure 13A - 13B. Other salient shared features of these WHY-clade subtilisins are: the sequence VQG (residues 63-65 in BspM04033) following deletion 1 within the motif (Figure 13A - 13B) and sequence VSG (residues 80-82 in BspM04033) following Deletion 2. This compilation of unique sequence regions impart the WHY-clade with salient differences from other commercial enzymes of the Peptidase S8 subtilisin family.

In Figure 13A - 13B, the WHY-clade motif is bracketed by the catalytic residues D33 and H66 (residue numbering according to BspM04033 linear sequence). The catalytic triad common to all serine proteases consists of Asp (D)33, His (H)66, and Ser (S)216.

The D33-H66 motif incorporates a common insertion (Insertion 1) and deletion (Deletion 1) found in all WHY-clade sequences when compared to *B. lentus* subtilisin and other commercial subtilisins. Insertion 1 results in the replacement of residues HPDLNIRGG (39-47) in *B. lentus* subtilisin with HQSLANLVNTSLG (40-52) in BspM04033. Deletion 1 results in replacement of residues VPGEPSTQDGNGH (51-64) in *B. lentus* subtilisin with residues VGGSTMDVQGH (56-66) in BspM04033. In pdb entry 1JEA, the numbering is with respect to subtilisin BPN'. Relative to subtilisin BPN', both *B. lentus* and Carlsberg subtilisins have a single residue deletion occurring at different sequence locations (see Figure 13A - 13B).

Outside of the WHY-clade motif described above, we find a second common deletion (Deletion 2) in the WHY-clade enzymes. In this instance, residues VAGTIAALNNSIGVLGVA PSAELYAVKV (68-95) of *B. lentus* subtilisin are replaced by VAGTIASYGSVSGVMHNATL VPVKV (70-94) in BspM04033. In subtilisins such as *B. lentus* as well as in BPN' and Carlsberg, this region forms a conserved calcium binding site. The residue modifications found in the Deletion 2 of BspM04033 sequence could result in loss of the corresponding calcium binding site.

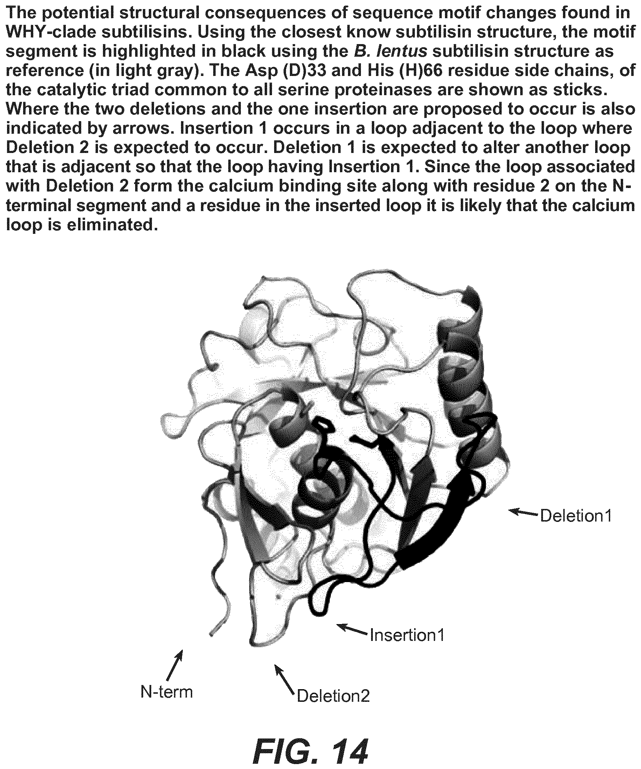

Figure 14 shows a model of the structure of a member of the WHY-clade using the structure of *B. lentus* subtilisin. The WHY-clade motif segment is highlighted in black using the *B. lentus* subtilisin structure as reference (in light gray). The Asp (D)33 and His (H)66 residue side chains of the catalytic triad common to all serine proteinases are shown as sticks. The juxtaposition of the loops where these two deletions and the one insertion are proposed to occur is also indicated by arrows.

In *B. lentus* subtilisin, along with subtilisin BPN' and Carlsberg, the segment encompassing residues 70-94 forms an extended loop to create a tight calcium binding site along with residue Asp 41 and the residue Gln2 found at the N-terminus of these subtilisins. This calcium binding site is an integral part of these subtilisins. Removal of this calcium binding by mutagenesis substantially reduces stability in subtilisin BPN' (Bryan et al. 1991 Biochemistry 31 4937-4945). Because of Deletion 2, it is expected that the reduced loop region comprised of residues TIASYGSVSGV (73-83) in BspM04033 subtilisin will no longer bind calcium. It is worth noting that Asp41 occurs in the region of Insertion 1 and the sequence at the N-terminus of WHY-clade subtilisins is substantially divergent from other subtilisins at the N-terminus of the mature protein (Figure 13A - 13B). It is postulated here that Insertion 1 along with the substantially disparate N-terminal sequence of WHY-clade proteases (Figure 13A - 13B) will confer protein stability to compensate for the removal of the aforementioned calcium binding loop. It is known that subtilisins BPN', Carlsberg, and *B. lentus* are strongly stabilized by calcium bound in the loop that is eliminated by Deletion 2. Since we find that the WHY-clade subtilisin are stabilized relative to these other subtilisins in the presence of detergent containing chelators such as EDTA, it is likely that this very attractive feature is a consequence of the unique sequence motif found in the WHY-clade subtilisins in combination with Deletion 2 and the altered N-terminus. Finally, the other common deletion, Deletion 1 is seen to occur in another nearby loop (Figure 14) and may be postulated to modulate the protein main chain fold to complement the changes imposed by Insertion 1 and Deletion 2. From Figure 14, it is clear that the Insertion 1 occurs in a loop that is adjacent to another loop that will be reduced by Deletion 2. It is postulated that in the three dimensional structure of the WHY-clade, the loop that is expanded will compensate for the cavity created by the Deletion 2.

Listed below are residue differences between the most stable member of the WHY-clade enzymes reported here, BspM04033, and another previously described member, *B. sp.* NN018132: P3N, Q6R, N10E, T20I, S26N, I28R, Q29I, H38A, Q41P, S42N, A44R, N48D, Q53R, S59G, M61G, H85Q, T88R, V90I, N96G, S98N, L103M, F104Y, T107Q, S113A, D115S, G117N, N131D, Q132S, S133D, A136N, A137N, A138I, Q139N, N143S, A144S, S146T, I147L, A157R, S168N, V169A, T178N, G179R, A180T, V204Y, N207G, G208Q, Y209F, A210R, F212L, S219T, A222V, N229I, R230K, A231S, V231A, S239T, N240Q, A241V, S243N, M245L, Q246R, N247D, P255L, T256N, F257Q, D264N, N266Y, Q271A, and S273G.

### EXAMPLE 15

### Crystallographic structures of WHY-clade subtilisins

The three-dimensional structures of two truncated WHY-clade structures were determined using X-ray crystallography. The structures of purified BspAG00296 (SEQ ID NO:4, 273 amino acids and purified SWT77 which consists of a truncated form SWT77-tr (SEQ ID NO:44, 273 amino acids) proteins were solved. The two proteins share the WHY-clade motif but have linear amino acid sequences that are only 74.4% identical.

The sequence of the truncated SWT77 protease (SWT77-tr, 273 amino acids) that was isolated and crystallized is depicted in SEQ ID NO:44:

MHPNQQWHYNMINAPQAWETTTGS
SSVIQAVLDTGIDHNHQSLANLVNTSLGQSFVGGSTMDVQGHGTHVAGTIASYGSVSG
VMHNATLVPVKVLNDSGSGSLFGITQGILYSADIGADVINMSLGGGGYNQSMAEAAQT
AVDAGSIVIAASGNDGAGSISYPAAYSSVIAVGSVTSTGARSNFSNYGSGLELMAPGSNI
YSTVPNNGYATFSGTSMAAPHAAGVAGLMRAVNSNLSVSDARSIMQNTAQYAGSPTF
YGYGIVDANAAVQQAS.

The structure of BspAG00296 was determined in the space group C2 having two molecules in the asymmetric unit with unit cell dimensions a=111.1, b=63.3 and c=72.7 Å and β =90.02° to a resolution of 1.5Å. The crystals were obtained by the hanging drop method starting with a 1% protein solution in 20mM sodium acetate buffer pH 5.5 and 0.15M NaCl. The reservoir solution contained 0.8M NH₄SO₄, 200mM MgCl₂ and 0.1M Bis-Tris Propane pH 6.5. Data was collected on a Bruker X8 Proteum system (Bruker Axis Inc., Madison, WI, USA). The structure was determined using molecular replacement with the coordinates of *B. lentus* subtiltin pdb entry 1JEA as a starting model. The coordinates for BspAG00296 were fitted in the resulting electron density using the program COOT (Emsley, P et al Acta Cryst. D66 486-501 (2010)). After fitting and refitting adjustments, the coordinates were refined using the REFMAC program with standard defaults in the CCP4 software suite. The final model had good stereochemistry and a R-work of 0.14 and R-free of 0.15 for all data to 1.5Å.

The structure of SWT77-tr was determined in the space group P21212 having four molecules in the asymmetric unit with unit cell dimensions a=149.3, b=80.1 and c=82.4Å to a resolution of 0.188Å. The crystals were obtained by the hanging drop method starting with a 20 mg/mL SWT-77-tr protein stock in 50mM sodium acetate pH 5.5 and 0.10M sodium chloride and 1mM PMSF. The reservoir solution contained 3.5M sodium formate + 0.10M Bicine pH 9.0. Data was collected on a Bruker X8 Proteum. The structure was determined using molecular replacement using a monomer of the BspAG00296 structure as a starting model. The coordinates for SWT77-tr were fitted using the Coot software package and the model was refined using the REFMAC program in the CCP4 software package system. The final model had good stereochemistry and a R-work of 0.17 and R-free of 0.22 for all data to 1.88Å.

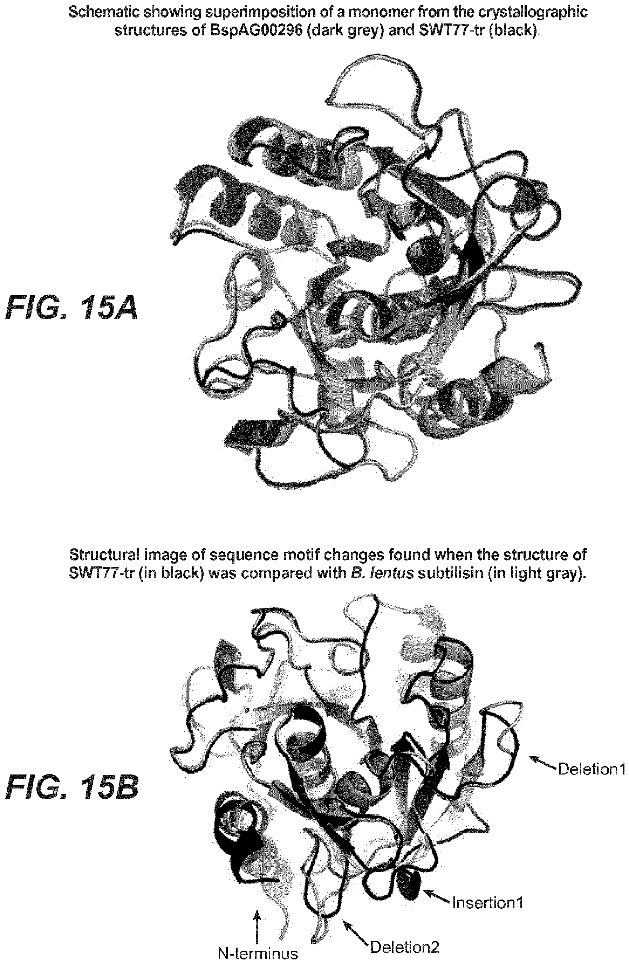

The coordinates of monomers of BspAG00296 and SWT77-tr superpose with an overall RMS of 0.342Å for 1567 common atoms. Though these two structures were determined in different space groups containing either two or four molecules in the asymmetric unit, the overall folding of the two structures is within experimental error, identical. This is illustrated in Figure 15A.

This confirms that the changes in the WHY-clade eliminate the tight calcium site found in the other known commercial subtilisins. As can be seen from Figure 15B, when a schematic of the main chain folding of SWT77-tr (in black) was compared with *B. lentus* subtilisin (in light gray) in the region of the segments including deletions 1 and 2, no electron density was found for calcium in the loop formed by residues 76-81 in SWT77-tr, that is somewhat compensated for by Insertion 1 and alterations in the N-terminal segment as well.

When the structures are superposed as shown in Figure 15B, changes arising from Deletion 2 and the N-terminus, which eliminate the calcium binding seen in other proteases, are clearly seen, as well as the change arising from Insertion 1 and Deletion 1.

## Claims

1. A recombinant polypeptide, or an active fragment thereof, comprising an amino acid sequence, wherein the amino acid sequence of the recombinant polypeptide or of the active fragment has at least 90% identity to the amino acid sequence of SEQ ID NO:3 or SEQ ID NO:4, and wherein the recombinant polypeptide or the active fragment has proteolytic activity, and wherein the recombinant polypeptide or the active fragment retains at least 80% activity after 20 minutes at 50°C under stressed conditions comprising a Tris/EDTA assay.

2. The recombinant polypeptide or the active fragment of claim 1, wherein:
(a) the polypeptide or the active fragment comprises a DTGIDXXHXXLXNLVXTSLGXSXVGGXXXDVXGH motif, wherein the initial D is the active site Aspartic acid, the terminal H is the active site Histidine, and X is any amino acid; or
(b) the polypeptide or the active fragment comprises a DTGIDXXHXXLXₐNLVXTSLGXSXVGGX_{b}XX_{c}DVXGH motif, wherein the initial D is the active site Aspartic acid, the terminal H is the active site Histidine, and X, Xa, Xb, and Xc are any amino acid, provided that when Xa is arginine, Xb and Xc are not glycine.

3. The recombinant polypeptide or the active fragment of claim 1 or claim 2, wherein:
(a) the polypeptide or the active fragment comprises a VSG sequence at residue positions 80-82, wherein the amino acid positions of the polypeptide or the active fragment are numbered by correspondence with the amino acid sequence set forth in SEQ ID NO:7;
(b) the polypeptide or the active fragment comprises an insertion of at least one amino acid residue compared to SEQ ID NO:18, wherein the insertion is between residue positions 39-47, wherein the residue positions are numbered by correspondence with the amino acid sequence set forth in SEQ ID NO:18, optionally wherein the residue positions 39-47 are replaced with HQSLANLVNTSLG;
(c) the polypeptide or the active fragment comprises a deletion of at least one amino acid residue compared to SEQ ID NO:18, wherein the deletion is between residue positions 51-64, wherein the residue positions are numbered by correspondence with the amino acid sequence set forth in SEQ ID NO:18, optionally wherein the residue positions 51-64 are replaced with VGGSTMDVQGH, VGGSA/PEDVQGH, VGGNPEDRQGH, or VGGTPADVHGH; and/or
(d) the polypeptide or the active fragment comprises a deletion of at least one amino acid residue compared to SEQ ID NO:18, wherein the deletion is between residue positions 68-95, wherein the residue positions are numbered by correspondence with the amino acid sequence set forth in SEQ ID NO:18, optionally wherein the residue positions 68-95 are replaced with VAGTIASYGSVSGVMHNATLVPVKV.

4. The recombinant polypeptide or the active fragment of any of the preceding claims, wherein the polypeptide retains at least 50% of its maximal protease activity at a pH range of 5 to 12;
the polypeptide retains at least 50% of its maximal protease activity at a temperature range of 55°C to 80°C; and/or
the recombinant polypeptide or the active fragment has at least 95% identity to the amino acid sequence of SEQ ID NO:3 or SEQ ID NO:4.

5. The recombinant polypeptide or the active fragment of any of the preceding claims, wherein the stressed conditions are the Tris/EDTA assay conditions of Example 11.

6. The recombinant polypeptide or the active fragment of any of the preceding claims, wherein the recombinant polypeptide or the active fragment comprises at least one substitution selected from X003N, X006R, X010E, X020I, X026N, X028R, X029I, X038A, X041P, X042N, X044R, X048D, X053R X059G, X061G, X085Q, X088R, X090I, X096G, X098N, X103M, X104Y, X107Q, X113A, X115S, X117N, X131D, X132S, X133D, X136N, X137N, X138I, X139N, X143S, X144S, X146T, X147L, X157R, X168N, X169A, X178N, X179R, X180T, X204Y, X207G, X208Q, X209F, X210R, X212L, X219T, X222V, X229I, X230K, X231S, X231A, X239T, X240Q, X241V, X243N, X245L, X246R, X247D, X255L, X256N, X257Q, X264N, X266Y, X271A, and X273G.

7. A composition comprising a surfactant and the recombinant polypeptide of any one of Claims 1-6, optionally wherein the composition is a detergent composition selected from a laundry detergent, a fabric softening detergent, an automatic dishwashing detergent, a hand dish detergent, and a hard-surface cleaning detergent.

8. The composition of claim 7, wherein said composition further comprises:
at least one calcium ion and/or zinc ion,
at least one stabilizer,
at least one bleaching agent,
at least one adjunct ingredient, and/or
one or more additional enzymes or enzyme derivatives selected from acyl transferases, alpha-amylases, beta-amylases, alpha-galactosidases, arabinosidases, aryl esterases, beta-galactosidases, carrageenases, catalases, cellobiohydrolases, cellulases, chondroitinases, cutinases, endo-beta-1, 4-glucanases, endo-beta-mannanases, esterases, exo-mannanases, galactanases, glucoamylases, hemicellulases, hyaluronidases, keratinases, laccases, lactases, ligninases, lipases, lipoxygenases, mannanases, oxidases, pectate lyases, pectin acetyl esterases, pectinases, pentosanases, peroxidases, phenoloxidases, phosphatases, phospholipases, phytases, polygalacturonases, proteases, pullulanases, reductases, rhamnogalacturonases, beta-glucanases, tannases, transglutaminases, xylan acetyl-esterases, xylanases, xyloglucanases, xylosidases, metalloproteases, additional serine proteases, and combinations thereof.

9. The composition of claim 7 or claim 8, wherein:
(a) said composition comprises from about 0.001% to about 1.0 weight % of a recombinant polypeptide of any one of Claims 1-6;
(b) said composition is phosphate-free;
(c) said composition contains phosphate;
(d) said composition is boron-free;
(e) said composition contains boron; or
(f) said composition is formulated at a pH of from about 7 to about 12.

10. A method of cleaning, comprising contacting a surface or an item with a composition comprising (i) a buffer and the recombinant polypeptide of any one of Claims 1-6, or (ii) the composition of any one of Claims 7-9.

11. A method for producing a recombinant polypeptide comprising:
(a) stably transforming a host cell with an expression vector comprising a polynucleotide encoding the polypeptide of any one of Claims 1-6;
(b) cultivating said transformed host cell under conditions suitable for said host cell to produce said polypeptide; and
(c) recovering said polypeptide.

12. A polynucleotide comprising a nucleic acid sequence encoding an amino acid sequence selected from SEQ ID NO:3, and SEQ ID NO:4.

13. An expression vector comprising the polynucleotide of Claim 12.

14. A host cell transformed with the vector of Claim 13.

15. A textile or leather processing composition or a feather processing, animal feed, contact lens cleaning, wound cleaning composition comprising the polypeptide of any one of Claims 1-6.

## Patentansprüche

1. Rekombinantes Polypeptid oder aktives Fragment davon, umfassend eine Aminosäuresequenz, wobei die Aminosäuresequenz des rekombinanten Polypeptids oder des aktiven Fragments mindestens 90 % Identität mit der Aminosäuresequenz von SEQ ID NO: 3 oder SEQ ID NO: 4 aufweist und wobei das rekombinante Polypeptid oder das aktive Fragment proteolytische Aktivität aufweist und wobei das rekombinante Polypeptid oder das aktive Fragment mindestens 80 % Aktivität nach 20 Minuten bei 50 °C unter beanspruchten Bedingungen, die einen Tris/EDTA-Assay umfassen, beibehält.

2. Rekombinantes Polypeptid oder aktives Fragment nach Anspruch 1, wobei:
(a) das Polypeptid oder das aktive Fragment ein DTGIDXXHXXLXNLVXTSLGXSXVGGXXXDVXGH-Motiv umfasst, wobei das Anfangs-D die Aktivstellen-Asparaginsäure ist, das endständige H das Aktivstellen-Histidin ist und X irgendeine Aminosäure ist; oder
(b) das Polypeptid oder das aktive Fragment ein DTGIDXXHXXLXₐNLVXTSLGXSXVGGX_{b}XX_{c}DVXGH-Motiv umfasst, wobei das Anfangs-D die Aktivstellen-Asparaginsäure ist, das endständige H das Aktivstellen-Histidin ist und X, Xₐ, X_{b} und X_{c} irgendeine Aminosäure sind, vorausgesetzt, dass, wenn Xₐ Arginin ist, Xb und X_{c} nicht Glycin sind.

3. Rekombinantes Polypeptid oder aktives Fragment nach Anspruch 1 oder Anspruch 2, wobei:
(a) das Polypeptid oder das aktive Fragment eine VSG-Sequenz an den Restpositionen 80-82 umfasst, wobei die Aminosäurepositionen des Polypeptids oder des aktiven Fragments durch Korrespondenz mit der Aminosäuresequenz, die in SEQ ID NO: 7 aufgeführt ist, nummeriert sind;
(b) das Polypeptid oder das aktive Fragment eine Insertion von mindestens einem Aminosäurerest im Vergleich mit SEQ ID NO: 18 umfasst, wobei die Insertion zwischen den Restpositionen 39-47 liegt, wobei die Restpositionen durch Korrespondenz mit der Aminosäuresequenz, die in SEQ ID NO: 18 aufgeführt ist, nummeriert sind, wobei wahlweise die Restpositionen 39-47 wahlweise durch HQSLANLVNTSLG ersetzt sind;
(c) das Polypeptid oder das aktive Fragment eine Deletion von mindestens einem Aminosäurerest im Vergleich mit SEQ ID NO: 18 umfasst, wobei die Deletion zwischen den Restpositionen 51-64 liegt, wobei die Restpositionen durch Korrespondenz mit der Aminosäuresequenz, die in SEQ ID NO: 18 aufgeführt ist, nummeriert sind, wobei die Restpositionen 51-64 wahlweise durch VGGSTMDVQGH, VGGSA/PEDVQGH, VGGNPEDRQGH oder VGGTPADVHGH ersetzt sind; und/oder
(d) das Polypeptid oder das aktive Fragment eine Deletion von mindestens einem Aminosäurerest im Vergleich mit SEQ ID NO: 18 umfasst, wobei die Deletion zwischen den Restpositionen 68-95 liegt, wobei die Restpositionen durch Korrespondenz mit der Aminosäuresequenz, die in SEQ ID NO: 18 aufgeführt ist, nummeriert sind, wobei wahlweise die Restpositionen 68-95 durch VAGTIASYGSVSGVMHNATLVPVKV ersetzt sind.

4. Rekombinantes Polypeptid oder aktives Fragment davon nach irgendeinem der vorhergehenden Ansprüche, wobei das Polypeptid mindestens 50 % seiner maximalen Proteaseaktivität in einem pH-Wertbereich von 5 bis 12 beibehält;
das Polypeptid mindestens 50 % seiner maximalen Proteaseaktivität in einem Temperaturbereich von 55 °C bis 80 °C beibehält; und/oder
das rekombinante Polypeptid oder das aktive Fragment mindestens 95 % Identität mit der Aminosäuresequenz von SEQ ID NO: 3 oder SEQ ID NO: 4 aufweist.

5. Rekombinantes Polypeptid oder aktives Fragment nach irgendeinem der vorhergehenden Ansprüche, wobei die beanspruchten Bedingungen die Tris/EDTA-Assaybedingungen von Beispiel 11 sind.

6. Rekombinantes Polypeptid oder aktives Fragment nach irgendeinem der vorhergehenden Ansprüche, wobei das rekombinante Polypeptid oder das aktive Fragment mindestens eine Substitutionen umfasst ausgewählt unter X003N, X006R, X010E, X020I, X026N, X028R, X029I, X038A, X041P, X042N, X044R, X048D, X053R, X059G, X061G, X085Q, X088R, X090I, X096G, X098N, X103M, X104Y, X107Q, X113A, X115S, X117N, X131D, X132S, X133D, X136N, X137N, X138I, X139N, X143S, X144S, X146T, X147L, X157R, X168N, X169A, X178N, X179R, X180T, X204Y, X207G, X208Q, X209F, X210R, X212L, X219T, X222V, X229I, X230K, X231S, X231A, X239T, X240Q, X241V, X243N, X245L, X246R, X247D, X255L, X256N, X257Q, X264N, X266Y, X271A und X273G.

7. Zusammensetzung umfassend ein Tensid und das rekombinante Polypeptid nach irgendeinem der Ansprüche 1-6, wobei wahlweise die Zusammensetzung eine Detergenszusammensetzung ist ausgewählt unter einem Waschpulver, einem Weichspüldetergens, einem Geschirrspülerdetergens, einem Handspülmittel und einem Reinigungsdetergens für harte Oberflächen.

8. Zusammensetzung nach Anspruch 7, wobei die Zusammensetzung ferner Folgendes umfasst:
mindestens ein Calciumion und/oder Zinkion,
mindestens einen Stabilisator,
mindestens ein Bleichmittel,
mindestens einen Zusatzbestandteil und/oder
ein oder mehrere zusätzliche Enzyme oder Enzymderivate ausgewählt unter Acyltransferasen, Alpha-Amylasen, Beta-Amylasen, Alpha-Galactosidasen, Arabinosidasen, Arylesterasen, Beta-Galactosidasen, Carrageenen, Katalasen, Cellobiohydrolasen, Cellulasen, Chondroitinasen, Cutinasen, Endo-beta-1,4-Glucanasen, Endo-beta-mannanasen, Esterasen, Exomannanasen, Galactanasen, Glucoamylasen, Hemicellulasen, Hyaluronidasen, Keratinasen, Laccasen, Lactasen, Ligninasen, Lipasen, Lipoxygenasen, Mannanasen, Oxidasen, Pectatlyasen, Pectinacetylesterasen, Pectinasen, Pentosanasen, Peroxidasen, Phenoloxidasen, Phosphatasen, Phospholipasen, Phytasen, Polygalacturonasen, Proteasen, Pullulanasen, Reductasen, Rhamnogalacturonasen, Beta-Glucanasen, Tannasen, Transglutaminasen, Xylanacetylesterasen, Xylanasen, Xyloglucanasen, Xylosidasen, Metalloproteasen, zusätzlichen Serinproteasen und Kombinationen davon.

9. Zusammensetzung nach Anspruch 7 oder Anspruch 8, wobei:
(a) die Zusammensetzung etwa 0,001 % bis etwa 1,0 Gew.-% eines rekombinanten Polypeptids nach irgendeinem der Ansprüche 1-6 umfasst;
(b) die Zusammensetzung phosphatfrei ist;
(c) die Zusammensetzung Phosphat enthält;
(d) die Zusammensetzung borfrei ist;
(e) die Zusammensetzung Bor enthält; oder
(f) die Zusammensetzung bei einem pH-Wert von etwa 7 bis etwa 12 formuliert wird.

10. Reinigungsverfahren umfassend das Kontaktieren einer Oberfläche oder eines Gegenstands mit einer Zusammensetzung umfassend (i) einen Puffer und das rekombinante Polypeptid nach irgendeinem der Ansprüche 1-6 oder (ii) die Zusammensetzung nach irgendeinem der Ansprüche 7-9.

11. Verfahren für die Herstellung eines rekombinanten Polypeptids, umfassend:
(a) stabiles Umwandeln einer Wirtszelle mit einem Expressionsvektor umfassend ein Polynukleotid, das für das Polypeptid nach irgendeinem der Ansprüche 1-6 codiert;
(b) Züchten der umgewandelten Wirtszelle unter Bedingungen, die geeignet sind, damit die Wirtszelle das Polypeptid herstellt; und
(c) Gewinnen des Polypeptids.

12. Polynukleotid umfassend eine Nukleinsäuresequenz, die für eine Aminosäuresequenz ausgewählt unter SEQ ID NO: 3 und SEQ ID NO: 4 codiert.

13. Expressionsvektor umfassend das Polynukleotid nach Anspruch 12.

14. Wirtszelle, die mit dem Vektor nach Anspruch 13 umgewandelt wird.

15. Textil- oder Lederverarbeitungszusammensetzung oder Federverarbeitungs-, Tierfutter-, Kontaktlinsenreinigung-, Wundreinigungszusammensetzung umfassend das Polypeptid nach irgendeinem der Ansprüche 1-6.

## Revendications

1. Polypeptide recombinant, ou un fragment actif de celui-ci, comprenant une séquence d'acides aminés, la séquence d'acides aminés du polypeptide recombinant ou du fragment actif ayant au moins 90 % d'identité vis-à-vis de la séquence d'acides aminés de SEQ ID no: 3 ou SEQ ID no: 4, et le polypeptide recombinant ou le fragment actif possédant une activité protéolytique, et le polypeptide recombinant ou le fragment actif conservant au moins 80 % d'activité après 20 minutes à 50°C sous des conditions de stress comprenant un essai Tris/EDTA.

2. Polypeptide recombinant ou fragment actif selon la revendication 1, dans lequel:
(a) le polypeptide ou le fragment actif comprend un motif DTGIDXXHXXLXNLVXTSLGXSXVGGXXXDVXGH, dans lequel le D initial est le site actif acide aspartique, le terminal H est le site actif histidine, et X est n'importe quel acide aminé; ou
(b) le polypeptide ou le fragment actif comprend un motif DTGIDXXHXXLXₐNLVXTSLGXSXVGGXbXX_{c}DVXGH, dans lequel le D initial est le site actif acide aspartique, le terminal H est le site actif histidine, et X, Xₐ, X_{b}, et X_{c} sont n'importe quel acide aminé, à condition que lorsque Xₐ est l'arginine, X_{b} et X_{c} ne soient pas la glycine.

3. Polypeptide recombinant ou fragment actif selon la revendication 1 ou la revendication 2:
(a) le polypeptide ou le fragment actif comprenant une séquence VSG aux positions de résidu 80 à 82, les positions d'acides aminés du polypeptide ou du fragment actif étant numérotées par correspondance avec la séquence d'acides aminés énoncée dans SEQ ID no: 7;
(b) le polypeptide ou le fragment actif comprenant une insertion d'au moins un résidu d'acide aminé comparée à SEQ ID no: 18, l'insertion se trouvant entre les positions de résidu 39 à 47, où les positions de résidu sont numérotées par correspondance avec la séquence d'acides aminés énoncée dans SEQ ID no: 18, éventuellement où les positions de résidu 39 à 47 sont remplacées par HQSLANLVNTSLG;
(c) le polypeptide ou le fragment actif comprenant une délétion d'au moins un résidu d'acide aminé comparé à SEQ ID no: 18, la délétion se trouvant entre les positions de résidu 51 à 64, les positions de résidu étant numérotées par correspondance avec la séquence d'acides aminés énoncée dans SEQ ID no: 18, éventuellement les positions de résidu 51 à 64 étant remplacées par VGGSTMDVQGH, VGGSA/PEDVQGH, VGGNPEDRQGH, ou VGGTPADVHGH; et/ou
(d) le polypeptide ou le fragment actif comprenant une délétion d'au moins un résidu d'acide aminé comparé à SEQ ID no: 18, la délétion se trouvant entre les positions de résidu 68 à 95, les positions de résidu étant numérotées par correspondance avec la séquence d'acides aminés énoncée dans SEQ ID no: 18, éventuellement les positions de résidu 68 à 95 étant remplacées par VAGTIASYGSVSGVMHNATLVPVKV.

4. Polypeptide recombinant ou fragment actif selon l'une quelconque des revendications précédentes, le polypeptide conservant au moins 50 % de son activité protéase maximale à une plage de pH de 5 à 12;
le polypeptide conservant au moins 50 % de son activité protéase maximale à une plage de températures de 55°C à 80°C; et/ou
le polypeptide recombinant ou le fragment actif possédant au moins 95 % d'identité vis-à-vis de la séquence d'acides aminés de SEQ ID no: 3 ou SEQ ID no: 4.

5. Polypeptide recombinant ou fragment actif selon l'une quelconque des revendications précédentes, les conditions de stress étant les conditions d'essai Tris/EDTA de l'Exemple 11.

6. Polypeptide recombinant ou fragment actif selon l'une quelconque des revendications précédentes, le polypeptide recombinant ou le fragment actif comprenant au moins une substitution sélectionnée parmi X003N, X006R, X010E, X020I, X026N, X028R, X029I, X038A, X041P, X042N, X044R, X048D, X053R X059G, X061G, X085Q, X088R, X090I, X096G, X098N, X103M, X104Y, X107Q, X113A, X115S, X117N, X131D, X132S, X133D, X136N, X137N, X138I, X139N, X143S, X144S, X146T, X147L, X157R, X168N, X169A, X178N, X179R, X180T, X204Y, X207G, X208Q, X209F, X210R, X212L, X219T, X222V, X229I, X230K, X231S, X231A, X239T, X240Q, X241V, X243N, X245L, X246R, X247D, X255L, X256N, X257Q, X264N, X266Y, X271A, et X273G.

7. Composition comprenant un tensioactif et polypeptide recombinant selon l'une quelconque des revendications 1 à 6, éventuellement la composition étant une composition détergente sélectionnée parmi un détergent de blanchisserie, un détergent d'assouplissement de textile, un détergent de lavage automatique de vaisselle, un détergent de lavage de vaisselle à la main, et un détergent de nettoyage des surfaces dures.

8. Composition selon la revendication 7, ladite composition comprenant en outre:
au moins un ion calcium et/ou un ion zinc,
au moins un agent stabilisant,
au moins un agent de blanchissage,
au moins un agent auxiliaire, et/ou
une ou plusieurs enzymes ou dérivés d'enzyme additionnels sélectionnés parmi les acyl transférases, alpha-amylases, bêta-amylases, alpha-galactosidases, arabinosidases, aryl estérases, bêta-galactosidases, carraghénases, catalases, cellobiohydrolases, cellulases, chondroïtinases, cutinases, endo-bêta-1,4-glucanases, endo-bêta-mannanases, estérases, exo-mannanases, galactanases, glucoamylases, hémicellulases, hyaluronidases, kératinases, laccases, lactases, ligninases, lipases, lipoxygénases, mannanases, oxydases, pectate lyases, pectine acétyl estérases, pectinases, pentosanases, peroxydases, phénoloxydases, phosphatases, phospholipases, phytases, polygalacturonases, protéases, pullulanases, réductases, rhamnogalacturonases, bêta-glucanases, tannases, transglutaminases, xylan acétyl-estérases, xylanases, xyloglucanases, xylosidases, métalloprotéases, sérine protéases additionnelles, et des combinaisons de ceux-ci.

9. Composition selon la revendication 7 ou la revendication 8, dans laquelle:
(a) ladite composition comprend d'environ 0,001 % à environ 1,0 % en poids d'un polypeptide recombinant selon l'une quelconque des revendications 1 à 6;
(b) ladite composition étant exempte de phosphate;
(c) ladite composition contenant du phosphate;
(d) ladite composition étant exempte de bore;
(e) ladite composition contenant du bore; ou
(f) ladite composition étant formulée à un pH d'environ 7 à environ 12.

10. Procédé de nettoyage, comprenant la mise en contact d'une surface ou d'un article avec une composition comprenant (i) un tampon et le polypeptide recombinant selon l'une quelconque des revendications 1 à 6, ou (ii) la composition selon l'une quelconque des revendications 7 à 9.

11. Procédé de production d'un polypeptide recombinant comprenant:
(a) la transformation de manière stable d'une cellule hôte avec un vecteur d'expression comprenant un polynucléotide codant pour le polypeptide selon l'une quelconque des revendications 1 à 6;
(b) la culture de ladite cellule hôte transformée sous des conditions appropriées pour que ladite cellule hôte produise ledit polypeptide; et
(c) la récupération dudit polypeptide.

12. Polynucléotide comprenant une séquence d'acides nucléiques codant pour une séquence d'acides aminés sélectionnée parmi SEQ ID no: 3, et SEQ ID no: 4.

13. Vecteur d'expression comprenant le polynucléotide selon la revendication 12.

14. Cellule hôte transformée avec le vecteur selon la revendication 13.

15. Composition de traitement de textile ou de cuir ou de traitement de plumes, aliments pour animaux, composition de nettoyage de lentille de contact, composition de nettoyage des plaies comprenant le polypeptide selon l'une quelconque des revendications 1 à 6.
